(19) Europäisches Patentamt
European Patent Office
Office européen des brevets

(11) EP 0 434 394 B1

(12) EUROPEAN PATENT SPECIFICATION

(45) Date of publication and mention of the grant of the patent: **05.08.1998 Bulletin 1998/32**

(21) Application number: **90313923.6**

(22) Date of filing: **19.12.1990**

(51) Int. Cl.[6]: **C07D 277/20**, A61K 31/425, C07D 277/14, C07D 277/34, C07D 277/36, C07D 327/04, C07D 333/30

(54) **Compounds for treating inflammatory bowel disease**

Verbindungen zur Behandlung von Bauchentzündungen

Composés pour le traitement d'inflammations intestinales

(84) Designated Contracting States:
**AT BE CH DE DK ES FR GB GR IT LI LU NL SE**

(30) Priority: **21.12.1989 US 454203**

(43) Date of publication of application:
**26.06.1991 Bulletin 1991/26**

(73) Proprietor:
**ELI LILLY AND COMPANY**
**Indianapolis, Indiana 46285 (US)**

(72) Inventors:
- **Gidda, Jaswant Singh**
  **Carmel, Indiana, 46032 (US)**
- **Panetta, Jill Ann**
  **Zionsville, Indiana 46077 (US)**
- **Phillips, Michael LeRoy**
  **Indianapolis, Indiana 46217 (US)**

(74) Representative:
**Tapping, Kenneth George et al**
**Lilly Industries Limited**
**European Patent Operations**
**Erl Wood Manor**
**Windlesham Surrey GU20 6PH (GB)**

(56) References cited:
**EP-A- 0 211 670** **EP-A- 0 391 644**

- **CHEMICAL AND PHARMACEUTICAL BULLETIN, vol. 34, no. 4, April 1979, TOKYO, pages: 1619 - 1627; IKUO KATSUMI et al.: "STUDIES ON STYRENE DERIVATIVES II . SYNTHESIS AND ANTI INFLAMMATORY ACTIVITY OF 3,5-DI-TERT-BUTYL-4 HYDROXYSTYRENES"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 434 394 B1

## Description

Background of the Invention

Mammals, both humans and animals, are known to suffer from various conditions involving inflammation of the bowels. Such conditions are typically characterized by unpleasant symptoms such as diarrhea, cramping and loss of appetite. Certain of the conditions, in particular ulcerative colitis, are also characterized by patches of ulceration. Accordingly, there is a need for a safe drug which will decrease the severity of bowel inflammation and alleviate the symptoms associated therewith.

Teuber et al., Liebigs Ann. Chem., 757 (1978) discloses 5-([3,5-bis(1,1-dimethylethyl)-4-hydroxyphenyl]-methylene)-2-thioxo-4-thiazolidinone as an intermediate in the preparation of certain compounds, which are in turn used for the spin-labeling of peptides. No biological activity is disclosed for this intermediate compound.

European Patent Application 211,670 discloses certain di-t-butylphenol substituted rhodanine derivatives which are useful in treating inflammation, stroke and arthritis in mammals. The inflammatory conditions which may be treated using the reference compounds involve inflammation of skin tissue and joint swelling. Such conditions are commonly associated with diseases such as rheumatoid arthritis, rheumatoid spondylitis, osteoarthritis, degenerative joint diseases and the like. Methods for treating inflammatory conditions involving inflammation of the bowels are not disclosed.

The present invention provides the use for preparing a medicament for of treating inflammatory bowel disease in a mammal suffering from said disease, or susceptible to said disease, of a compound of the Formula (I)

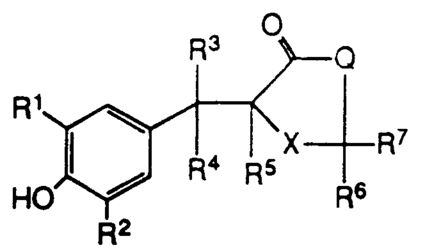

(I)

wherein:

$R^1$ and $R^2$ are each independently hydrogen, $C_1$-$C_6$ alkyl, $C_1$-$C_6$ alkoxy, $C_2$-$C_6$ alkenyl, $C_2$-$C_6$ alkynyl,

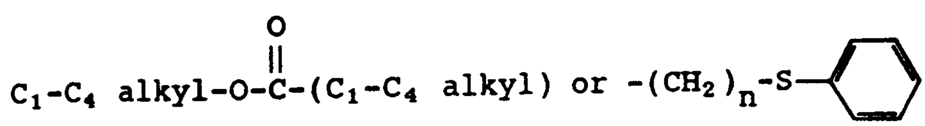

where n is an integer from 0 to 3, both inclusive;
$R^3$ is hydrogen or $C_1$-$C_6$ alkyl;
$R^4$ and $R^5$ are each hydrogen or when taken together form a bond;
$R^6$ and $R^7$ are each hydrogen or when taken together are =S, or when one of $R^6$ and $R^7$ is hydrogen the other is -OH or $-SCH_3$;
X is

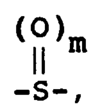

where m is 0, 1 or 2; and
Q is $-CH_2-$, -O- or $NR^8$ where $R^8$ is hydrogen, $C_1$-$C_6$ alkyl, $C_2$-$C_6$ alkenyl, $C_3$-$C_8$ cycloalkyl, $-SO_2CH_3$ or $-(CH_2)_n-Y$, where n is an integer from 0 to 3, both inclusive, and Y is cyano, $OR^9$,

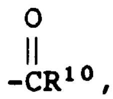

tetrazolyl, $-NR^{11}R^{12}$, -SH, $-S(C_1-C_4$ alkyl) or

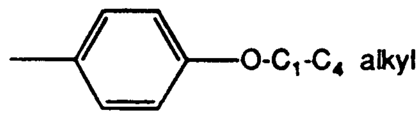

where $R^9$ is hydrogen, $C_1-C_4$ alkyl, or

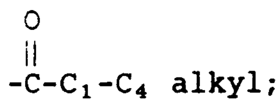

$R^{10}$ is $C_1-C_4$ alkyl, $C_1-C_4$ alkoxy or $-NH_2$; $R^{11}$ and $R^{12}$ are each independently hydrogen, $C_1-C_6$ alkyl, $C_2-C_6$ alkenyl, $C_2-C_6$ alkynyl, $-(CH_2)_qOH$, $-(CH_2)_q-N(C_1-C_4\ alkyl)_2$, $-(CH_2)_q-S(C_1-C_4$ alkyl) or

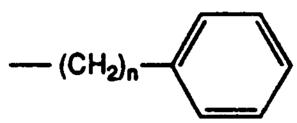

where n is as defined above and q is an integer from 1 to 6, both inclusive; or $R^{11}$ and $R^{12}$ taken together form a morpholinyl, piperidinyl, piperazinyl or N-methylpiperazinyl ring; or a pharmaceutically acceptable salt thereof. The present invention provides for safe and efficacious reduction in the severity of bowel inflammation, and also alleviates the unpleasant symptoms associated therewith.

The present invention further provides new compounds of the Formula II

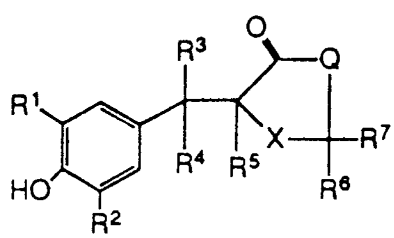

(II)

wherein:

$R^1$ is $C_2-C_6$ alkenyl, $C_2-C_6$ alkynyl or

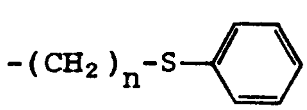

where n is an integer from 0 to 3, both inclusive;
$R^2$ is hydrogen, $C_1-C_6$ alkyl, $C_1-C_6$ alkoxy, $C_2-C_6$ alkenyl, $C_2-C_6$ alkynyl,

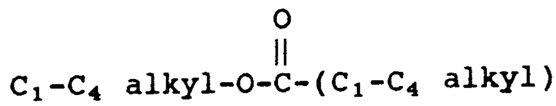 or 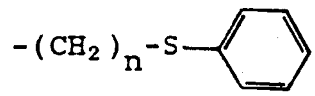

where n is as defined above; and $R^3$, $R^4$, $R^5$, $R^6$, $R^7$, X and Q are as defined for Formula I; and pharmaceutically acceptable salts thereof.

Preferably $R^1$ is $C_2$-$C_6$ alkenyl, $C_2$-$C_6$ alkynyl or

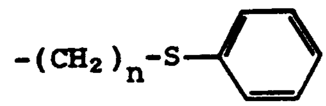

where n is an integer from 0 to 3, both inclusive;

$R^2$ is hydrogen, $C_1$-$C_6$ alkyl, $C_1$-$C_6$ alkoxy, $C_2$-$C_6$ alkenyl, $C_2$-$C_6$ alkynyl, or

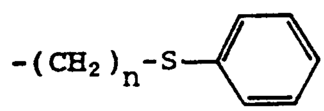

where n is an integer from 0 to 3, both inclusive;
$R^3$ is hydrogen or $C_1$-$C_6$ alkyl;
$R^4$ and $R^5$ are each hydrogen or when taken together form a bond;
$R^6$ and $R^7$ are each hydrogen or when taken together are =S, or when one of $R^6$ and $R^7$ is hydrogen the other is -OH or $-SCH_3$;
X is

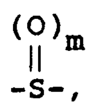

where m is 0, 1 or 2; and
Q is $-CH_2-$, -O- or $NR^8$ where $R^8$ is hydrogen, $C_1$-$C_6$ alkyl, $C_3$-$C_8$ cycloalkyl, $-SO_2CH_3$ or $-(CH_2)_n-Y$, where n is an integer from 0 to 3, both inclusive, and Y is cyano, $OR^9$,

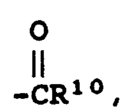

tetrazolyl, $-NR^{11}R^{12}$ or

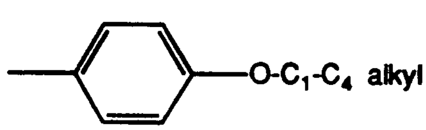

where $R^9$ is hydrogen, $C_1$-$C_4$ alkyl, or

$$-\overset{\displaystyle O}{\overset{\|}{C}}-C_1-C_4\ \text{alkyl;}$$

$R^{10}$ is $C_1$-$C_4$ alkyl, $C_1$-$C_4$ alkoxy or $-NH_2$; $R^{11}$ and $R^{12}$ are each independently hydrogen, $C_1$-$C_6$ alkyl, $-(CH_2)_qOH$, $-(CH_2)_q-N(C_1-C_4\ \text{alkyl})_2$, $-(CH_2)_q-S(C_1-C_4\ \text{alkyl})$ or

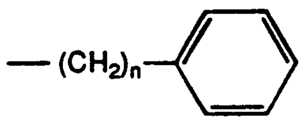

where n is as defined above and q is an integer from 1 to 6, both inclusive; or $R^{11}$ and $R^{12}$ taken together form a morpholinyl, piperidinyl, piperazinyl or N-methylpiperazinyl ring,

while more preferably $R^1$ is $C_2$-$C_6$ alkenyl, $R^2$ is $C_2$-$C_6$ alkenyl or $C_1$-$C_6$ alkyl; $R^3$ is hydrogen; $R^4$ and $R^5$ are hydrogen or when taken together form a bond; $R^6$ and $R^7$ are each hydrogen or when taken together are =S; X is

$$\overset{\displaystyle (O)_m}{\overset{\|}{-S-}},$$

where m is O; and Q is -O- or $NR^8$, where $R^8$ is as defined above.

According to a further aspect of the present invention, there are provided pharmaceutical compositions comprising as active ingredient a compound of Formula II, or a pharmaceutically acceptable salt thereof, in association with one or more pharmaceutically acceptable diluents, carriers or excipients therefor.

The present invention also provides a process for selectively isolating, in substantially pure enantiomeric form, one of the enantiomers of a racemic mixture of a compound of Formula I wherein X is -S-; $R^4$ and $R^5$ are hydrogen; and $R^1$, $R^2$, $R^3$, $R^6$, $R^7$ and Q are as defined for Formula I, comprising

a) reacting the racemic sulfide compound with a reagent prepared from the combination of a tartrate ligand, a titanium alkoxide, a hydroperoxide and, optionally, water until substantially all of the undesired enantiomer of the sulfide substrate has been converted to its sulfoxide analog; and

b) separating the unreacted portion of the sulfide starting material, consisting essentially of substantially pure desired enantiomer, from the reaction mixture.

As used herein, the term "$C_1$-$C_6$ alkyl" refers to straight and branched chain aliphatic radicals of 1 to 6 carbon atoms, both inclusive, such as methyl, ethyl, propyl, isopropyl, butyl, isobutyl, sec-butyl, tert-butyl, n-pentane, isopentane, n-hexane, isohexane and the like. The term "$C_1$-$C_6$ alkyl" includes within its definition the term "$C_1$-$C_4$ alkyl".

The term "$C_1$-$C_6$ alkoxy" refers to the alkyl radicals of 1 to 6 carbon atoms, both inclusive, attached to the remainder of the molecule by oxygen and includes methoxy, ethoxy, propoxy, isopropoxy, butoxy, isobutoxy, sec-butoxy, tert-butoxy, pentoxy, hexoxy and the like. The term "$C_1$-$C_6$ alkoxy" includes within its definition the term "$C_1$-$C_4$ alkoxy".

The term "$C_2$-$C_6$ alkenyl" refers to straight and branched chain radicals of 2 to 6 carbon atoms, both inclusive, having a double bond. As such, the term includes ethylene, propylene, isopropylene, 1-butene, 2-butene, 2-methyl-1-propene, 1-pentene, 2-pentene, 2-methyl-2-butene and the like.

The term "$C_2$-$C_6$ alkynyl" refers to straight and branched chain radicals of 2 to 6 carbon atoms, both inclusive, having a triple bond. As such , the term includes acetylene, propyne, 1-butyne, 2-butyne, 1-pentyne, 2-pentyne, 3-methyl-1-butyne, 1-hexyne, 2-hexyne, 3-hexyne and the like.

Compounds of Formula I wherein $R^1$ and $R^2$ are other than

$$C_1\text{-}C_4\ \text{alkyl-O-}\overset{\text{O}}{\overset{\|}{\text{C}}}\text{-}(C_1\text{-}C_4\ \text{alkyl}),$$

$R^8$ is other than $C_2$-$C_6$ alkenyl, Y is other than -SH or -S($C_1$-$C_4$ alkyl) and $R^{11}$ and $R^{12}$ are other than $C_2$-$C_6$ alkenyl or $C_2$-$C_6$ alkynyl are preferred for use in the method of treating inflammatory bowel disease of the present invention. Of this preferred group of compounds, somewhat more preferred are those compounds of Formula I wherein $R^1$ and $R^2$ are each $C_1$-$C_6$ alkyl, $C_2$-$C_6$ alkenyl, $C_1$-$C_6$ alkoxy or

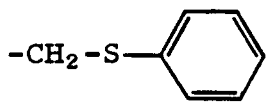 ;

$R^3$ is hydrogen; $R^4$ and $R^5$ are each hydrogen or when taken together form a bond; $R^6$ and $R^7$ are each hydrogen or when taken together are =S; X is

$$\overset{(\text{O})_m}{\overset{\|}{\text{-S-}}},$$

where m is 0; and Q is -O- or $NR^8$, where $R^8$ is as defined for the preferred group of compounds. Of this somewhat more preferred group of compounds, particularly preferred compounds for use in treating inflammatory bowel disease are those compounds wherein $R^1$, $R^2$, $R^3$, $R^4$, $R^5$, $R^6$, $R^7$, X and m are as set forth immediately above, and Q is $NR^8$ where $R^8$ is hydrogen, $C_1$-$C_6$ alkyl or -$(CH_2)_n$-Y; where n is 0 and Y is -$NR^{11}R^{12}$ ($R^{11}$ and $R^{12}$ each being independently hydrogen or $C_1$-$C_6$ alkyl).

Of these particularly preferred compounds, especially preferred compounds for use in the method of the present invention are those compounds wherein $R^1$ and $R^2$ are independently $C_1$-$C_6$ alkyl, in particular 1,1-dimethylethyl; $R^3$, $R^4$, $R^5$, $R^6$ and $R^7$ are hydrogen; X is

$$\overset{(\text{O})_m}{\overset{\|}{\text{-S-}}},$$

where m is 0; and Q is $NR^8$ where $R^8$ is hydrogen. The most preferred compounds for use in the method of treating inflammatory bowel disease provided by the present invention are 5-[[3,5-bis(1,1-dimethylethyl)-4-hydroxyphenyl]methyl]-4-thiazolidinone, 5-[[3-(1,1-dimethylethyl)-4-hydroxy-5-propylphenyl]methyl]-4-thiazolidinone and 5-[[3,5-dipropyl-4-hydroxyphenyl]methyl]-4-thiazolidinone.

Compounds of Formula II wherein $R^2$ is other than

$$C_1\text{-}C_4\ \text{alkyl-O-}\overset{\text{O}}{\overset{\|}{\text{C}}}\text{-}(C_1\text{-}C_4\ \text{alkyl}),$$

$R^8$ is other than $C_2$-$C_6$ alkenyl, Y is other than -SH or -S($C_1$-$C_4$ alkyl) and $R_{11}$ and $R_{12}$ are other than $C_2$-$C_6$ alkenyl or $C_2$-$C_6$ alkynyl are preferred. Of this preferred group of compounds, somewhat more preferred are those compounds of Formula II wherein $R^1$ is $C_2$-$C_6$ alkenyl; $R^2$ is $C_1$-$C_6$ alkyl or $C_2$-$C_6$ alkenyl; $R^3$ is hydrogen; $R^4$ and $R^5$ are each hydrogen or when taken together form a bond; $R^6$ and $R^7$ are each hydrogen or when taken together are =S; X is

$$\overset{(O)_m}{\overset{\|}{-S-}},$$

where m is 0; and Q is -O- or $NR^8$, where $R^8$ is as defined for the preferred group of compounds. Of this somewhat more preferred group of compounds, particularly preferred compounds are those compounds wherein $R^1$, $R^2$, $R^3$, $R^4$, $R^5$, $R^6$, $R^7$, X and m are as set forth immediately above, and Q is $NR^8$ where $R^8$ is hydrogen, $C_1$-$C_6$ alkyl or $-(CH_2)_n$-Y; where n is 0 and Y is $-NR^{11}R^{12}$ ($R^{11}$ and $R^{12}$ each being independently hydrogen or $C_1$-$C_6$ alkyl). Of these particularly preferred compounds, especially preferred compounds are those compounds wherein $R^1$ and $R^2$ are each independently $C_2$-$C_6$ alkenyl; $R^3$, $R^4$, $R^5$, $R^6$ and $R^7$ are hydrogen; X is

$$\overset{(O)_m}{\overset{\|}{-S-}},$$

where m is 0; and Q is $NR^8$ where $R^8$ is hydrogen. The most preferred compound of the present invention is 5-[[3,5-di-2-propenyl-4-hydroxyphenyl]methyl]-4-thiazolidinone.

The compounds of the present invention, as well as the compounds employed in the method of the present invention, wherein $R^4$ and $R^5$ are hydrogen have an asymmetric center at the carbon atom at the 5-position of the rhodanine, or rhodanine derivative, ring. As such, the compounds can exist as either a racemic mixture, or as individual stereoisomers. The method and compounds of the present invention encompass both the racemate and its individual stereoisomers. A process of the invention provides a method for obtaining stereoisomers of certain of the compounds of the present invention, as well as certain of the compounds used in the method of the present invention.

Pharmaceutically acceptable salts are considered to be encompassed within the compounds and method of the present invention. Such salts may be prepared by reacting a compound of Formula I or II with a strong base, such as sodium hydroxide, or a strong acid such as hydrochloric acid.

Compounds of the present invention include the following:

5-[[3,5-diethenyl-4-hydroxyphenyl]methylene]-3-(3-methoxypropyl)-2-thioxo-4-thiazolidinone
5-[[3,5-bis(4-pentyne)-4-hydroxyphenyl]methyl]-3-ethylamino-4-thiazolidinone
5-[[3-ethylthiophenyl-4-hydroxy-5-methylphenyl]methylene]-2-thioxo-4-thiazolidinone
5-[[3-(2-butene)-4-hydroxy-5-isopropoxyphenyl]methyl]-3-(3-diethylaminopropyl)-4-thiazolidinone
5-[[3-(2-propenyl)-4-hydroxy-5-(1,1-dimethylethyl)phenyl]methylene]-3-cyclohexyl-4-thiazolidinone
5-[[3,5-(methylthiophenyl)-4-hydroxyphenyl]methylene]-3-propyl-2-thioxo-4-thiazolidinone
5-[[3,5-diacetylene-4-hydroxyphenyl]methyl]-4-thiazolidinone
5-[[3-(3-methyl-1-butene)-4-hydroxy-5-propylphenyl]methylene]-3-ethylcyano-4-thiazolidinone
5-[[3-(2-propenyl)-4-hydroxy-5-methoxyphenyl]methyl]-3-ethoxy-4-thiazolidinone
5-[[3,5-di-2-propenyl)-4-hydroxyphenyl]methylene]-3-(methylaminomethyl)-2-thioxo-4-thiazolidinone

The following compounds illustrate representative compounds, in addition to those mentioned above, which are suitable for use in the method of the present invention.

5-[[3,5-bis(1,1-dimethylethyl)-4-hydroxyphenyl]methylene]-3-(3-methoxypropyl)-2-thioxo-4-thiazolidinone
5-[[3,5-bis(1,1-dimethylethyl)-4-hydroxyphenyl]methylene]-2-thioxo-4-thiazolidinone
5-[[3,5-bis(1,1-dimethylethyl)-4-hydroxyphenyl]methylene]-4-thiazolidinone
5-[[3,5-bis(1,1-dimethylethyl)-4-hydroxyphenyl]methyl]-4-thiazolidinone
5-[[3,5-bis(1,1-dimethylethyl)-4-hydroxyphenyl]methyl]-2-thioxo-4-thiazolidinone
3-acetyl-5-[[3,5-bis(1,1-dimethylethyl)-4-hydroxyphenyl]methylene]-4-thiazolidinone
5-[[3,5-bis(1,1-dimethylethyl)-4-hydroxyphenyl]methyl-3-[methyl(1-methylethyl)amino]-4-thiazolidinone
5-[4-hydroxybenzal]rhodanine
5-(4-hydroxy-3-methoxybenzylidene)rhodanine
5-[(4-hydroxy-3,5-dipropylphenyl)methylene]-3-[2-(dimethylamino)ethyl]-4-thiazolidinone
5-[[3,5-bis(1-methylpropyl)-4-hydroxyphenyl]methyl]-3-methyl-4-thiazolidinone
5-[[3,5-dimethyl-4-hydroxyphenyl]methylene]-3-methyl-4-thiazolidinone
5-[[3,5-bis(1,1-dimethylethyl)-4-hydroxyphenyl]methyl]-3-(methylsulfonyl)-4-thiazolidinone

5-[[4-hydroxy-3,5-bis(1,1-dimethylethyl)phenyl]methyl]-3-(propylamino)-4-thiazolidinone
3-amino-5-[[3,5-bis(1,1-dimethylethyl)-4-hydroxyphenyl]methylene]-2-thioxo-4-thiazolidinone
5-[[3,5-bis(1-methylethyl)-4-hydroxyphenyl]methyl]-3-methyl-4-thiazolidinone
5-[(4-hydroxy-3,5-dimethoxyphenyl)methyl]-3-methyl-2-thioxo-4-thiazolidinone
5-[(4-hydroxy-3,5-dimethoxyphenyl)methylene]-3-methyl-2-thioxo-4-thiazolidinone

Some of the compounds employed in the use of the present invention are known, see, e.g., European Patent Application 211,670 and Teuber et al., Leibigs Ann. Chem., 757 (1978). However, the majority of the compounds used in the method of the present invention, as well as the compounds of the present invention, are novel. In general, these compounds may be synthesized as follows.

Teuber et al. disclose 5-[[3,5-bis(1,1-dimethylethyl)-4-hydroxyphenyl]methylene]-2-thioxo-4-thiazolidinone (referred to in the following discussion as Compound A). The compound is prepared by reacting 3,5-di-tert-butyl-4-hydroxybenzaldehyde with rhodanine at reflux temperature in glacial acetic acid using fused sodium acetate as catalyst. 5-[[3,5-Bis(1,1-dimethylethyl)-4-hydroxyphenyl]methylene]-4-thiazolidinone (Compound B), 5-[[3,5-bis(1,1-dimethylethyl)-4-hydroxyphenyl]methyl]-4-thiazolidinone (Compound C) and 5-[[3,5-bis(1,1-dimethylethyl)-4-hydroxyphenyl]methyl]-2-thioxo-4-thiazolidinone (Compound D) can be prepared from Compound A.

For example, when Compound A is subjected to catalytic hydrogenation, one obtains both Compounds B and C. The relative proportions obtained depend upon the temperature, pressure, and duration of hydrogenation, the solvent employed, and the particular catalyst used. For example, when Compound A is treated with 5% palladium on carbon in ethanol at 100°C for approximately 18 hours, the relative ratios of Compound B: C are approximately 60:40. Alternatively, these transformations may be accomplished by heating Compound A in a mixture of hydrochloric acid and an alcohol, such as ethanol, in the presence of zinc. Reduction of the thione without affecting the benzylic double bond may be accomplished by heating the thione with a reducing agent such as tri-n-butyl tin hydride in a non-reactive solvent, such as toluene, and preferably in the presence of a free radical initiator, such as azobisisobutyronitrile. However, for such reduction to work an N-substituted rhodanine substrate (i.e., Q cannot be -NH) must be employed.

The transformation of Compound A to D may be accomplished by a variety of methods known in the art. A preferred method is that taught by Nakamura et al., Tetrahedron Letters, 25, 3983 (1984). In this reaction, Compound A is treated with a dihydropyridine such as diethyl 2,6-dimethyl-1,4-dihydro-3,5-pyridinedicarboxylate in the presence of silica gel. The reaction is best carried out in the presence of a nonreactive solvent such as benzene or toluene, preferably under an inert atmosphere. The reaction may be accomplished at temperatures from about 25°C up to the reflux temperature of the mixture. At the preferred temperature of approximately 80°C, the reaction is essentially complete after 12-18 hours.

Other thiazolidinones may, depending on the values selected for the various substituents, be prepared in an analogous fashion. For example, compounds of Formula I and Formula II wherein Q is $NR^8$ and $R^8$ is hydrogen, $C_1$-$C_6$ alkyl, $C_3$-$C_8$ cycloalkyl or $-(CH_2)_n$-Y, where n is an integer from 0 to 3, both inclusive, and Y is cyano or $NR^{11}R^{12}$ where $R^{11}$ and $R^{12}$ are each independently hydrogen or $C_1$-$C_6$ alkyl, may be prepared by the method of Teuber et al. described above, employing the appropriate N-substituted rhodanine and $R^1$, $R^2$-substituted-4-hydroxybenzaldehyde. Alternatively, rhodanine may be used for the condensation with the aldehyde to form those species wherein Q is $NR^8$ and $R^8$ is hydrogen, followed by alkylation with the appropriate $R^8$-containing halide, such as an iodide or bromide, to provide the corresponding N-substituted derivatives, i.e., those compounds of Formulae I or II in which $R^8$ is $C_1$-$C_6$ alkyl, $C_2$-$C_6$ alkenyl, $C_3$-$C_8$ cycloalkyl, or $-(CH_2)_n$-Y, where Y is cyano, $OR^9$, -SH, -S($C_1$-$C_4$ alkyl), $-NR^{11}R^{12}$ or

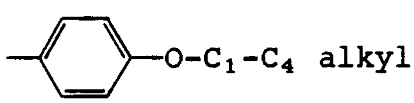

and n, $R^9$, $R^{11}$ and $R^{12}$ are as defined for Formula I. The alkylation is usually accomplished in an inert solvent such as tetrahydrofuran (THF) or dimethylformamide (DMF) in the presence of a strong base such as sodium hydride. In a similar fashion, rhodanine may be use for the condensation with the aldehyde to form those species wherein Q is $NR^8$ and $R^8$ is hydrogen followed by acylation with the appropriate $R^8$-containing halide to provide N-substituted derivatives of Formulae I or II in which $R^8$ is $-(CH_2)_n$-Y and Y is

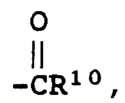

where n and $R^{10}$ are as defined for Formula I.

Compounds of Formulae I and II wherein Q is $NR^8$ and $R^8$ is $-(CH_2)_n-Y$ (Y is $OR^9$ or $NR^{11}R^{12}$, wherein $R^9$ is hydrogen, acetyl or tosyl and $R^{11}$ and $R^{12}$ are as defined for Formula I) may also be prepared according to the following reaction scheme:

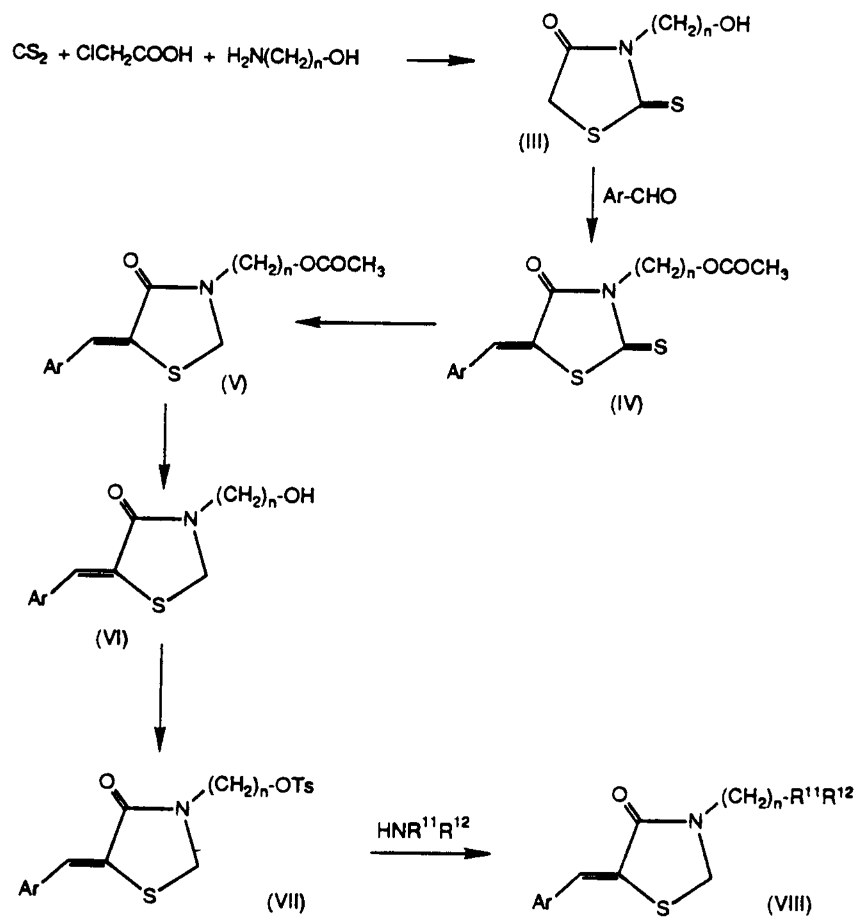


where Ts = tosyl; Ar =

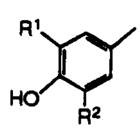

A hydroxyalkyl rhodanine III is prepared by condensing carbon disulfide, chloroacetic acid, and the appropriate hydroxyalkylamine by standard techniques. When condensed with the appropriate $R^1$,$R^2$-substituted-4-hydroxybenzaldehyde, as described above, the resulting product is the condensed 2-thioxo-4-thiazolidinone IV, which has been transformed into the acetyl derivative. The thioxo compound may optionally be converted to the methylene compound of formula V as described above. The acetyl group of intermediate V may be removed upon treatment with aqueous ammonia in a solvent such as acetonitrile to provide compound VI (i.e., the compound of Formulae I and II wherein Q is $NR^8$ and $R^8$ is $-(CH_2)_n-Y$ where Y is $OR^9$ and $R^9$ is hydrogen). The hydroxy compound VI is then converted to the tosyl derivative (VII) upon treatment with p-toluenesulfonyl chloride in pyridine, preferably at a temperature of around

0°C. The versatile tosyl intermediate VII may then be transformed into additional compounds of Formulae I and II upon treatment with an appropriate $HNR^{11}R^{12}$ amine, where $R^{11}$ and $R^{12}$ are as stated in the preceeding paragraph. This latter transformation is best accomplished by allowing VII to react in the presence of a molar excess of the amine. Once again, a solvent such as acetonitrile is useful for accomplishing this transformation.

The corresponding 1,3-oxothiolan-5-ones of Formulae I and II may be prepared from β-(3,5-di-t-butyl-4-hydroxyphenyl)-α-mercaptoacrylic acid (IX). Compound IX may be treated with carbon disulfide to prepare the thione analog (Formulae I and II, Q is -O-, $R^6$ and $R^7$ are =S), while reaction of IX with formic acid provides the corresponding desthione (Formulae I and II, Q is -O-, $R^6$ and $R^7$ are each hydrogen). Compound IX can be prepared by known methods (see, e.g., Campaigne et al., J. Org. Chem., 26, 359 (1961); id., 26, 1326 (1961); Chakrabarti, et al., Tetrahedron, 25(14), 2781 (1969)), or upon heating Compound A with dilute aqueous base.

Compounds of Formulae I and II wherein Q is $NR^8$ and $R^8$ is $-(CH_2)_n$-Y (n=0) and Y is $NR^{11}R^{12}$, where $R^{11}$ and $R^{12}$ are as defined for Formula I, may be prepared according to the following reaction sequence:

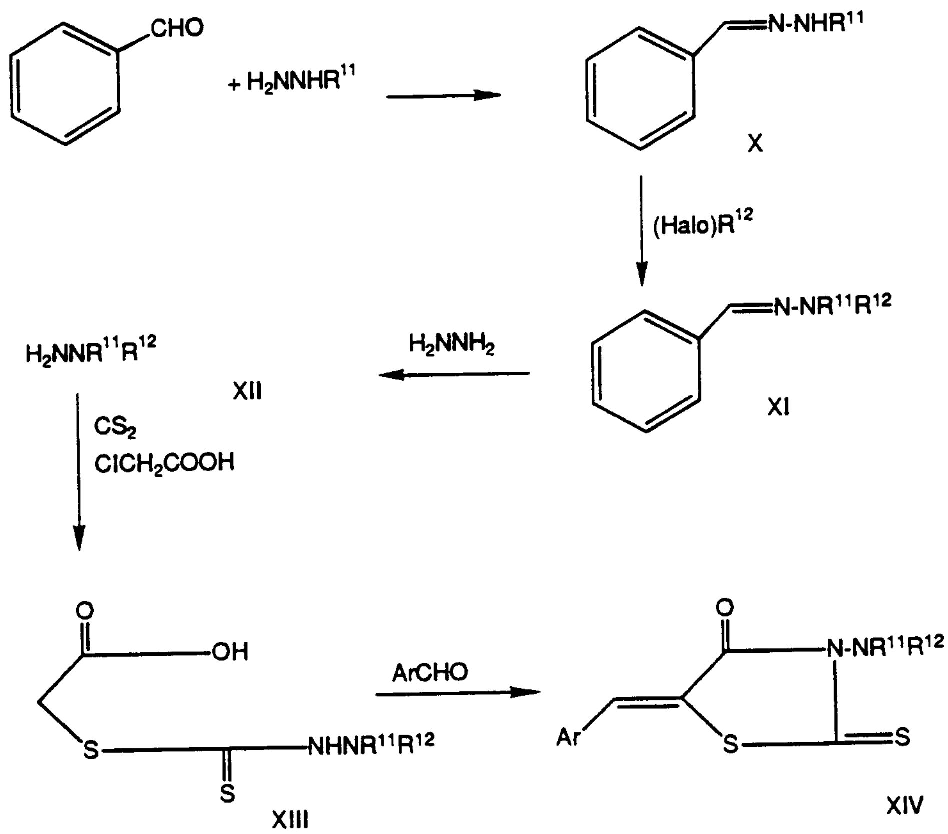


where Ar =

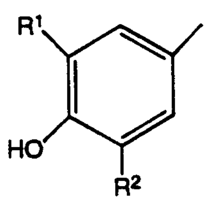

The $R^{11}$-substituted hydrazine is treated with benzaldehyde in an alcoholic (preferably methanol) solvent to yield intermediate X, which, in turn, is reacted with the appropriate $R^{12}$-halide in the presence of triethylamine and acetonitrile to render intermediate XI. XI is then treated with hydrazine to render the $R^{11}$,$R^{12}$-hydrazine, XII. XII may alternatively be prepared by reducing a nitroso-$R^{11}R^{12}$ amine using zinc dust and acetic acid or aluminum and a strong

base. The nitroso-$R^{11}R^{12}$ amine itself is prepared from an $R^{11}$,$R^{12}$ amine as described in J. Am. Chem. Soc., 77, 790 (1955) by treatment with sodium nitrite in HCl. XII is then treated with carbon disulfide, chloroacetic acid and triethylamine to yield intermediate XIII. Condensation of XIII with the appropriate $R^1$,$R^2$-substituted-4-hydroxybenzaldehyde (i.e., ArCHO) renders XIV. As described previously, the thione may be reduced by treatment with a reducing agent such as tri-n-butyl tin hydride in a non-reactive solvent such as toluene, preferably in the presence of a free radical initiator such as azobisisobutyronitrile. Preparation of the species wherein one of $R^{11}$ or $R^{12}$ is hydrogen may be effected before or after reduction of the thione, as desired, by heating the disubstituted compound in a mixture of ethanol/water in the presence of a catalyst, such as a rhodium catalyst.

Those compounds of Formulae I and II wherein X is

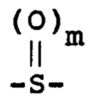

and m is 1 or 2 are readily prepared from the sulfide (i.e., m=0) by treatment with an oxidizing agent, such as m-chloroperbenzoic acid, in an appropriate organic solvent, such as chloroform, for a time sufficient to effect the desired oxidation.

Compounds of Formulae I and II wherein $R^3$ is $C_1$-$C_6$ alkyl are prepared by conventional Friedel-Crafts alkylation of the appropriate $R^1$, $R^2$-substituted phenol, followed by condensation with rhodanine, or the desired N-substituted rhodanine, as described herein, or is used as described in other reaction schemes depicted herein.

It will be readily appreciated by one skilled in the art that the aryl portion of the present compounds of Formulae I and II are either commercially available or may be readily prepared by known techniques from commercially available starting materials. For example, p-hydroxybenzaldehyde may be alkylated under Friedel-Crafts conditions to yield an alkylbenzaldehyde which in turn may itself be alkylated. Similarly, the rhodanine or N-substituted rhodanine starting material is either commercially available or may be prepared by well known methodology from commercially available starting materials.

Those compounds of Formulae I and II wherein one of $R^6$ or $R^7$ is hydrogen and the other is -OH (and X is

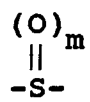

where m is 0) are conveniently prepared from their precursors of Formulae I and II where $R^6$ and $R^7$ are both hydrogen (and X is

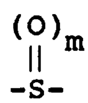

where m is 1) by treatment of the precursor with, for example, trifluoroacetic anhydride in an inert solvent (preferably methylene chloride) at reduced temperatures. Similarly, compounds of Formulae I and II where, in the definition of Q, Y is cyano are prepared by treating the non-cyanated analog with the desired halo-substituted aliphatic nitrile. From the cyano derivative, the tetrazolyl is prepared by treatment with tri-n-butyl tin azide in, for example, ethylene glycol dimethyl ether. Other compounds of Formulae I and II may be prepared as more fully described below from compounds whose synthesis was described generically, supra.

The use and compounds of the present invention encompass both the racemate and its individual stereoisomers. In general, the stereoisomers may be obtained according to procedures well known in the art. However, for compounds of Formulae I and II wherein X is -S-; $R^4$ and $R^5$ are hydrogen; and $R^1$, $R^2$, $R^3$, $R^6$, $R^7$ and Q are as defined for those Formulae, the individual stereoisomers may be isolated in substantially pure isomeric form according to the following novel process. In the following process, preferred compounds whose stereoisomers may be isolated are those compounds of Formulae I and II wherein X is -S-; $R^4$ and $R^5$ are hydrogen; and $R^1$, $R^2$, $R^3$, $R^6$, $R^7$ and Q are as defined for the preferred, somewhat preferred, particularly preferred, especially preferred and most preferred compounds of the use of the present invention.

The racemic sulfide compound of Formulae I or II is reacted with a reagent prepared by combining a tartrate ligand,

a titanium alkoxide, a hydroperoxide and, optionally, water. Suitable titanium alkoxides for use in the present process include titanium alkoxides having the formula Ti($C_1$-$C_4$ alkoxy)$_4$. A particularly preferred titanium alkoxide is one wherein the $C_1$-$C_4$ alkoxy group is isopropoxy. Similarly, suitable tartrate ligands for use in the present process include the di($C_1$-$C_4$ alkyl) tartrates, with diethyl tartrate or diisopropyl tartrate particularly preferred. Finally, suitable hydroperoxides which may be used in the present process include cumenehydroperoxide, t-butylhydroperoxide, and the like. A particularly preferred hydroperoxide is t-butylhydroperoxide.

The present reaction is conducted by mixing the above reagents in an inert solvent. Suitable inert solvents include aromatic solvents such as toluene and the like; halogenated alkanes such as methylene chloride, 1,2-dichloroethane, chloroform and the like; ethers such as tetrahydrofuran, diethyl ether and the like; and ketones such as acetone and the like. A particularly preferred inert solvent is methylene chloride. In general, the amount of solvent used should be sufficient to ensure that all compounds stay in solution during reaction. However, excessive amounts of solvent should be avoided since unnecessary product loss may occur during product isolation.

The amount of titanium alkoxide used in the present reaction is not critical. The titanium alkoxide may be employed in quantities of from about 0.4 equivalents to about 2.0 equivalents relative to the racemic sulfide starting material. For reasons explained more fully below, the titanium alkoxide is preferably employed in quantities sufficient to provide a titanium alkoxide/sulfide substrate ratio of from about 0.5/1.0 to about 0.75/1.0. If the titanium alkoxide is used in less than equimolar quantities relative to the sulfide starting material, 3Å- or 4Å- molecular sieves may be added, if desired, to avoid the possibility of water deactivation of the titanium complex.

The amount of tartrate ligand, hydroperoxide and water employed are based on the amount of titanium alkoxide used, and are also not critical. In general, the tartrate ligand is employed in quantities sufficient to provide a tartrate ligand/titanium alkoxide ratio of from about 1/1 to about 5/1, with a preferred ratio being about 2/1. Similarly, the hydroperoxide may be employed in from about equimolar quantities relative to the titanium alkoxide to about two equivalents relative to that same compound. The amount of water employed may vary from anhydrous reaction condition (i.e. no equivalents of water) to as much as about 5 equivalents of water relative to the amount of titanium alkoxide present. When anhydrous reaction condition are employed, the tartrate ligand should be used in an amount sufficient to provide a tartrate ligand/titanium alkoxide ratio corresponding to the higher end of the tartrate ligand/titanium alkoxide ratio described above.

The stereochemistry of the tartrate ligand determines which stereoisomer will be obtained from the racemic sulfide substrate. For example, if (+)-diisopropyltartrate is employed in the present reaction the (-) enantiomer of the sulfide starting material will be isolated in substantially pure isomeric form. Correspondingly, if (-)-diethyltartrate is used, substantially pure (+) enantiomer of the sulfide substrate will be obtained. Accordingly, the tartrate ligand must be chosen so that its stereochemistry is opposite that of the isomeric form desired.

The racemic sulfide substrate of the present process is reacted with the reagent prepared from the titanium alkoxide, the tartrate ligand, the hydroperoxide and, optionally, water until substantially all of the undesired enantiomer of the sulfide starting material has been converted to its sulfoxide analog. Conversion to the sulfoxide readily occurs at temperatures in the range of from about -50°C to about 50°C, with a preferred temperature being about -20°C. Once substantially all of the undesired enantiomer has been converted to its sulfoxide analog, the reaction is terminated by quenching the reaction mixture according to techniques well known in the art.

To ensure that substantially all of the undesired enantiomer is converted to the sulfoxide, while minimizing conversion of the desired enantiomer, only about 50 to about 70 percent of the racemic sulfide substrate should be allowed to react with the reagent containing titanium alkoxide. Limiting reaction to between about 50% to about 70% may be accomplished in at least two ways. Firstly, the hydroperoxide may be employed in quantities which provide a hydroperoxide/sulfide substrate ratio of from about 0.5/1.0 to about 0.75/1.0. Alternatively, the hydroperoxide may be used in amounts greater than about 0.75 equivalents relative to the sulfide substrate provided the progress of the reaction is monitored by standard analytical techniques such as thin layer chromatography (TLC) or high performance liquid chromatography (HPLC). Once these techniques indicate that between about 50 to about 70 percent of the sulfide starting material has been converted the reaction is quenched to prevent further conversion.

Once the reaction has been quenched the unreacted portion of the sulfide substrate may be recovered from the quenched reaction mixture using techniques well known to those skilled in the art. This unreacted portion will consist of the desired enantiomer in substantially pure enantiomeric form.

According to a final aspect of the invention, there is provided a process for preparing a novel compound of Formula II which comprises

(A) reacting a compound of the formula

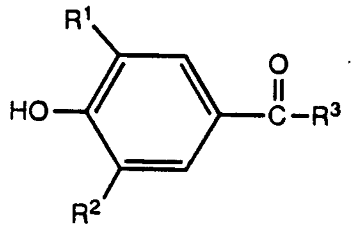

with a compound of the formula

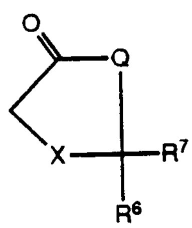

wherein $R^1$, $R^2$, $R^3$ and X are as in Formula II, Q is $-CH_2-$ or $NR^8$ (where $R^8$ is as defined in Formual II) and $R^6$ and $R^7$ taken together are =S, so as to provide a compound of the formula

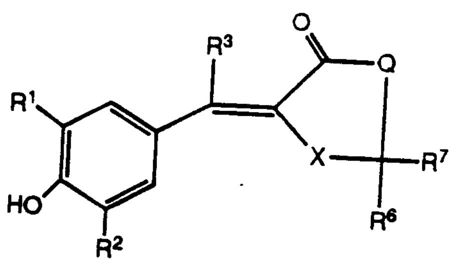

wherein $R^1$, $R^2$, $R^3$, $R^6$, $R^7$, X and Q are as set forth above;
(B) reducing a compound of Formula II wherein $R^6$ and $R^7$ taken together are =S so as to prepare a compound of Formula II in which $R^6$ and $R^7$ are hydrogen;
(C) reducing a compound of Formula II in which $R^4$ and $R^5$ taken Together form a bond so as to prepare a compound of Formula II in which $R^4$ and $R^5$ are hydrogen;
(D) reducing a compound of Formula II in which $R^4$ and $R^5$ taken together form a bond and $R^6$ and $R^7$ taken together are =S so as to prepare a compound of Formula II in which $R^4$, $R^5$, $R^6$ and $R^7$ are all hydrogen;
(E) alkylating a compound of Formula II in which $R^8$ is hydrogen so as to prepare a compound of Formula II in which $R^8$ is $C_1$-$C_6$ alkyl, $C_2$-$C_6$ alkenyl, $C_3$-$C_8$ cycloalkyl or $-(CH_2)_n-Y$ (where n is an integer from 0 to 3, both inclusive, and Y is cyano, $OR^9$, -SH, -S($C_1$-$C_4$ alkyl), $-NR^{11}R^{12}$ or

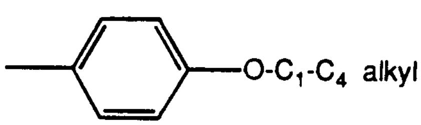

where $R^9$, $R^{11}$ and $R^{12}$ are as defined in Formula II);
(F) acylating a compound of Formula II in which $R^8$ is hydrogen so as to prepare a compound of Formula II in which $R^8$ is $-(CH_2)_n-Y$, where n is an integer from 0 to 3, both inclusive, and Y is

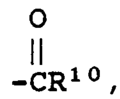

where $R^{10}$ is as defined in Formula II;
(G) oxidizing a compound of Formula II wherein X is

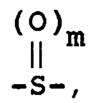

where m is 0, so as to prepare a compound of Formula II wherein X is

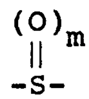

and m is 1;
(H) oxidizing a compound of Formula II wherein X is

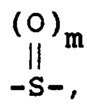

where m is 0, so as to prepare a compound of Formula II wherein X is

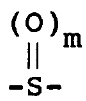

and m is 2;
(I) oxidizing a compound of Formula II wherein X is

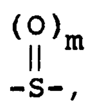

where m is 1, so as to prepare a compound of Formula II wherein X is

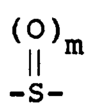

and m is 2;
(J) reacting a compound of the formula

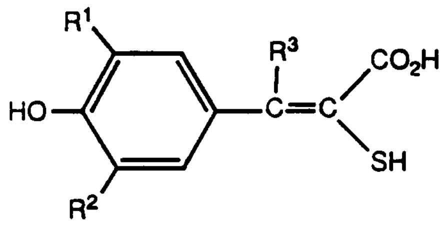

with

i) formic acid, so as to provide a compound of Formula II wherein Q is O, $R^4$ and $R^5$ taken together form a bond and $R^6$ and $R^7$ are hydrogen; or
ii) carbon disulfide, so as to provide a compound of Formula II wherein Q is O, $R^3$ and $R^4$ taken together form a bond and $R^6$ and $R^7$ taken together are =S;

(K) reacting a compound of the formula

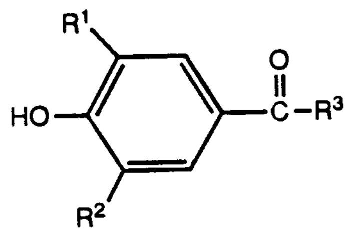

with a compound of the formula

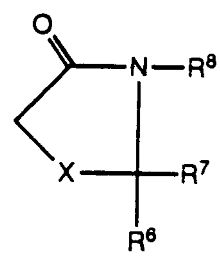

wherein $R^1$, $R^2$, $R^3$ and X are as defined in Formula II, $R^6$ and $R^7$ taken together are =S, and $R^8$ is $-(CH_2)_n-Y$ (where n is an integer from 0 to 3, both inclusive, and Y is $OR^9$, where $R^9$ is hydrogen) so as to provide a compound of the formula

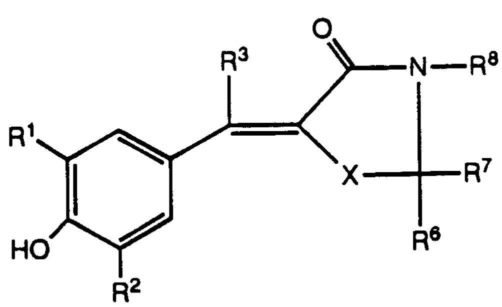

wherein $R^1$, $R^2$, $R^3$, $R^6$, $R^7$ and X are as set forth above and $R^8$ is $-(CH_2)_n-Y$ (where n is an integer from 0 to 3, both inclusive, and Y is $OR^9$, where $R^9$ is

$$\overset{\displaystyle O}{\overset{||}{-C}}-CH_3);$$

(L) reducing a compound of Formula II in which $R^8$ is $-(CH_2)_n$-Y wherein n is 0 to 3, both inclusive, and Y is $OR^9$, where $R^9$ is

$$\overset{\displaystyle O}{\overset{||}{-C}}-C_1-C_4 \text{ alkyl},$$

so as to prepare a compound of Formula II in which $R^8$ is $-(CH_2)_n$-Y, wherein n is 0 to 3, both inclusive, and Y is $OR^9$, where $R^9$ is hydrogen;

(M) reacting a compound of Formula II in which $R^8$ is $-(CH_2)_n$-Y, wherein n is 0 to 3, both inclusive, and Y is $-OR^9$, where $R^9$ is hydrogen, with a tosyl-halide so as to prepare a compound of Formula II in which $R^8$ is $-(CH_2)_n$-Y, wherein n is 0 to 3, both inclusive, and Y is $OR^9$, where $R^9$ is tosyl;

(N) reacting a compound of Formula II in which $R^8$ is $-(CH_2)_n$-Y, wherein n is 0 to 3, both inclusive, and Y is $-OR^9$, where $R^9$ is tosyl, with an amine of the formula $HNR^{11}R^{12}$ (where $R^{11}$ and $R^{12}$ are as defined for Formula II) so as to prepare a compound of Formula II in which $R^8$ is $-(CH_2)_n$-Y, wherein n is 0 to 3, both inclusive, and Y is $-NR^{11}R^{12}$;

(O) treating a compound of Formula II in which $R^8$ is $-(CH_2)_n$-Y, wherein n is 0 to 3, both inclusive, and Y is cyano with tri-n-butyl tin azide so as to prepare a compound of Formula II in which $R^8$ is $-(CH_2)_n$-Y, wherein n is 0 to 3, both inclusive, and Y is tetrazolyl;

(P) reacting a compound of the formula

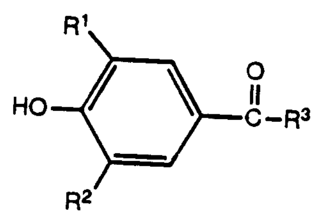

with a compound of the formula

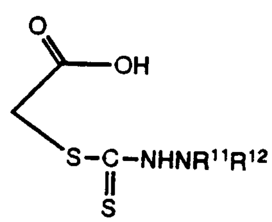

wherein $R^1$, $R^2$, $R^3$, $R^{11}$ and $R^{12}$ are as in Formula II, so as to provide a compound of the formula

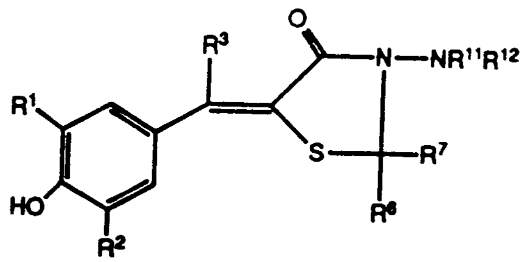

wherein $R^6$ and $R^7$ taken together are =S and $R^1$, $R^2$, $R^3$, $R^{11}$ and $R^{12}$ are as defined in Formula II;

(Q) heating a compound of Formula II in which $R^8$ is $-(CH_2)_n-Y$ and Y is $-NR^{11}R^{12}$ (neither of $R^{11}$ or $R^{12}$ being hydrogen) in an ethanol/water mixture in the presence of a catalyst so as to prepare a compound of Formula II in which $R^8$ is $-(CH_2)_n-Y$ and Y is $-NR^{11}R^{12}$ (where one of $R^{11}$ or $R^{12}$ is hydrogen and the other is not hydrogen);

(R) reacting a compound of Formula II in which $R^6$ and $R^7$ are both hydrogen with trifluoroacetic anhydride so as to prepare a compound of Formula II in which one of $R^6$ and $R^7$ is hydrogen and the other is -OH;

(S) salifying a compound of Formula II by reacting the non-salt form of the compound with either a strong acid or a strong base.

The following examples further illustrate the preparation of the compounds of this invention, as well as the compounds used in this invention. The examples also illustrate the process for selective enantiomeric isolation provided by the present invention.

Example 1

5-[[3,5-Bis(1,1-dimethylethyl)-4-hydroxyphenyl]methylene]-2-thioxo-4-thiazolidinone (Compound A)

Under a nitrogen atmosphere, 117.2 g of 3,5-di-tert-butyl-4-hydroxybenzaldehyde, 66.6 g of rhodanine, and 143.5 g of fused sodium acetate were heated at reflux in 2500 ml of glacial acetic acid. After heating for 23 hours, the reaction mixture was cooled and poured into a mixture of 1 liter of ethanol and 1 liter of ice, with stirring. Water (500 ml) was added and, after stirring for 30 minutes, the resulting precipitate was recovered by filtration. The solid was slurried with 500 ml of ethyl acetate and filtered. The precipitate was dissolved in 3 liters of ethanol, heated to boiling, and water was added until the solution remained cloudy (approximately 450 ml of water). Upon cooling to room temperature, 99.6 g of title product were recovered by filtration, m.p. approximately 260°C.

| Analysis for $C_{18}H_{23}NO_2S_2$: | | | |
|---|---|---|---|
| Calculated: | C, 61.86; | H, 6.63; | N, 4.01; |
| Found: | C, 62.13; | H, 6.55; | N, 4.15. |

Examples 2-3

5-[[3,5-Bis(1,1-dimethylethyl)-4-hydroxyphenyl]methylene]-4-thiazolidinone (Compound B) and 5-[[3,5-Bis(1,1-dimethylethyl)-4-hydroxyphenyl]methyl]-4-thiazolidinone (Compound C)

A solution of 69.90 g of 5-[[3,5-bis(1,1-dimethylethyl)-4-hydroxyphenyl]methylene]-2-thioxo-4-thiazolidinone in 4 liters of ethanol was hydrogenated at 500 pounds per square inch in the presence of 200 g of 5% palladium on carbon overnight at 100°C. The reaction mixture was filtered and evaporated to dryness. In sections, the material was dissolved in 1 volume of hot ethyl acetate, diluted with 2 volumes of hexane, filtered, and loaded onto a silica gel chromatography column. Elution with 35% ethyl acetate in hexane provided various fractions which were combined according to the purities of the respective compounds. A total of 4.6 g of Compound B were isolated by chromatography. Fractions which were predominantly Compound B were crystallized from ethyl acetate/hexane providing a total yield of Compound B of 13.79 g. Rechromatography of fractions containing impure Compound C on silica eluting with 25% ethyl acetate in hexane provided 9.82 g of Compound C.

2. 5-[[3,5-bis(1,1-dimethylethyl)-4-hydroxyphenyl]methylene]-4-thiazolidinone, m.p. 209-213°C.

| Analysis for $C_{18}H_{25}NO_2S$: | | | |
|---|---|---|---|
| Calculated: | C, 67.67; | H, 7.89; | N, 4.38; |
| Found: | C, 67.44; | H, 8.11; | N, 4.65. |

3. 5-[[3,5-bis(1,1-dimethylethyl)-4-hydroxyphenyl]methyl]-4-thiazolidinone, m.p. 149-152°C.

| Analysis for $C_{18}H_{27}NO_2S$: | | | |
|---|---|---|---|
| Calculated: | C, 67.25; | H, 8.47; | N, 4.36; |
| Found: | C, 67.43; | H, 8.44; | N, 4.21. |

Example 4

5-[[3,5-Bis(1,1-dimethylethyl)-4-hydroxyphenyl]methyl]-2-thioxo-4-thiazolidinone (Compound D)

Under a nitrogen atmosphere, 13.98 g of 5-[[3,5-bis(1,1-dimethylethyl)-4-hydroxyphenyl]methylene]-2-thioxo-4-thiazolidinone, 13.17 g of diethyl 2,6-dimethyl-1,4-dihydro-3,5-pyridinedicarboxylate and 600 ml of toluene were stirred to effect solution. Forty grams of silica gel 60 (finer than 230 mesh), previously dried in vacuo at 50°C for 7 hours, were added to the reaction. The reaction was heated at reflux for 18 hours and filtered hot. The filtrate was evaporated to dryness. The residue was dissolved in 500 ml of ethyl acetate, washed 5 times each with 400 ml of 1N hydrochloric acid, dried over sodium sulfate, filtered, and evaporated in vacuo to provide a yellow solid. Chromatography over silica gel eluting with 2.5% ethyl acetate in toluene provided 8.0 g of title product, m.p. 178-179°C.

| Analysis for $C_{18}H_{25}NO_2S_2$: | | | |
|---|---|---|---|
| Calculated: | C, 61.50; | H, 7.17; | N, 3.98; |
| Found: | C, 61.28; | H, 7.19; | N, 3.94. |

Example 5

5-[[3,5-Bis(1,1-dimethylethyl)-4-hydroxyphenyl]methylene]-3-methyl-2-thioxo-4-thiazolidinone

The title compound was prepared in 76% yield from 3,5-di-tert-butyl-4-hydroxybenzaldehyde and N-methylrhodanine following the procedure of Example 1, m.p. >230°C.

| Analysis for $C_{19}H_{25}NO_2S_2$: | | | | |
|---|---|---|---|---|
| Calculated: | C, 62.77; | H, 6.93; | N, 3.85; | S, 17.64; |
| Found: | C, 62.54; | H, 7.05; | N, 3.66; | S, 17.82. |

Example 6

5-[[3,5-Bis(1,1-dimethylethyl)-4-hydroxyphenyl]methylene]-3-methyl-4-thiazolidinone

The title compound was prepared in 71% yield from 10.31 g of the thione of Example 5 upon heating with 38.15 ml of tri-n-butyltin hydride and 1.16 g of azobisisobutyronitrile (AIBN) in 142 ml of toluene at reflux temperature for one hour. The product was isolated by adding water to the cooled reaction mixture, separating the layers, washing the organic layer with 1N hydrochloric acid and a saturated sodium chloride solution, drying over magnesium sulfate, concentrating in vacuo, and purifying the residue by chromatography over silica gel eluting with a 10-50% hexane in ethyl acetate gradient. The purified product had a melting point of 142-144°C.

| Analysis for $C_{19}H_{27}NO_2S$: | | | |
|---|---|---|---|
| Calculated: | C, 68.43; | H, 8.16; | N, 4.20; |
| Found: | C, 68.68; | H, 8.00; | N, 3.97. |

Example 7

5-[[3,5-Bis(1,1-dimethylethyl)-4-hydroxyphenyl]methyl]-3-methyl-4-thiazolidinone

To 100 ml of THF were added 6.43 g of the compound of Example 3. Sodium hydride (0.9 g) was added, resulting in the evolution of a gas. Iodomethane (1.25 ml, 1.0 eq.) was added and the resultant mixture was stirred at room temperature for 23 hours after which the mixture was diluted with a volume of diethyl ether and 1N HCl. The organic layer was separated and dried over sodium sulfate, filtered and evaporated. The resultant solid was chased with chloroform to render an orange foam. A 5.93 g sample of this material was dissolved in 14 ml of a hot mixture of ethyl acetate diluted with 225 ml of hexane and then allowed to cool to room temperature overnight. The solvent was evaporated and the resultant solid was dissolved in 40 ml of a hot mixture of diethyl ether diluted with about 400 ml of hexane. The mixture was allowed to cool to room temperature overnight and a precipitate formed which was collected by filtration, washed with hexane and dried in vacuo to render 3.98 g of title compound, m.p. 102°-105°C.

| Analysis for $C_{19}H_{29}NO_2S$: | | | |
|---|---|---|---|
| Calculated: | C, 68.02; | H, 8.71; | N, 4.17; |
| Found: | C, 68.22; | H, 8.80; | N, 4.21. |

Example 8

5-[[3,5-Bis(1,1-dimethylethyl)-4-hydroxyphenyl]methylene]-3-dimethylamino-2-thioxo-4-thiazolidinone

The title compound was prepared in 65% yield from 3,5-di-tert-butyl-4-hydroxybenzaldehyde and N-dimethylaminorhodanine following the procedure of Example 1.

Example 9

5-[[3,5-Bis(1,1-dimethylethyl)-4-hydroxyphenyl]methylene]-3-dimethylamino-4-thiazolidinone

The compound of Example 8 was reduced using the procedure of Example 6 to provide the title compound in 41% yield, m.p. 138-141°C.

| Analysis for $C_{20}H_{30}N_2O_2S$: | | | |
|---|---|---|---|
| Calculated: | C, 66.26; | H, 8.34; | N, 7.73; |
| Found: | C, 66.55; | H, 8.59; | N, 7.47. |

Example 10

5-[[3,5-Bis(1,1-dimethylethyl)-4-hydroxyphenyl]methylene]-3-(methylamino)-4-thiazolidinone

A. Preparation of benzaldehydemethylhydrazone

Benzaldehyde (50.8 ml, 500 mmol) and 26.5 ml (500 mmol) of methylhydrazine were dissolved in 1 liter of methanol. The mixture was stirred together at room temperature for 75 minutes and then stripped of solvent to give 67.8 g of the subtitled intermediate.

B. Preparation of benzaldehyde N-methyl, N-2-propenylhydrazone

The above compound (67.8 g), 60.5 g of allyl bromide and 50.5 g of triethylamine were dissolved together in 1 liter of acetonitrile. The mixture was heated at reflux temperature for 16 hours and then cooled. An additional 45 g of allyl bromide and 38 g of triethylamine were added and the mixture was again heated at reflux for an additional 7 hours, cooled and then stripped of solvent to yield 268 g of a residue. To this residue was added 500 ml of THF and the resultant mixture was shaken, filtered and washed with an additional 125 ml of THF. The filtrate was stripped of solvent to yield 67 g of the subtitled intermediate.

C. Preparation of N-methyl, N-2-propenyl-hydrazine

The above compound (59.9 g), 44 g of hydrazine and 137 ml of ethanol were heated at reflux temperature for 21.5 hours and allowed to cool. The reflux condenser was replaced with a distillation head and the mixture was distilled at one atmosphere pressure. The first three distillates were collected, combined and 100 ml of 1N HCl were added. An additional 100 ml of concentrated HCl were added, with ice, and the resultant mixture separated and washed with a small amount of ethyl acetate. The resultant layers were separated and the water distilled off until solids clogged the stir bar. The solids were filtered off and the filtrate was stripped and added to 125 ml of chilled 50% NaOH. The resulting solid was filtered off and discarded. The filtrate contained two layers which were separated. The top layer contained the subtitled intermediate and the bottom, aqueous layer, was extracted with diethyl ether which, upon stripping, gave additional product.

D. Preparation of N-Methyl, N-3-propenyl-5-carboxymethyl-dithiocarbamate

To 12.67 g of the above compound in 23 ml of ethanol chilled to 0°C was added a solution of 11.18 g of carbon disulfide in 26 ml of diethyl ether. The resultant mixture was removed from the ice bath and allowed to stand at room temperature for about 15.5 hours, after which the solvent was stripped to yield approximately 36.5 g of a residue. To this residue was added 13.9 g of chloroacetic acid dissolved in 29.5 ml of 5N NaOH (chilled in an ice bath). The resultant solution was allowed to stand for 3 hours at room temperature. The pH of the solution was lowered to about 3 by adding 8 ml of concentrated hydrochloric acid. Diethyl ether (50 ml) was then added, resulting in a three phase separation. The aqueous phases were pooled and extracted with 50 ml of chloroform, then stripped of solvent to yield approximately 40.4 g of the subtitled intermediate.

E. Preparation of 5-[[3,5-bis(1,1-dimethylethyl)-4-hydroxyphenyl]methylene]-2-thioxo-3-(methyl-2-propenylamino)-4-thiazolidinone

3,5-Di-tert-butyl-4-hydroxybenzaldehyde (29.3 g), 38.8 g of the above compound and 40.34 g of sodium acetate were mixed in 810 ml of acetic acid and the resultant solution heated at reflux temperature for 24 hours. The solution was allowed to cool and stirred for an additional 60 hours at room temperature. The solution was then poured into 2 liters of ice water, separated and washed with an additional volume of water to yield about 44 g of the subtitled intermediate.

F. Preparation of 5-[[3,5-bis(1,1-dimethylethyl)-4-hydroxyphenyl]methylene]-3-(methyl-2-propenylamino)-4-thiazolidinone

Utilizing the procedure described in Example 6, 42.8 g of the above thione were reduced to the subtitled intermediate (8.34 g).

G. Preparation of 5-[[3,5-bis(1,1-dimethylethyl)-4-hydroxyphenyl]methylene]-3-(methylamino)-4-thiazolidinone

The above compound (6.11 g) was dissolved in a mixture of 135 ml ethanol and 15.3 ml of water and the mixture was heated to 70°C. Tris-(triphenylphosphine)-rhodium (I) chloride (50 mg) was added and the mixture heated at reflux temperature for 50 minutes, after which an additional 550 mg of the catalyst were added followed by heating at reflux temperature for an additional 2.5 hours. The mixture was cooled and stirred at room temperature overnight and stripped of solvent to give 2.05 g of title product after further workup, m.p. 151-153.5°C.

| Analysis for $C_{19}H_{28}N_2O_2S$: | | | |
|---|---|---|---|
| Calculated: | C, 65.86; | H, 7.56; | N, 8.09; |
| Found: | C, 65.67; | H, 7.81; | N, 8.34. |

<u>Examples 11 and 12</u>

5-[(3,5-Di-2-propenyl-4-hydroxyphenyl)methylene]-4-thiazolidinone and 5-[(3,5-Di-2-propenyl-4-hydroxyphenyl)methyl]-4-thiazolidinone

A. Preparation of 3,5-di-(2-propenyl)-4-hydroxybenzaldehyde

Under a nitrogen atmosphere and using a mechanical stirrer, 250 g of parahydroxybenzaldehyde, 247.6 g of allyl bromide, 311.7 g of potassium bicarbonate and 650 ml of acetone were heated to reflux temperature for about 18 hours. The mixture was allowed to cool, after which about 1 liter of water was added followed by extraction with two 800 ml portions of diethyl ether. Subsequent distillation of the organic phase rendered about 299 g of 4-(2-propenyl)oxybenzaldehyde which was then heated with about 300 ml of diethylaniline for 5.5 hours at 195-205°C. The mixture was cooled and 750 ml of ethyl acetate were added. The mixture was washed with three 500 ml portions of 1N HCl which, followed by subsequent workup, yielded about 138 g of 3-(2-propenyl)-4-hydroxybenzaldehyde. The mono-substituted aldehyde (159 g) was again heated to reflux with 152 g of potassium carbonate and 465 ml of acetone for 3 hours and then allowed to cool. The mixture was poured into 900 ml of ice water and subsequently extracted with two 430 ml portions of diethyl ether to yield about 170 g of 3-(2-propenyl)-4-(2-propenyloxy)-benzaldehyde. The di-substituted aldehyde was then heated, in about 500 ml of diethylaniline, under a nitrogen atmosphere to 195-205°C for about 6.5 hours. The mixture was cooled and dissolved in about 800 ml of ethyl acetate, washed with three 1 liter portions of 1N HCl and, following workup, rendered about 121.9 g of the subtitled intermediate.

B. Preparation of 5-[(3,5-di-2-propenyl-4-hydroxyphenyl)methylene]-2-thioxo-4-thiazolidinone

The above compound (50.5 g), 36.6 g of rhodanine and 164 g of sodium acetate were heated together at reflux temperature in 1.25 liters of acetic acid for 14.5 hours. The resultant solution was cooled, poured into 2 liters of ice water to yield, upon separation, about 75 g of the subtitled intermediate, m.p. 157-160°C.

C. Preparation of 5-[(3,5-di-2-propenyl-4-hydroxyphenyl]methylene]-4-thiazolidinone and 5-[(3,5-di-2-propenyl-4-hydroxyphenyl)methyl]-4-thiazolidinone

The above compound (74.8 g) was reduced by treatment with zinc dust (62 g) and concentrated hydrochloric acid (950 ml) in 2.1 liters of hot (approximately 82°C) ethanol. Once the reactants were combined the solution was allowed to cool to room temperature, stirred for one hour, and then added to 3.75 liters of ice water. The resulting solution was allowed to sit overnight to provide a gum. The liquid layer was decanted and extracted with 750 ml of chloroform, while

the gum was dissolved in 560 ml of chloroform and the resulting solution washed, successively, with 75 ml of a saturated sodium carbonate solution, 75 ml of water and 75 ml of a saturated brine solution. The above chloroform solutions were combined and then triturated with 100 ml of methylene chloride. The titled products were obtained using silica gel chromatography. Elution with a 25-60% ethyl acetate in hexane gradient provided various fractions which were treated as follows.

Fractions 13-15 were concentrated and then washed with ethyl acetate to provide 2.91 g of 5-[(3,5-di-2-propenyl-4-hydroxyphenyl)methyl]-4-thiazolidinone. Fractions 16-18 were concentrated to a residue which was triturated with 30 ml of methylene chloride. Fractions 19-23 were concentrated to a residue which was triturated with 35 ml of methylene chloride. Following trituration, the remaining insolubles were isolated by filtration and triturated with 40 ml of ethyl acetate to provide 3.85 g of 5-[(3,5-di-2-propenyl-4-hydroxyphenyl)methylene]-4-thiazolidinone.

The ethyl acetate wash from fractions 13-15, the methylene chloride solution containing fractions 16-18 and the methylene chloride and ethyl acetate solutions obtained from fractions 19-23 were combined and loaded onto a silica gel chromatography column. Elution with a 1:1 ethyl acetate/hexane solution provided various fractions which were combined according to the purities of the respective compounds. Fractions which were predominately 5-[(3,5-di-2-propenyl-4-hydroxyphenyl)methyl]-4-thiazolidinone were crystallized from hot ethyl acetate to provide 1.24 g of that compound (total yield of 5-[(3,5-di-2-propenyl-4-hydroxyphenyl)methyl]-4-thiazolidinone - 4.14 g). Fractions which were predominately 5-[(3,5-di-2-propenyl-4-hydroxyphenyl)methylene]-4-thiazolidinone were triturated with 30 ml of hot ethyl acetate to provide 1.73 g of that compound (total yield of 5-[(3,5-di-2-propenyl-4-hydroxyphenyl)methylene]-4-thiazolidinone - 5.58 g).

11. 5-[(3,5-di-2-propenyl-4-hydroxyphenyl)methylene]-4-thiazolidinone, m.p. 184-188°C

| Analysis for $C_{16}H_{17}NO_2S$: | | | |
|---|---|---|---|
| Calculated: | C, 66.87; | H, 5.96; | N, 4.87; |
| Found: | C, 66.62, | H, 5.92; | N, 4.89. |

12. 5-[3,5-di-2-propenyl-4-hydroxyphenyl)methyl]-4-thiazolidinone, m.p. 142-144°C

| Analysis for $C_{16}H_{19}NO_2S$: | | | |
|---|---|---|---|
| Calculated: | C, 66.41; | H, 6.62; | N, 4.84; |
| Found: | C, 66.18; | H, 6.69; | N, 4.60. |

Utilizing the procedures set forth in Examples 11, 12, and elsewhere herein, the following additional compounds were prepared.

Example 13

5-[(3,5-Di-2-propenyl-4-hydroxyphenyl)methylene]-3-methyl-4-thiazolidinone, m.p. 155-159°C

| Analysis for $C_{17}H_{19}NO_2S$: | | | |
|---|---|---|---|
| Calculated: | C, 67.74; | H, 6.35; | N, 4.65; |
| Found: | C, 67.53; | H, 6.09; | N, 4.45. |

Example 14

5-[(3,5-Dipropyl-4-hydroxyphenyl)methylene]-3-methyl-4-thiazolidinone, m.p. 162-165°C.

| Analysis for $C_{17}H_{23}NO_2S$: | | | |
|---|---|---|---|
| Calculated: | C, 66.85; | H, 7.59; | N, 4.59; |
| Found: | C, 67.12; | H, 7.37; | N, 4.52. |

Example 15

5-[(3,5-Dipropyl-4-hydroxyphenyl)methylene]-4-thiazolidinone, m.p. 202-205°C

| Analysis for $C_{16}H_{21}NO_2S$: | | | |
|---|---|---|---|
| Calculated: | C, 65.95; | H, 7.26; | N, 4.81; |
| Found: | C, 66.16; | H, 7.49; | N, 4.79. |

Example 16

5-[(3,5-Dipropyl-4-hydroxyphenyl)methyl]-4-thiazolidinone, m.p. 155-157°C

| Analysis for $C_{16}H_{23}NO_2S$: | | | |
|---|---|---|---|
| Calculated: | C, 65.49; | H, 7.90; | N, 4.77; |
| Found: | C, 65.71; | H, 7.73; | N, 4.99. |

Example 17

5-[[3-(1,1-Dimethylethyl)-4-hydroxy-5-methylphenyl]methylene]-4-thiazolidinone.

A. Preparation of 4-hydroxy-3-methyl-5-(1,1-dimethylethyl)benzaldehyde

Under a nitrogen atmosphere, 76.65 g of 2-(1,1-dimethylethyl)-6-methylphenol (Aldrich), 65.42 g of hexamethylenetetramine and 700 ml of trifluoroacetic acid were stirred at reflux temperature for about 24 hours. The reaction solution was allowed to cool and the liquid removed by evaporation. The resulting residue was taken up in 1500 ml of water and 1000 ml of chloroform and then neutralized to pH 7 with solid sodium carbonate. The resultant layers were separated and the aqueous layer was washed with chloroform. The organic layer was combined with the chloroform wash and the resulting solution was washed with water, then dried over sodium sulfate overnight. After removal of the sodium sulfate the chloroform was evaporated. The resultant residue was taken up in 375 ml of toluene, heated on a steam bath and then allowed to cool to room temperature overnight. Subsequent workup gave 28.3 g of the subtitled intermediate.

B. Preparation of 5-[[3-(1,1-dimethylethyl)-4-hydroxy-5-methylphenyl]methylene]-2-thioxo-4-thiazolidinone

The above intermediate (28.3 g), 24 g of N-aminorhodanine, 48.3 g of sodium acetate in 735 ml of acetic acid were heated to reflux temperature for about 7 hours and then allowed to cool to room temperature with continual stirring overnight. The resultant mixture was poured into 1500 ml of ice water with stirring and then filtered. The wet filter cake was transferred to a beaker and dissolved in a mixture of ethyl acetate and water. The resulting organic and aqueous layers were separated. The organic layer was dried over sodium sulfate and then filtered to remove that substance. Further

workup, followed by trituration in hot chloroform and subsequent drying under vacuum, rendered about 18 g of the subtitled intermediate, m.p. 210-216°C.

C. Preparation of 5-[[3-(1,1-dimethylethyl)-4-hydroxy-5-methylphenyl]methylene]-4-thiazolidinone.

Reduction of the above thione was effected by methods described herein which, following workup, rendered 1.56 g of the titled product, m.p. 162-165°C.

| Analysis for $C_{15}H_{19}NO_2S$: | | | |
|---|---|---|---|
| Calculated: | C, 64.95; | H, 6.90; | N, 5.05; |
| Found: | C, 65.12; | H, 7.05; | N, 4.99. |

Utilizing the procedures set forth in Example 17, and elsewhere herein, the following additional compounds were prepared.

Example 18

5-[[3,5-Bis(1-methylethyl)-4-hydroxyphenyl]methylene]-3-methyl-4-thiazolidinone, m.p. 200-210°C.

| Analysis for $C_{17}H_{23}NO_2S$: | | | |
|---|---|---|---|
| Calculated: | C, 66.85; | H, 7.59; | N, 4.59; |
| Found: | C, 67.03; | H, 7.55; | N, 4.37. |

Example 19

5-[[3,5-Bis(1-methylethyl)-4-hydroxyphenyl]methyl]-2-thioxo-4-thiazolidinone

Example 20

5-[[3-(1,1-Dimethylethyl)-4-hydroxy-5-methylphenyl]methyl]-4-thiazolidinone

A solution of 0.28 g of the compound of Example 17 in 30 ml of tetrahydrofuran was hydrogenated at 60 pounds per square inch in the presence of 1.12 g of 5% palladium on carbon overnight at 60°C. The reaction mixture was filtered and evaporated to dryness. The resulting residue was dissolved in 3.5 ml of a 1:1.5 ethyl acetate/hexane solution and loaded onto a silica gel chromatography column. Elution with 40% ethyl acetate in hexane produced fractions which, upon evaporation to dryness, provided 0.05 g of title compound. m.p. 64-68°C.

| Analysis for $C_{15}H_{21}NO_2S$: | | | |
|---|---|---|---|
| Calculated: | C, 64.48; | H, 7.58; | N, 5.01; |
| Found: | C, 64.32; | H, 7.66; | N, 4.79. |

Example 21

5-[[3,5-Bis(1-methylethyl)-4-hydroxyphenyl]methyl]-4-thiazolidinone

Using the method described in Example 20, 4.73 g of the compound of Example 19 were converted to 1.88 g of

title compound. m.p. 136-139°C.

| Analysis for $C_{16}H_{23}NO_2S$: | | | |
|---|---|---|---|
| Calculated: | C, 65.49; | H, 7.90; | N, 4.77; |
| Found: | C, 65.79; | H, 7.90; | N, 4.81. |

Example 22

5-[[3-(1,1-Dimethylethyl)-4-hydroxy-5-propylphenyl]methyl]-4-thiazolidinone

A. Preparation of 3-[2-(1,1-dimethylethyl)phenoxypropene

Allyl bromide (69.2 ml), 2-t-butylphenol (122.9 ml) and potassium carbonate (121.6 g) were stirred in 265 ml of acetone at reflux temperature for 50 hours and then cooled to 35°C. Water (600 ml) was added and the resulting layers were separated. The aqueous layer was extracted with 600 ml of diethyl ether. The organic layer was combined with the aqueous layer's ether extract and the resulting solution was dried over sodium sulfate overnight. After sodium sulfate removal, the solvent was evaporated to provide, after further workup, 147 g of the subtitled intermediate.

B. Preparation of 2-(1,1-dimethylethyl)-6-(2-propenyl)phenol

All 147 g of the above compound were rearranged as described in Examples 11A and 12A to provide 100.8 g of the subtitled intermediate.

C. Preparation of 2-(1,1-dimethylethyl)-6-propylphenol.

A solution of 54.9 g of the above compound in 575 ml of toluene was hydrogenated at 60 pounds per square inch in the presence of 55 g of Raney nickel for 3 hours at room temperature. The reaction mixture was filtered and evaporated to dryness to provide 59.2 g of the subtitled intermediate.

D. Preparation of 3-(1,1-dimethylethyl)-4-hydroxy-5-propylbenzaldehyde.

The above compound (55.48 g) was converted to 23.33 g of the subtitled intermediate using the method described in Example 17A.

E. Preparation of 5-[[3-(1,1-dimethylethyl)-4-hydroxy-5-propylphenyl]methylene]-2-thioxo-4-thiazolidinone.

Using the method described in Example 17B, 5.51 g of the above compound were converted to 6.26 g of the subtitled intermediate m.p. 190.5 - 192° C.

F. Preparation of 5-[[3-(1,1-dimethylethyl-4-hydroxy-5-propylphenyl]methyl]-2-thioxo-4-thiazolidinone.

Using the method described in Example 4, 4.73 g of the above compound were converted to 2.1 g of the subtitled intermediate.

G. Preparation of 5-[[3-(1,1-dimethylethyl-4-hydroxy-5-propylphenyl]methyl]-4-thiazolidinone.

A solution of 2.1 g of the above compound in 185 ml of ethanol was hydrogenated at 500 pounds per square inch in the presence of 8.4 g of 5% palladium on carbon for 20 hours at 100° C. The reaction mixture was filtered and evaporated to dryness. The resulting residue was dissolved in 25 ml of methylene chloride and loaded onto a silica gel chromatography column. Elution with 2000 ml of a 10-50% ethyl acetate in hexane gradient, followed by elution with 2000 ml of a 1:1 ethyl acetate/hexane solution, provided fractions which, upon evaporation to dryness, rendered 0.75 g of titled product. m.p. 50-55° C.

| Analysis for $C_{17}H_{25}NO_2S$: | | | |
|---|---|---|---|
| Calculated: | C, 66.41; | H, 8.20: | N, 4.56; |
| Found: | C, 66.61; | H, 8.22; | N, 4.55. |

Example 23

5-[[3-Methylthiophenyl-4-hydroxy-5-ethoxyphenyl]methylene]-3-dimethylamino-4-thiazolidinone.

A. Preparation of 5-[[3-ethoxy-4-hydroxyphenyl]methylene]-3-dimethylamino-2-thioxo-4-thiazolidinone.

3-Ethoxy-4-hydroxybenzaldehyde (45.7 g), N-dimethylaminorhodanine (53.35 g) and fused sodium acetate (92.4 g) were reacted in the manner described in Example 1 to provide 52.92 g of the subtitled intermediate. m.p. 194-198° C.

B. Preparation of 5-[[3-ethoxy-4-hydroxyphenyl]methylene]-3-dimethylamino-4-thiazolidinone.

Using the method described in Example 6, 47.66 g of the above compound were converted to 14.02 g of the subtitled intermediate.

C. Preparation of 5-[[3-ethoxy-4-hydroxy-5-(methylthiophenyl)phenyl]methylene]-3-dimethylamino-4-thiazolidinone.

Sodium hydroxide (0.95 g) and 17.3 ml of a 40% by weight solution of formaldehyde were dissolved in 50 ml of 2-ethoxyethanol. Phenylthiol (2.62 g) and 7.0 g of the above compound were added and the resulting solution was refluxed for 4 hours, then cooled. Ethyl acetate (50 ml) and water (25 ml) were added to the cooled reaction mixture and the pH of the resulting two-phase solution was lowered to approximately 5 using concentrated hydrochloric acid. The organic phase was separated from the aqueous phase, washed with a saturated brine solution and then loaded onto a silica gel chromatography column. Elution with 4 liters of methylene chloride, followed by 4 liters of a 3% methanol/97% methylene chloride solution, provided fractions containing the title product. These fractions were combined and loaded once more onto a silica gel chromatography column. Elution with 4 liters of methylene chloride, followed by 1 liter of a 22.5% acetonitrile in methylene chloride solution, provided fractions which, upon evaporation of the solvent, rendered title product. This product was further purified by trituration with a hot solution of 50 ml of hexane and 30 ml of ethyl acetate to provide 6.20 g of 5-[[3-methylthiophenyl-4-hydroxy-5-ethoxyphenyl]methylene]-3-dimethylamino-4-thiazolidinone. m.p. 118-120°C.

| Analysis for $C_{21}H_{24}N_2O_3S_2$: | | | | |
|---|---|---|---|---|
| Calculated: | C, 60.55; | H, 5.81; | N, 6.73; | S, 15.39; |
| Found: | C, 60.75; | H, 5.76; | N, 6.76; | S, 15.58. |

Example 24

(-)-5-[[3,5-Bis(1,1-dimethylethyl)-4-hydroxyphenyl]methyl]-4-thiazolidinone.

To a 50 ml, three-neck, round bottom flask containing 25 ml of methylene chloride were added 1.31 g of 4Å - molecular sieves, 0.56 ml (1.88 mmol) of titanium isopropoxide, 0.79 ml (3.75 mmol) of (+)-diisopropyl tartrate and 34 µl (1.88 mmol) of deionized water. The resulting solution was stirred for twenty minutes and then 0.8 g (2.5 mmol) of a racemic mixture of 5-[[3,5-bis(1,1-dimethylethyl)-4-hydroxyphenyl]methyl-4-thiazolidinone were added. The resulting solution was cooled to -20°C and 0.73 ml (1.88 mmol) of a 2.57M solution of t-butylhydroperoxide in isooctane were added. The reaction solution was then stirred for 6 hours at -20°C.

After 6 hours, the reaction solution was quenched by pouring it into 50 ml of a solution prepared from 9.9 g of Iron (II) sulfate heptahydrate, 3.3 g of citric acid monohydrate and water. The resulting solution was stirred for 30 minutes

and then stirring was stopped so that the organic and aqueous layers could separate. The aqueous layer was decanted and washed with methylene chloride. The methylene chloride wash was combined with the above organic layer and the resulting solution was washed with a saturated brine solution and then dried over sodium sulfate. The sodium sulfate was removed by filtration and the remaining liquid was evaporated to provide 1.81 g of a residue.

The residue was dissolved in 25 ml of methylene chloride and the resulting solution was chromatographed on a silica gel chromatography column. Elution with 6000 ml of a 10-50% ethyl acetate in hexane gradient provided various fractions containing the above titled compound. These fractions were combined and the liquid evaporated to provide 0.19 g of title compound.

$[\alpha]^{25}$ = -73.6° (c=1.0, MeOH).

| Analysis for $C_{18}H_{27}NO_2S$: | | | |
|---|---|---|---|
| Calculated: | C, 67.25; | H, 8,47; | N, 4.36; |
| Found: | C, 67.50; | H, 8.53; | N, 4.48. |

Example 25, 26 and 27

(+)-5-[[3,5-Bis(1,1-dimethylethyl)-4-hydroxyphenyl]methyl]-4-thiazolidinone, (-)-5-[[3,5-bis(1,1-dimethylethyl)-4-hydroxyphenyl]methyl]-4-thiazolidinone-1-oxide and (+)-5-[[3,5-bis(1,1-dimethylethyl)-4-hydroxyphenyl]methyl]-4-thiazolidinone-1-oxide.

In a similar manner as that described in Example 24, 0.89 ml (3.0 mmol) of titanium isopropoxide, 1.27 ml (6.0 mmol) of (-)-diisopropyl tartrate, 54 µl (3.0 mmol) of deionized water, 1.61 g (5.0 mmol) of racemic 5-[[3,5-bis(1,1-dimethylethyl)-4-hydroxyphenyl]methyl-4-thiazolidinone and 2.4 ml (6.5 mmol) of a 2.57M solution of t-butylhydroperoxide in isooctane were reacted to provide a residue. The residue was dissolved in 75 ml of methylene chloride and the resulting solution was chromatographed on a silica gel chromatography column. Elution with 6000 ml of a 10-50% ethyl acetate in hexane gradient provided various fractions containing (+)-5-[[3,5-bis(1,1-dimethylethyl)-4-hydroxyphenyl]methyl-4-thiazolidinone. These fractions were combined and the liquid evaporated to provide 0.43 g of product compound. Further elution with 4000 ml of a 50% isopropanol in hexane solution provided various fractions. Fractions believed to contain (-)-5-[[3,5-bis(1,1-dimethylethyl)-4-hydroxyphenyl]methyl]-4-thiazolidinone-1-oxide were combined and the liquid evaporated to provide 0.87 g of product. Fractions believed to contain (+)-5-[[3,5-bis(1,1-dimethylethyl)-4-hydroxyphenyl]methyl]-4-thiazolidinone-1-oxide were combined and the liquid evaporated to provide 0.27 g of product.

25. (+)-5-[[3,5-bis(1,1-dimethylethyl)-4-hydroxyphenyl]methyl]-4-thiazolidinone

$[\alpha]^{25}$ = +70.41° (c=1.0, MeOH).

| Analysis for $C_{18}H_{27}NO_2S$: | | | |
|---|---|---|---|
| Calculated: | C, 67.25; | H, 8.47; | N, 4.36; |
| Found: | C, 66.95; | H, 8.22; | N, 4.26. |

26. (-)-5-[[3,5-bis(1,1-dimethylethyl)-4-hydroxyphenyl]methyl]-4-thiazolidinone-1-oxide m.p. 182-184°C.

$[\alpha]^{25}$ = -21.84° (c=1.0, MeOH).

| Analysis for $C_{18}H_{27}NO_3S$: | | | |
|---|---|---|---|
| Calculated: | C, 64.06; | H, 8.06; | N, 4.15; |
| Found: | C, 63.84; | H, 8.09; | N, 4.12. |

27. (+)-5-[3,5-[bis(1,1-dimethylethyl)-4-hydroxyphenyl]methyl]-4-thiazolidinone-1-oxide m.p. 177-181°C.

$[\alpha]^{25}$ = +163.05° (c=1.0, MeOH).

| Analysis for $C_{18}H_{27}NO_3S$: | | | |
|---|---|---|---|
| Calculated: | C, 64.06; | H, 8.06; | N, 4.15; |
| Found: | C, 63.88; | H, 8.12; | N, 4.29. |

Example 28

(-)-5-[[3,5-Bis(1,1-dimethylethyl)-4-hydroxyphenyl]methyl]-3-methyl-4-thiazolidinone

In a similar manner as that described in Example 24, 0.45 ml (1.5 mmol) of titanium isopropoxide, 0.63 ml (3.0 mmol) of (+)-diisopropyltartrate, 27 µl (1.5 mmol) of water, 0.84 g (2.5 mmol) of racemic 5-[[3,5-bis(1,1-dimethylethyl)-4-hydroxyphenyl]methyl]-3-methyl-4-thiazolidinone and 0.58 ml (1.5 mmol) of a 2.75M solution of t-butylhydroperoxide in isooctane were reacted to provide a residue. The residue was dissolved in 25 ml of methylene chloride and the resulting solution was chromatographed on a silica gel chromatography column. Elution with 1000 ml of methylene chloride, then 6000 ml of a 0-10% ethyl acetate in methylene chloride gradient, then 4000 ml of a 20-50% isopropyl alcohol in hexane gradient and then 2000 ml of a 50% isopropyl alcohol/hexane solution provided various fractions containing the above-titled compound. These fractions were combined and the liquid evaporated to provide 0.35 g of title compound.

| Analysis for $C_{19}H_{29}NO_2S$: | | | |
|---|---|---|---|
| Calculated: | C, 68.02; | H, 8.71; | N, 4.17; |
| Found: | C, 67.95; | H, 8.55; | N, 4.18. |

NMR (300 MHz; $CDCl_3$)$\delta$ = 1.4 (s, 18H), 2.9 (s, 3H), 3.0 (dd, 1H), 3.3 (dd, 1H), 3.8 (dd, 1H), 4.0 (d, 1H), 4.2 (d, 1H), 5.1 (s, 1H), 7.1 (s, 2H).

Example 29

5-[[3-(1,1-dimethylethyl)-4-hydroxyphenyl]methyl]-4-thiazolidinone

A. Preparation of 3-(1,1-dimethylethyl)-4-hydroxybenzaldehyde

Into 184.4 ml (1.494 mol) of N-methylformanilide were added dropwise, with cooling, 130.9 ml (1.404 mol) of phosphoryl chloride over a period of 20 minutes. The mixture was allowed to warm to room temperature and stirred for one hour. Ortho-tertbutyl-phenol (138.2 ml; 0.9 mol) was then added dropwise to the reaction solution over a period of 25 minutes. After phenol addition was complete, the resulting reaction mixture was stirred for an additional 30 minutes at

room temperature and then heated to approximately 60°C and stirred for five hours at that temperature. The reaction mixture was poured into a volume of crushed ice and extracted with chloroform. The aqueous layer was separated and washed again with chloroform. The chloroform layers were combined and extracted with 2000 ml of a 5% potassium hydroxide solution. The aqueous potassium hydroxide extract was then added to 1000 ml of chloroform. The pH of the resulting two-phase mixture was adjusted to approximately pH 2.0 with concentrated hydrochloric acid. The mixture's layers were separated and the aqueous layer was again extracted with chloroform. The organic layer from the two-phase mixture and the chloroform extract were combined, washed with water and then dried over sodium sulfate. The volatile components of the solution were removed under reduced pressure to provide a residue. This residue was dissolved in 100 ml of hot toluene and the resulting solution was diluted with 100 ml of hexanes. The solution was slowly cooled to room temperature while a precipitate formed. This precipitate was recovered by filtration, washed with hexanes and then dried under vacuum to provide 20.0 g of the desired substitled intermediate.

B. Preparation of 5-[[3-(1,1-dimethylethyl)-4-hydroxyphenyl]methylene]-3-amino-2-thioxo-4-thiazolidinone

The benzaldehyde intermediate from Example 29A (20.0 g; 112.2 mmol), N-aminorhodanine (18.29 g; 123.4 mmol) and sodium acetate (36.8 g; 448.8 mmol) were suspended in 560 ml of acetic acid. The suspension was heated to reflux, stirred at that temperature for 7 hours (at which time a precipitate had formed) and then cooled to room temperature with stirring. The precipitate was recovered by filtration and then washed successively with a 1:1 ethyl acetate/diethyl ether solution then a diethyl ether wash. The recovered precipitate was dried under vacuum at 60°C for 2 hours to provide 14.5 g of the desired subtitled intermediate. m.p. >225°C.

C. Preparation of 5-[[3-(1,1-dimethylethyl)-4-hydroxyphenyl]methylene]-4-thiazolidinone

The intermediate from Example 29B (14.3 g; 46.4 mmol) was suspended in 230 ml of hot (60°C) toluene. To this suspension were added tri-n-butyl tin hydride (62.4 ml; 232 mmol) and AIBN (1.14 g; 6.96 mmol). The resulting suspension was heated to reflux while the suspended solids slowly dissolved. Additional AIBN was added at 30 and 55 minutes (two 1.14 g portions) after heating was started. Eighty minutes after heating was started the hot reaction solution was transferred to a separatory funnel and 1N hydrochloric acid was added. The resulting two-phase mixture was diluted with ethyl acetate and the layers were separated. The aqueous layer was washed with ethyl acetate, which wash was then combined with the organic layer from the two-phase mixture. The combined solution was washed with a saturated sodium chloride solution and then dried over sodium sulfate. The volatile components of the solution were removed under reduced pressure to provide 87.7 g of a yellow solid. This solid was suspended in 1000 ml of hexanes and the resulting suspension was stirred for 15 minutes. After fifteen minutes the suspension was filtered and the recovered solid was dissolved in 500 ml of diethyl ether. The diethyl ether solution was chromatographed on a silica gel column using an 8000 ml gradient of 5-20% isopropyl alcohol in hexanes, then a 2000 ml gradient of 20-30% isopropyl alcohol in hexanes and then a 2000 ml gradient of 30-35% isopropyl alcohol in hexanes. Those fractions identified as containing product were evaporated and chased with methylene chloride. The resulting residue was dissolved in ethyl acetate, reduced to dryness under reduced pressure and then chased with ethanol to provide 4.31 g of the desired subtitled intermediate. m.p. 110°C (decomposition).

| Analysis for $C_{14}H_{17}NO_2S$: | | | |
|---|---|---|---|
| Calculated: | C, 63.85; | H, 6.51; | N, 5.32; |
| Found: | C, 64.15; | H, 6.73; | N, 5.60. |

D. Preparation of 5-[[3-(1,1-dimethylethyl)-4-hydroxyphenyl]methyl]-4-thiazolidinone.

A portion of the intermediate from Example 29C (395.1 mg; 1.5 mmol) was dissolved in 9 ml of methanol. Magnesium (72.9 mg; 3.0 mmol) was then added to the solution and the resulting reaction mixture was stirred at room temperature for 3 hours. After 3 hours, most of the magnesium which had been added originally appeared to be gone so an additional 182.3 mg (7.5 mmol) of magnesium were added. Stirring of the reaction solution at room temperature continued overnight. By the next morning a yellow precipitate had formed. This precipitate was dissolved by adding the methanolic reaction solution to an ethyl acetate/1N hydrochloric acid mixture. The organic layer from the resulting two-phase mixture was isolated and then dried over sodium sulfate. The volatile components of the organic layer were removed and the resulting residue was chased with methylene chloride. The residue was then dissolved in 25 ml of

methylene chloride and the resulting solution was chromatographed on a silica gel chromatography column using a 5-20% isopropyl alcohol in hexanes gradient. Those fractions identified as containing essentially pure product were evaporated to provide 0.29 g of title compound. m.p. 65-70°C.

| Analysis for $C_{14}H_{19}NO_2S$: | | | |
|---|---|---|---|
| Calculated: | C, 63.37; | H, 7.22; | N, 5.28; |
| Found: | C, 63.08; | H, 7.30; | N, 4.99. |

The present invention provides a method of treating inflammatory bowel disease in mammals. Such activity was demonstrated in the following test system.

Sprague-Dawley rats from Charles River Laboratories, Portage, MI (either sex, weight approximately 250 g) were dosed orally twice a day with test compound (10 mg/kg) or vehicle (control) for three days. On the third day, the rats were given an intracolonic enema of 2% acetic acid via a cannula, the tip of which was placed 8 cm above the anal verge. This concentration of acetic acid produced a severe inflammatory response in the colon characterized by rectal bleeding, diarrhea, epithelial erosions and destructions of crypts and gland cells. Twenty-four hours later the test and control animals were killed and the distal ten centimeters of the colon were removed and opened longitudinally. The tissue lesions contained within the removed, opened, section of colon were scored by three independent, blinded observers on a scale of 0 to 4 (zero = normal, four = worst inflammation). In each test group 5-7 rats were used. The results of such testing are reported in Table I, below.

Table I

| Inhibition of Acetic Acid Induced Colitis | |
|---|---|
| Compound of Example No. | Lesion Score |
| Control | 3.4 ± 0.3 |
| Example 1 | 2.2 ± 0.5 |
| Example 2 | 1.1 ± 0.5 |
| Example 3 | 0.4 ± 0.1 |
| Example 4 | 1.5 ± 0.3 |
| Example 6 | 2.4 ± 0.5 |
| Example 7 | 2.1 ± 0.1 |
| Example 9 | 2.2 ± 0.5 |
| Example 10 | 1.2 ± 0.3 |
| Example 11 | 2.4 ± 0.7 |
| Example 12 | 2.0 ± 0.6 |
| Example 16 | 1.2 ± 0.5 |
| Example 18 | 2.8 ± 0.5 |
| Example 21 | 1.5 ± 0.5 |
| Example 22 | 0.8 ± 0.2 |
| Example 23 | 2.7 ± 0.6 |
| Example 24 | 1.0 ± 0.2 |
| Example 25 | 2.5 ± 0.7 |
| Example 26 | 2.4 ± 0.6 |

Table I (continued)

| Inhibition of Acetic Acid Induced Colitis | |
|---|---|
| Compound of Example No. | Lesion Score |
| Example 27 | 2.2 ± 0.5 |
| Example 29 | 2.2 ± 0.5 |

Sprague-Dawley rats from Charles River Laboratories, Portage, MI (male, weight approximately 300 g) were fasted for 24 hours. After 24 hours, the test animals were dosed orally with 3 ml/kg of rat weight of vehicle (control) or test compound dissolved in vehicle. Thirty minutes later each animal was given a solution consisting of 100% ethanol. Sixty minutes after ethanol administration, all animals were killed and their stomachs were removed and washed. The tissue lesions contained within the removed, opened, stomach were scored by three independent, blinded observers on a scale of 0 to 5 (zero = normal, 5 = severe damage). In each test group, six rats were employed. Test results from animals given test compound dissolved in vehicle were compared with test results from animals given vehicle alone in order to determine the percentage of lesion inhibition attributable to the test compound. The results of such testing are reported in Table II below.

Table II

| Inhibition of Ethanol Induced Injury | | |
|---|---|---|
| Compound of Example No. | Dose (mg/kg of rat weight) | % Inhibition |
| 3 | 0.7 | 24 |
| 3 | 1.00 | 35 |
| 3 | 3.00 | 47 |
| 3 | 7.00 | 61 |
| 9 | 0.7 | 9 |
| 9 | 1.00 | 13 |
| 9 | 3.00 | 34 |
| 9 | 7.00 | 40 |
| 9 | 10.00 | 24 |
| 10 | 0.07 | 34 |
| 10 | 0.3 | 57 |
| 10 | 0.7 | 55 |
| 10 | 1.00 | 32 |
| 10 | 3.00 | 11 |

The data in Tables I and II establish that the compounds used in the method of the present invention can treat inflammatory bowel disease. The term "inflammatory bowel disease", as used for purposes of the present invention, means any disorder of the digestive system which is characterized by inflammation. Examples of such disorders include Crohn's disease, mucous colitis, ulcerative colitis, psuedomembranous enterocolitis, non-specific colonic ulcers, collagenous colitis, cathartic colon, ulcerative proctitis, radiation enteritis and colitis, idiopathic diffuse ulcerative nongranulamatus enteritis, nonsteroidal antiinflammatory drug induced inflammations, celic sprue and the like.

The method of the present invention comprises administering to a mammal suffering from inflammatory bowel disease an effective amount of one or more of the compounds of Formula I. Administration may be done either therapeutically or prophylactically and is accomplished by means of pharmaceutical compositions which are prepared by techniques well known in the pharmaceutical sciences.

The compounds of Formula I are effective over a wide dosage range in treating inflammatory bowel disease. Thus, as used herein, the term "effective amount" refers to a dosage range of from about 0.001 to about 200 mg/kg of body

weight per day. In the treatment of adult humans, the range of about 0.1 to about 50 mg/kg, in single or divided doses, is preferred. However, it will be understood that the amount of compound actually administered will be determined by a physician in light of the relevant circumstances, including the condition to be treated, the choice of compound to be administered, the chosen route of administration, the age, weight, and response of the individual patient, and the severity of the patient's symptoms. Therefore, the above dosage ranges are not intended to limit the scope of the invention in any way.

While the compounds of Formula I are preferably administered orally or intrarectally, the compounds may also be administered by a variety of other routes such as the transdermal, subcutaneous, intranasal, intramuscular and intravenous routes.

The present invention provides new compounds of Formula II which are also useful in treating inflammatory bowel disease. Accordingly, the present invention is also directed to pharmaceutical compositions which include at least one compound of Formula II in association with one or more pharmaceutically acceptable diluents, excipients or carriers therefor.

In making the pharmaceutical compositions of the present invention, one or more compounds of Formula II will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of active compound, soft and hard gelatin capsules, suppositories, sterile injectable solutions and sterile packaged powders.

Some examples of suitable carriers, excipients, and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl - and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide rapid, sustained or delayed release of the active ingredient after administration to the patient by employing procedures well known in the art.

The compositions are formulated, preferably in a unit dosage form, such that each dosage contains from about 5 to about 500 mg, more usually about 25 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with one or more suitable pharmaceutical diluents, excipients or carriers.

The following formulation examples may employ as active ingredients any of the compounds of Formula II. The examples are illustrative only and are not intended to limit the scope of the invention in any way.

Example 30

Hard gelatin capsules are prepared using the following ingredients:

| | Quantity (mg/capsule) |
|---|---|
| Compound of Example 11 | 250 |
| Starch dried | 200 |
| Magnesium stearate | 10 |

The above ingredients are mixed and filled into hard gelatin capsules in 460 mg quantities.

Example 31

A tablet formula is prepared using the ingredients below:

| | Quantity (mg/tablet) |
|---|---|
| Compound of Example 11 | 250 |
| Cellulose, microcrystalline | 400 |
| Silicon dioxide, fumed | 10 |
| Stearic acid | 5 |

The components are blended and compressed to form tablets each weighing 665 mg.

Example 32

An aerosol solution is prepared containing the following components:

| | Weight % |
|---|---|
| Compound of Example 12 | 0.25 |
| Ethanol | 29.75 |
| Propellant 22 (Chlorodifluoromethane) | 70.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

Example 33

Tablets each containing 60 mg of active ingredient are made up as follows:

| | |
|---|---|
| Compound of Example 12 | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60°C and passed through a No. 18 mesh U.S. sieve. The sodium carboxymethyl starch, magnesium stearate and talc, previously passed through a No. 60 mesh U.S. sieve, are then added to the granules which, after mixing, are compressed by a tablet machine to yield tablets each weighing 150 mg.

Example 34

Capsules each containing 80 mg of medicament are made as follows:

| | |
|---|---|
| Compound of Example 12 | 80 mg |
| Starch | 59 mg |
| Microcrystalline cellulose | 59 mg |
| Magnesium stearate | 2 mg |
| Total | 200 mg |

The active ingredient, cellulose, starch and magnesium stearate are blended, passed through a No. 45 mesh U.S. sieve, and filled into hard gelatin capsules in 200 mg quantities.

Example 35

Suppositories each containing 225 mg of active ingredient are made as follows:

| | |
|---|---|
| Compound of Example 13 | 225 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Example 36

Suspensions each containing 50 mg of medicament per 5 ml dose are made as follows:

| | |
|---|---|
| Compound of Example 11 | 50 mg |
| Sodium carboxymethylcellulose | 50 mg |
| Syrup | 1.25 ml |
| Benzoic acid solution | 0.10 ml |
| Flavor | q.v. |
| Color | q.v. |
| Purified water to | 5 ml |

The medicament is passed through a No. 45 mesh U.S. sieve and mixed with the sodium carboxymethyl cellulose and syrup to form a smooth paste. The benzoic acid solution, flavor and color are diluted with some of the water and added, with stirring. Sufficient water is then added to produce the required volume.

Example 37

Capsules each containing 150 mg of medicament are made as follows:

| | |
|---|---|
| Compound of Example 13 | 150 mg |
| Starch | 164 mg |
| Microcrystalline cellulose | 164 mg |
| Magnesium stearate | 22 mg |
| Total | 500 mg |

The active ingredient, cellulose, starch and magnesium stearate are blended, passed through a No. 45 mesh U.S. sieve, and filled into hard gelatin capsules in 500 mg quantities.

Example 38

Hard gelatin capsules are prepared using the following ingredients:

| | Quantity (mg/capsule) |
|---|---|
| Compound of Example 3 | 250 |
| Starch dried | 200 |
| Magnesium stearate | 10 |

The above ingredients are mixed and filled into hard gelatin capsules in 460 mg quantities.

Example 39

Tablets each containing 60 mg of active ingredient are made up as follows:

| | |
|---|---|
| Compound of Example 3 | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60°C and passed through a No. 18 mesh U.S. sieve. The sodium carboxymethyl starch, magnesium stearate and talc, previously passed through a No. 60 mesh U.S. sieve, are then added to the granules which, after mixing, are compressed by a tablet machine to yield tablets each weighing 150 mg.

Example 40

Suppositories each containing 225 mg of active ingredient are made as follows:

| | |
|---|---|
| Compound of Example 3 | 225 mg |
| Saturated fatty acid glycerides | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

## Claims

### Claims for the following Contracting States : AT, BE, CH, DK, FR, GB, IT, LI, LU, NL, SE

1. A compound of Formula (II)

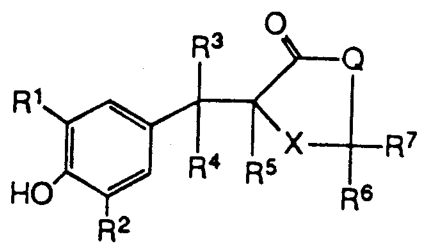

(II)

wherein:

$R^1$ is $C_2$-$C_6$ alkenyl, $C_2$-$C_6$ alkynyl or

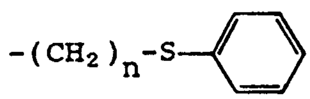

where n is an integer from 0 to 3, both inclusive;
$R^2$ is hydrogen, $C_1$-$C_6$ alkyl, $C_1$-$C_6$ alkoxy, $C_2$-$C_6$ alkenyl, $C_2$-$C_6$ alkynyl,

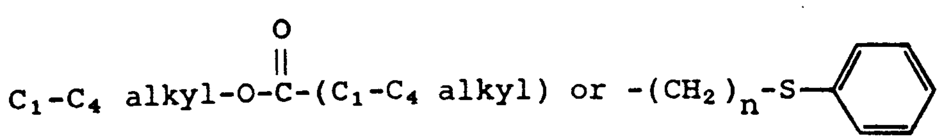

where n is an integer from 0 to 3, both inclusive;
$R^3$ is hydrogen or $C_1$-$C_6$ alkyl;
$R^4$ and $R^5$ are each hydrogen or when taken together form a bond;
$R^6$ and $R^7$ are each hydrogen or when taken together are =S, or when one of $R^6$ and $R^7$ is hydrogen the other is -OH or $-SCH_3$;
X is

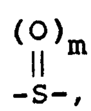

where m is 0, 1 or 2; and
Q is $-CH_2-$, -O- or $NR^8$ where $R^8$ is hydrogen, $C_1$-$C_6$ alkyl, $C_2$-$C_6$ alkenyl, $C_3$-$C_8$ cycloalkyl, $-SO_2CH_3$ or $-(CH_2)_n$-Y, where n is an integer from 0 to 3, both inclusive, and Y is cyano, $OR^9$,

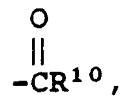

tetrazolyl, $-NR^{11}R^{12}$, -SH, -S($C_1$-$C_4$ alkyl) or

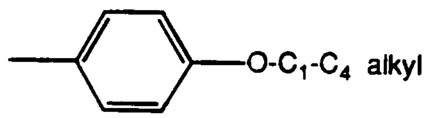

where $R^9$ is hydrogen, $C_1$-$C_4$ alkyl, or

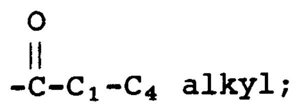

$R^{10}$ is $C_1$-$C_4$ alkyl, $C_1$-$C_4$ alkoxy or $-NH_2$; $R^{11}$ and $R^{12}$ are each independently hydrogen, $C_1$-$C_6$ alkyl, $C_2$-$C_6$ alkenyl, $C_2$-$C_6$ alkynyl, $-(CH_2)_qOH$, $-(CH_2)_q$-N($C_1$-$C_4$ alkyl)$_2$, $-(CH_2)_q$-S($C_1$-$C_4$ alkyl) or

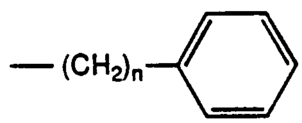

where n is as defined above and q is an integer from 1 to 6, both inclusive; or $R^{11}$ and $R^{12}$ taken together form a morpholinyl, piperidinyl, piperazinyl or N-methylpiperazinyl ring; or a pharmaceutically acceptable salt thereof.

**2.** A compound of Claim 1 wherein

$R^1$ is $C_2$-$C_6$ alkenyl, $C_2$-$C_6$ alkynyl or

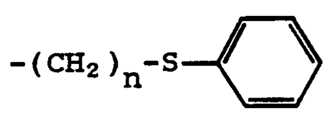

where n is an integer from 0 to 3, both inclusive;
$R^2$ is hydrogen, $C_1$-$C_6$ alkyl, $C_1$-$C_6$ alkoxy, $C_2$-$C_6$ alkenyl, $C_2$-$C_6$ alkynyl, or

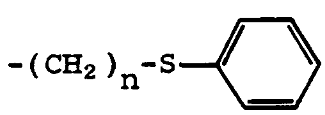

where n is an integer from 0 to 3, both inclusive;

$R^3$ is hydrogen or $C_1$-$C_6$ alkyl;
$R^4$ and $R^5$ are each hydrogen or when taken together form a bond;
$R^6$ and $R^7$ are each hydrogen or when taken together are =S, or when one of $R^6$ and $R^7$ is hydrogen the other is -OH or -$SCH_3$;
X is

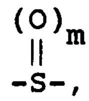

where m is 0, 1 or 2; and
Q is -$CH_2$-, -O- or $NR^8$ where $R^8$ is hydrogen, $C_1$-$C_6$ alkyl, $C_3$-$C_8$ cycloalkyl, -$SO_2CH_3$ or -$(CH_2)_n$-Y, where n is an integer from 0 to 3, both inclusive, and Y is cyano, $OR^9$,

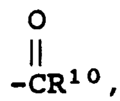

tetrazolyl, -$NR^{11}R^{12}$ or

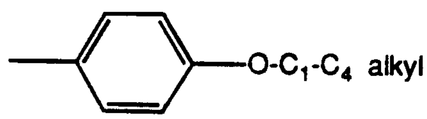

where $R^9$ is hydrogen, $C_1$-$C_4$ alkyl, or

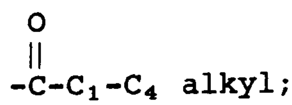

$R^{10}$ is $C_1$-$C_4$ alkyl, $C_1$-$C_4$ alkoxy or -$NH_2$; $R^{11}$ and $R^{12}$ are each independently hydrogen, $C_1$-$C_6$ alkyl, -$(CH_2)_q$OH, -$(CH_2)_q$-N($C_1$-$C_4$ alkyl$)_2$, -$(CH_2)_q$-S($C_1$-$C_4$ alkyl) or

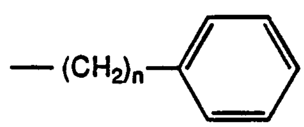

where n is as defined above and q is an integer from 1 to 6, both inclusive; or $R^{11}$ and $R^{12}$ taken together form a morpholinyl, piperidinyl, piperazinyl or N-methylpiperazinyl ring; or a pharmaceutically acceptable salt thereof.

**3.** A compound of Claim 2 wherein $R^1$ is $C_2$-$C_6$ alkenyl, $R^2$ is $C_2$-$C_6$ alkenyl or $C_1$-$C_6$ alkyl; $R^3$ is hydrogen; $R^4$ and $R^5$ are hydrogen or when taken together form a bond; $R^6$ and $R^7$ are each hydrogen or when taken together are =S; X is

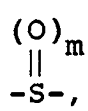

where m is O; and Q is -O- or $NR^8$, where $R^8$ is as defined in Claim 2, and pharmaceutically acceptable salts thereof.

4. A pharmaceutical composition comprising as an active ingredient a compound of any one of Claims 1 to 3, or a pharmaceutically acceptable salt thereof, in association with one or more pharmaceutically acceptable diluents, excipients or carriers therefor.

5. A compound as claimed in any one of Claims 1 to 3 for use in treating inflammatory bowel disease.

6. A process for preparing a compound of Formula II as claimed in any one of Claims 1 to 3 which comprises:

(A) reacting a compound of the formula

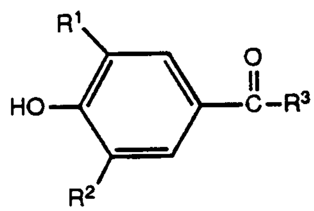

with a compound of the formula

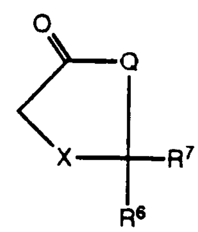

wherein $R^1$, $R^2$, $R^3$ and X are as defined in any one of Claims 1 to 3, Q is $-CH_2-$ or $NR^8$ (where $R^8$ is as defined in any one of Claims 1 to 3) and $R^6$ and $R^7$ taken together are =S, so as to provide a compound of the formula

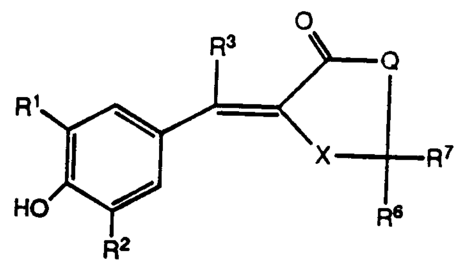

wherein $R^1$, $R^2$, $R^3$, $R^6$, $R^7$, X and Q are as set forth above;
(B) reducing a compound of Formula II wherein $R^6$ and $R^7$ taken together are =S so as to prepare a compound of Formula II in which $R^6$ and $R^7$ are hydrogen;
(C) reducing a compound of Formula II in which $R^4$ and $R^5$ taken together form a bond so as to prepare a compound of Formula II in which $R^4$ and $R^5$ are hydrogen;
(D) reducing a compound of Formula II in which $R^4$ and $R^5$ taken together form a bond and $R^6$ and $R^7$ taken together are =S so as to prepare a compound of Formula II in which $R^4$, $R^5$, $R^6$ and $R^7$ are all hydrogen;
(E) alkylating a compound of Formula II in which $R^8$ is hydrogen so as to prepare a compound of Formula II in which $R^8$ is $C_1$-$C_6$ alkyl, $C_2$-$C_6$ alkenyl, $C_3$-$C_8$ cycloalkyl or $-(CH_2)_n-Y$ (where n is an integer from 0 to 3, both inclusive, and Y is cyano, $OR^9$, -SH, $-S(C_1$-$C_4$ alkyl), $-NR^{11}R^{12}$ or

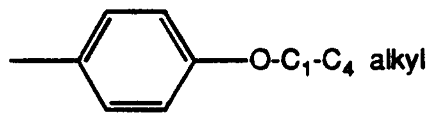

where $R^9$, $R^{11}$ and $R^{12}$ are as defined in any one of Claims 1 to 3);
(F) acylating a compound of Formula II in which $R^8$ is hydrogen so as to prepare a compound of Formula II in which $R^8$ is $-(CH_2)_n-Y$, where n is an integer from 0 to 3, both inclusive, and Y is

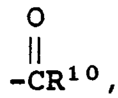

where $R^{10}$ is as defined in any one of Claims 1 to 3;
(G) oxidizing a compound of Formula II wherein X is

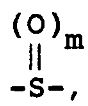

where m is 0, so as to prepare a compound of Formula II wherein X is

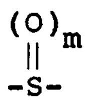

and m is 1;
(H) oxidizing a compound of Formula II wherein X is

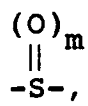

where m is 0, so as to prepare a compound of Formula II wherein X is

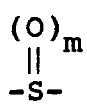

and m is 2;
(I) oxidizing a compound of Formula II wherein X is

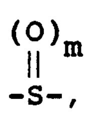

where m is 1, so as to prepare a compound of Formula II wherein X is

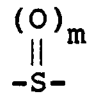

and m is 2;

(J) reacting a compound of the formula

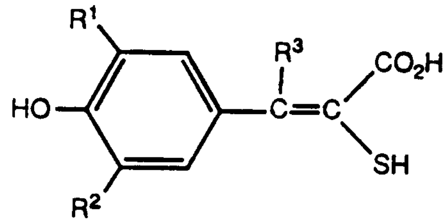

with

i) formic acid, so as to provide a compound of Formula II wherein Q is O, $R^4$ and $R^5$ taken together form a bond and $R^6$ and $R^7$ are hydrogen; or

ii) carbon disulfide, so as to provide a compound of Formula II wherein Q is O, $R^3$ and $R^4$ taken together form a bond and $R^6$ and $R^7$ taken together are =S;

(K) reacting a compound of the formula

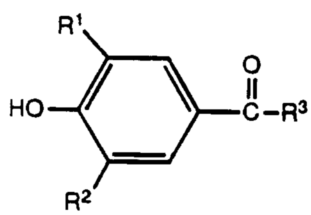

with a compound of the formula

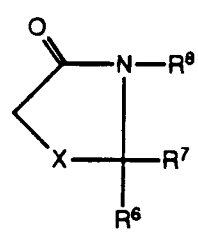

wherein $R^1$, $R^2$, $R^3$ and X are as defined in any one of Claims 1 to 3,
$R^6$ and $R^7$ taken together are =S, and $R^8$ is $-(CH_2)_n-Y$ (where n is an integer from 0 to 3, both inclusive, and Y is $OR^9$, where $R^9$ is hydrogen) so as to provide a compound of the formula

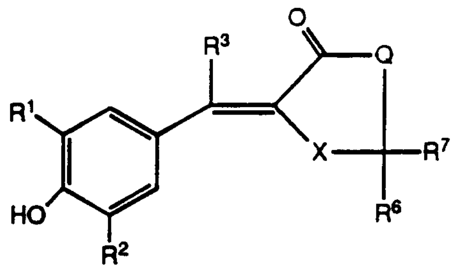

wherein $R^1$, $R^2$, $R^3$, $R^6$, $R^7$ and X are as set forth above and $R^8$ is $-(CH_2)_n$-Y (where n is an integer from 0 to 3, both inclusive, and Y is $OR^9$, where $R^9$ is

$$-\overset{\overset{\displaystyle O}{||}}{C}-CH_3);$$

(L) reducing a compound of Formula II in which $R^8$ is $-(CH_2)_n$-Y, wherein n is 0 to 3, both inclusive, and Y is $OR^9$, where $R^9$ is

$$-\overset{\overset{\displaystyle O}{||}}{C}-C_1-C_4 \text{ alkyl},$$

so as to prepare a compound of Formula II in which $R^8$ is $-(CH_2)_n$-Y, wherein n is 0 to 3, both inclusive, and Y is $OR^9$, where $R^9$ is hydrogen;

(M) reacting a compound of Formula II in which $R^8$ is $-(CH_2)_n$-Y, wherein n is 0 to 3, both inclusive, and Y is -$OR^9$, where $R^9$ is hydrogen, with a tosyl-halide so as to prepare a compound of Formula II in which $R^8$ is -$(CH_2)_n$-Y, wherein n is 0 to 3, both inclusive, and Y is $OR^9$, where $R^9$ is tosyl;

(N) reacting a compound of Formula II in which $R^8$ is $-(CH_2)_n$-Y, wherein n is 0 to 3, both inclusive, and Y is -$OR^9$, where $R^9$ is tosyl, with an amine of the formula $HNR^{11}R^{12}$ (where $R^{11}$ and $R^{12}$ are as defined in any one of Claims 1 to 3) so as to prepare a compound of Formula II in which $R^8$ is $-(CH_2)_n$-Y, wherein n is 0 to 3, both inclusive, and Y is $-NR^{11}R^{12}$;

(O) treating a compound of Formula II in which $R^8$ is $-(CH_2)_n$-Y, wherein n is 0 to 3, both inclusive, and Y is cyano with tri-n-butyl tin azide so as to prepare a compound of Formula II in which $R^8$ is $-(CH_2)_n$-Y, wherein n is 0 to 3, both inclusive, and Y is tetrazolyl;

(P) reacting a compound of the formula

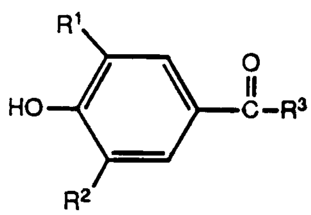

with a compound of the formula

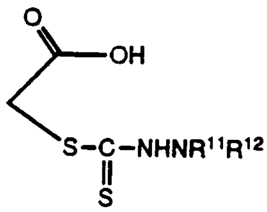

wherein $R^1$, $R^2$, $R^3$, $R^{11}$ and $R^{12}$ are as defined in any one of Claims 1 to 3, so as to provide a compound of the formula

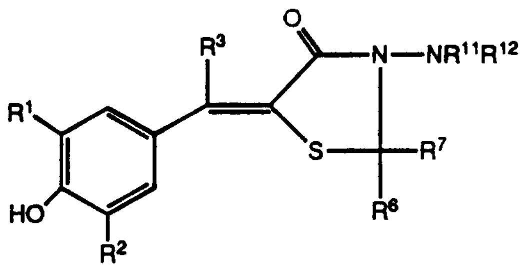

wherein $R^6$ and $R^7$ taken together are =S and $R^1$, $R^2$, $R^3$, $R^{11}$ and $R^{12}$ are as defined in any one of Claims 1 to 3;

(Q) heating a compound of Formula II in which $R^8$ is $-(CH_2)_n$-Y and Y is $-NR^{11}R^{12}$ (neither of $R^{11}$ or $R^{12}$ being hydrogen) in an ethanol/water mixture in the presence of a catalyst so as to prepare a compound of Formula II in which $R^8$ is $-(CH_2)_n$-Y and Y is $-NR^{11}R^{12}$ (where one of $R^{11}$ or $R^{12}$ is hydrogen and the other is not hydrogen);

(R) reacting a compound of Formula II in which $R^6$ and $R^7$ are both hydrogen with trifluoroacetic anhydride so as to prepare a compound of Formula II in which one of $R^6$ and $R^7$ is hydrogen and the other is -OH;

(S) salifying a compound of Formula II by reacting the non-salt form of the compound with either a strong acid or a strong base.

**7.** A process for selectively isolating, in substantially pure enantiomeric form, one of the enantiomers of a racemic mixture of a compound of the formula

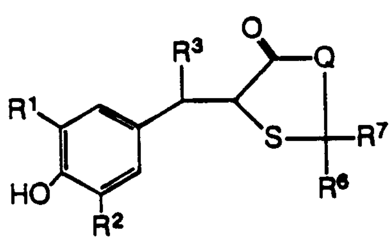

wherein:

$R^1$ and $R^2$ are each independently hydrogen, $C_1$-$C_6$ alkyl, $C_1$-$C_6$ alkoxy, $C_2$-$C_6$ alkenyl, $C_2$-$C_6$ alkynyl,

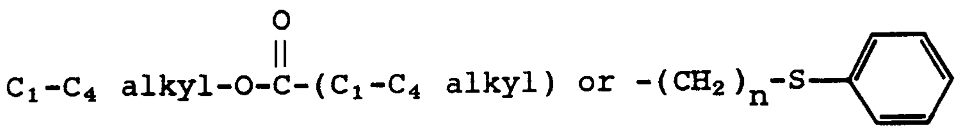

where n is an integer from 0 to 3, both inclusive;
$R^3$ is hydrogen or $C_1$-$C_6$ alkyl;
$R^6$ and $R^7$ are each hydrogen or when taken together are =S, or when one of $R^6$ and $R^7$ is hydrogen the other is -OH or -$SCH_3$;
Q is -$CH_2$-, -O- or $NR^8$ where $R^8$ is hydrogen, $C_1$-$C_6$ alkyl, $C_2$-$C_6$ alkenyl, $C_3$-$C_8$ cycloalkyl, -$SO_2CH_3$ or -$(CH_2)_n$-Y, where n is an integer from 0 to 3, both inclusive, and Y is cyano, $OR^9$,

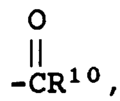

tetrazolyl, -$NR^{11}R^{12}$, -SH, -S($C_1$-$C_4$ alkyl) or where $R^9$ is hydrogen, $C_1$-$C_4$ alkyl

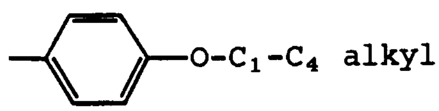

or

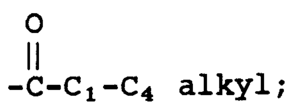

$R^{10}$ is $C_1$-$C_4$ alkyl, $C_1$-$C_4$ alkoxy or -$NH_2$; $R^{11}$ and $R^{12}$ are each independently hydrogen, $C_1$-$C_6$ alkyl, $C_2$-$C_6$ alkenyl, $C_2$-$C_6$ alkynyl, -$(CH_2)_q$OH, -$(CH_2)_q$-N($C_1$-$C_4$ alkyl)$_2$, -$(CH_2)_q$-S($C_1$-$C_4$ alkyl) or

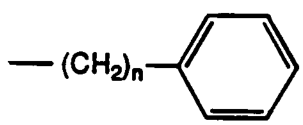

where n is as defined above and q is an integer from 1 to 6, both inclusive; or $R^{11}$ and $R^{12}$ taken together form a morpholinyl, piperidinyl, piperazinyl or N-methylpiperazinyl ring, comprising:

a) reacting the racemic sulfide compound with a reagent prepared from the combination of a tartrate ligand, a titanium alkoxide, a hydroperoxide and, optionally, water until substantially all of the undesired enantiomer of the sulfide substrate has been converted to its sulfoxide analog; and
b) separating the unreacted portion of the sulfide starting material, consisting essentially of substantially pure desired enantiomer, from the reaction mixture.

**8.** The use of a compound of the Formula

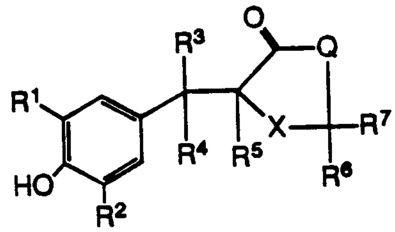

wherein:

$R^1$ and $R^2$ are each independently hydrogen, $C_1$-$C_6$ alkyl, $C_1$-$C_6$ alkoxy, $C_2$-$C_6$ alkenyl, $C_2$-$C_6$ alkynyl,

$$C_1\text{-}C_4\ \text{alkyl-O-}\overset{\overset{\displaystyle O}{\|}}{C}\text{-}(C_1\text{-}C_4\ \text{alkyl})\ \text{or}\ \text{-}(CH_2)_n\text{-S-}$$ 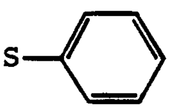

where n is an integer from 0 to 3, both inclusive;
$R^3$ is hydrogen or $C_1$-$C_6$ alkyl;
$R^4$ and $R^5$ are each hydrogen or when taken together form a bond;
$R^6$ and $R^7$ are each hydrogen or when taken together are =S, or when one of $R^6$ and $R^7$ is hydrogen the other is -OH or $-SCH_3$;
X is

$$\overset{\overset{\displaystyle (O)_m}{\|}}{\text{-S-}},$$

where m is 0, 1 or 2; and
Q is $-CH_2$-, -O- or $NR^8$ where $R^8$ is hydrogen, $C_1$-$C_6$ alkyl, $C_2$-$C_6$ alkenyl, $C_3$-$C_8$ cycloalkyl, $-SO_2CH_3$ or -$(CH_2)_n$-Y, where n is an integer from 0 to 3, both inclusive, and Y is cyano, $OR^9$,

$$\text{-}\overset{\overset{\displaystyle O}{\|}}{C}R^{10},$$

tetrazolyl, $-NR^{11}R^{12}$, -SH -S($C_1$-$C_4$ alkyl) or

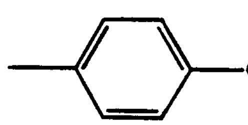 O-$C_1$-$C_4$ alkyl

where $R^9$ is hydrogen, $C_1$-$C_4$ alkyl, or

$$\text{-}\overset{\overset{\displaystyle O}{\|}}{C}\text{-}C_1\text{-}C_4\ \text{alkyl};$$

$R^{10}$ is $C_1$-$C_4$ alkyl, $C_1$-$C_4$ alkoxy or $-NH_2$; $R^{11}$ and $R^{12}$ are each independently hydrogen, $C_1$-$C_6$ alkyl, $C_2$-$C_6$ alkenyl, $C_2$-$C_6$ alkynyl, $-(CH_2)_qOH$, $-(CH_2)_q$-N($C_1$-$C_4$ alkyl)$_2$, $-(CH_2)_q$-S($C_1$-$C_4$ alkyl) or

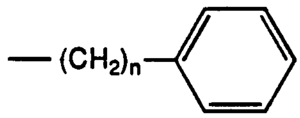

where n is as defined above and q is an integer from 1 to 6, both inclusive; or $R^{11}$ and $R^{12}$ taken together form a morpholinyl, piperidinyl, piperazinyl or N-methyl-piperazinyl ring; or a pharmaceutically acceptable salt thereof, to prepare a medicament against inflammatory bowel disease.

**9.** The use of a compound of Claim 8 wherein

$R^1$ and $R^2$ are each independently hydrogen, $C_1$-$C_6$ alkyl, $C_1$-$C_6$ alkoxy, $C_2$-$C_6$ alkenyl, $C_2$-$C_6$ alkynyl, or

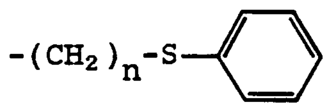

where n is an integer from 0 to 3, both inclusive;
$R^3$ is hydrogen or $C_1$-$C_6$ alkyl;
$R^4$ and $R^5$ are each hydrogen or when taken together form a bond;
$R^6$ and $R^7$ are each hydrogen or when taken together are =S, of when one of $R^6$ and $R^7$ is hydrogen the other is -OH or $-SCH_3$;
X is

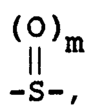

where m is 0, 1 or 2; and
Q is $-CH_2$-, -O- or $NR^8$ where $R^8$ is hydrogen, $C_1$-$C_6$ alkyl, $C_3$-$C_8$ cycloalkyl, $-SO_2CH_3$ or $-(CH_2)_n$-Y, where n is an integer from 0 to 3, both inclusive, and Y is cyano, $OR^9$,

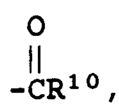

tetrazolyl, $NR^{11}R^{12}$ or

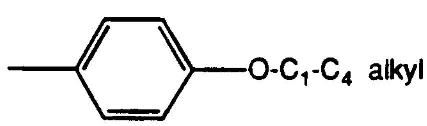

where $R^9$ is hydrogen, $C_1$-$C_4$ alkoxy or

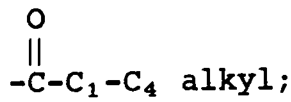

$R^{10}$ is $C_1$-$C_4$ alkyl, $C_1$-$C_4$ alkoxy or $-NH_2$; $R^{11}$ and $R^{12}$ are each independently hydrogen, $C_1$-$C_6$ alkyl, $-(CH_2)_qOH$, $-(CH_2)_q-N(C_1$-$C_4$ alkyl$)_2$, $-(CH_2)_q-S(C_1$-$C_4$ alkyl) or

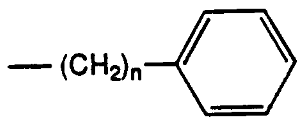

where n is as defined above and q is an integer from 1 to 6, both inclusive; or $R^{11}$ and $R^{12}$ taken together form a morpholinyl, piperidinyl, piperazinyl or N-methyl-piperazinyl ring; or a pharmaceutically acceptable salt thereof, to prepare a medicament against inflammatory bowel disease.

**10.** The use of 5-[[3,5-bis(1,1-dimethylethyl)-4-hydroxyphenyl]methyl]-4-thiazolidinone to prepare a medicament against inflammatory bowel disease.

**Claims for the following Contracting States : ES, GR**

**1.** A process for preparing a compound of the Formula (II)

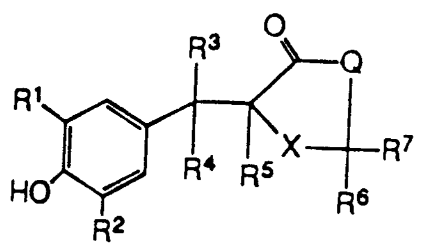

(II)

wherein:

$R^1$ is $C_2$-$C_6$ alkenyl, $C_2$-$C_6$ alkynyl or

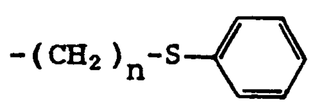

where n is an integer from 0 to 3, both inclusive;
$R^2$ is hydrogen, $C_1$-$C_6$ alkyl, $C_1$-$C_6$ alkoxy, $C_2$-$C_6$ alkenyl, $C_2$-$C_6$ alkynyl,

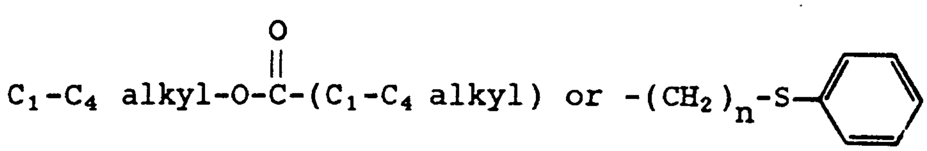

where n is an integer from 0 to 3, both inclusive;

$R^3$ is hydrogen or $C_1$-$C_6$ alkyl;

$R^4$ and $R^5$ are each hydrogen or when taken together form a bond;

$R^6$ and $R^7$ are each hydrogen or when taken together are =S, or when one of $R^6$ and $R^7$ is hydrogen the other is -OH or $-SCH_3$;

X is

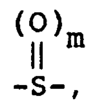

where m is 0, 1 or 2; and

Q is $-CH_2-$, -O- or $NR^8$ where $R^8$ is hydrogen, $C_1$-$C_6$ alkyl, $C_2$-$C_6$ alkenyl, $C_3$-$C_8$ cycloalkyl, $-SO_2CH_3$ or $-(CH_2)_n$-Y, where n is an integer from 0 to 3, both inclusive, and Y is cyano, $OR^9$,

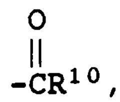

tetrazolyl, $-NR^{11}R^{12}$, -SH, -S($C_1$-$C_4$ alkyl) or

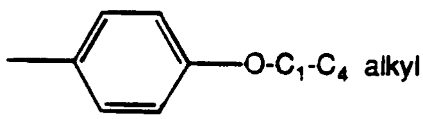

where $R^9$ is hydrogen, $C_1$-$C_4$ alkyl, or

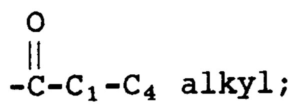

$R^{10}$ is $C_1$-$C_4$ alkyl, $C_1$-$C_4$ alkoxy or $-NH_2$; $R^{11}$ and $R^{12}$ are each independently hydrogen, $C_1$-$C_6$ alkyl, $C_2$-$C_6$ alkenyl, $C_2$-$C_6$ alkynyl, $-(CH_2)_qOH$, $-(CH_2)_q-N(C_1-C_4\ alkyl)_2$, $-(CH_2)_q-S(C_1-C_4\ alkyl)$ or

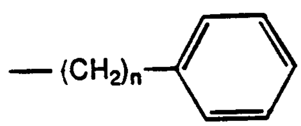

where n is as defined above and q is an integer from 1 to 6, both inclusive; or $R^{11}$ and $R^{12}$ taken together form a morpholinyl, piperidinyl, piperazinyl or N-methylpiperazinyl ring; or a pharmaceutically acceptable salt thereof, which comprises:

(A) reacting a compound of the formula

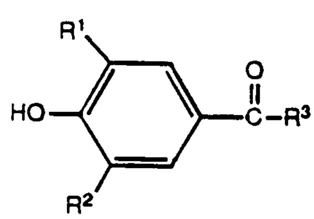

with a compound of the formula

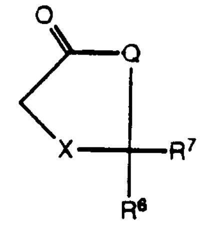

wherein $R^1$, $R^2$, $R^3$ and X are as defined above, Q is $-CH_2-$ or $NR^8$ (where $R^8$ is as defined above) and $R^6$ and $R^7$ taken together are =S, so as to provide a compound of the formula

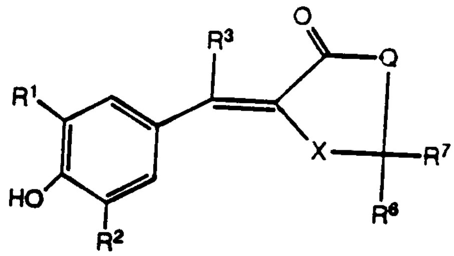

wherein $R^1$, $R^2$, $R^3$, $R^6$, $R^7$, X and Q are as set forth above;

(B) reducing a compound of Formula II wherein $R^6$ and $R^7$ taken together are =S so as to prepare a compound of Formula II in which $R^6$ and $R^7$ are hydrogen;

(C) reducing a compound of Formula II in which $R^4$ and $R^5$ taken together form a bond so as to prepare a compound of Formula II in which $R^4$ and $R^5$ are hydrogen;

(D) reducing a compound of Formula II in which $R^4$ and $R^5$ taken together form a bond and $R^6$ and $R^7$ taken together are =S so as to prepare a compound of Formula II in which $R^4$, $R^5$, $R^6$ and $R^7$ are all hydrogen;

(E) alkylating a compound of Formula II in which $R^8$ is hydrogen so as to prepare a compound of Formula II in which $R^8$ is $C_1$-$C_6$ alkyl, $C_2$-$C_6$ alkenyl, $C_3$-$C_8$ cycloalkyl or $-(CH_2)_n-Y$ (where n is an integer from 0 to 3, both inclusive, and Y is cyano, $OR^9$, -SH, $-S(C_1$-$C_4$ alkyl), $-NR^{11}R^{12}$ or

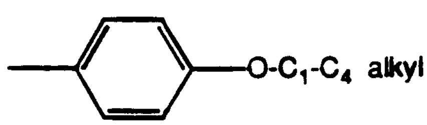

where $R^9$, $R^{11}$ and $R^{12}$ are as defined above);

(F) acylating a compound of Formula II in which $R^8$ is hydrogen so as to prepare a compound of Formula II in which $R^8$ is $-(CH_2)_n-Y$, where n is an integer from 0 to 3, both inclusive, and Y is

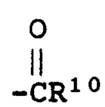,

where $R^{10}$ is as defined above;

(G) oxidizing a compound of Formula II wherein X is

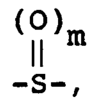

where m is 0, so as to prepare a compound of Formula II wherein X is

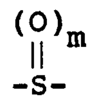

and m is 1;

(H) oxidizing a compound of Formula II wherein X is

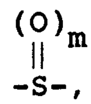

where m is 0, so as to prepare a compound of Formula II wherein X is

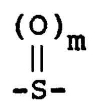

and m is 2;

(I) oxidizing a compound of Formula II wherein X is

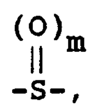

where m is 1, so as to prepare a compound of Formula II wherein X is

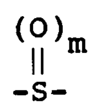

and m is 2;

(J) reacting a compound of the formula

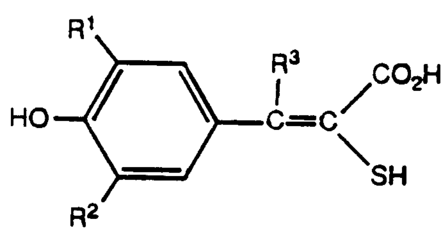

with

i) formic acid, so as to provide a compound of Formula II wherein Q is O, $R^4$ and $R^5$ taken together form a bond and $R^6$ and $R^7$ are hydrogen; or

ii) carbon disulfide, so as to provide a compound of Formula II wherein Q is O, $R^3$ and $R^4$ taken together form a bond and $R^6$ and $R^7$ taken together are =S;

(K) reacting a compound of the formula

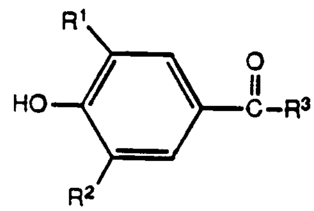

with a compound of the formula

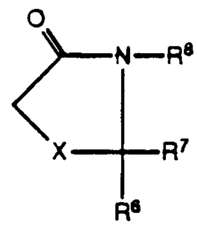

wherein $R^1$, $R^2$, $R^3$ and X are as defined above, $R^6$ and $R^7$ taken together are =S, and $R^8$ is $-(CH_2)_n-Y$ (where n is an integer from 0 to 3, both inclusive, and Y is $OR^9$, where $R^9$ is hydrogen) so as to provide a compound of the formula

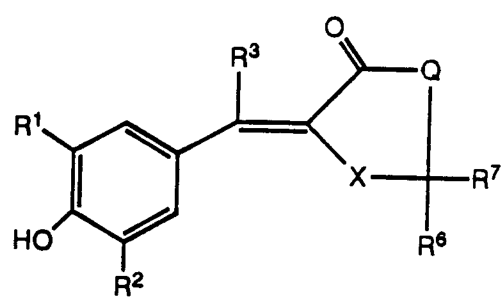

wherein $R^1$, $R^2$, $R^3$, $R^6$, $R^7$ and X are as set forth above and $R^8$ is $-(CH_2)_n-Y$ (where n is an integer from 0 to 3, both inclusive, and Y is $OR^9$, where $R^9$ is

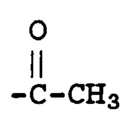 );

(L) reducing a compound of Formula II in which $R^8$ is $-(CH_2)_n-Y$, wherein n is 0 to 3, both inclusive, and Y is $OR^9$, where $R^9$ is

$$-\overset{O}{\overset{\|}{C}}-C_1-C_4 \text{ alkyl,}$$

so as to prepare a compound of Formula II in which $R^8$ is $-(CH_2)_n$-Y, wherein n is 0 to 3, both inclusive, and Y is $OR^9$, where $R^9$ is hydrogen;

(M) reacting a compound of Formula II in which $R^8$ is $-(CH_2)_n$-Y, wherein n is 0 to 3, both inclusive, and Y is $-OR^9$, where $R^9$ is hydrogen, with a tosyl-halide so as to prepare a compound of Formula II in which $R^8$ is $-(CH_2)_n$-Y, wherein n is 0 to 3, both inclusive, and Y is $OR^9$, where $R^9$ is tosyl;

(N) reacting a compound of Formula II in which $R^8$ is $-(CH_2)_n$-Y, wherein n is 0 to 3, both inclusive, and Y is $-OR^9$, where $R^9$ is tosyl, with an amine of the formula $HNR^{11}R^{12}$ (where $R^{11}$ and $R^{12}$ are as defined above) so as to prepare a compound of Formula II in which $R^8$ is $-(CH_2)_n$-Y, wherein n is 0 to 3, both inclusive, and Y is $-NR^{11}R^{12}$;

(O) treating a compound of Formula II in which $R^8$ is $-(CH_2)_n$-Y, wherein n is 0 to 3, both inclusive, and Y is cyano with tri-n-butyl tin azide so as to prepare a compound of Formula II in which $R^8$ is $-(CH_2)_n$-Y, wherein n is 0 to 3, both inclusive, and Y is tetrazolyl;

(P) reacting a compound of the formula

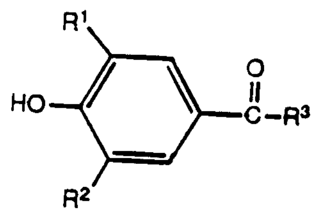

with a compound of the formula

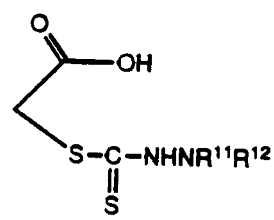

wherein $R^1$, $R^2$, $R^3$, $R^{11}$ and $R^{12}$ are as defined above, so as to provide a compound of the formula

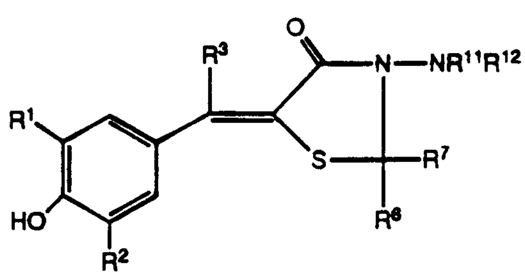

wherein $R^6$ and $R^7$ taken together are =S and $R^1$, $R^2$, $R^3$, $R^{11}$ and $R^{12}$ are as defined above;

(Q) heating a compound of Formula II in which $R^8$ is $-(CH_2)_n$-Y and Y is $-NR^{11}R^{12}$ (neither of $R^{11}$ or $R^{12}$ being hydrogen) in an ethanol/water mixture in the presence of a catalyst so as to prepare a compound of Formula II in which $R^8$ is $-(CH_2)_n$-Y and Y is $-NR^{11}R^{12}$ (where one of $R^{11}$ or $R^{12}$ is hydrogen and the other is not hydrogen);

(R) reacting a compound of Formula II in which $R^6$ and $R^7$ are both hydrogen with trifluoroacetic anhydride

so as to prepare a compound of Formula II in which one of $R^6$ and $R^7$ is hydrogen and the other is -OH;

(S) salifying a compound of Formula II by reacting the non-salt form of the compound with either a strong acid or a strong base.

**2.** A process according to Claim 1 for preparing a compound of the Formula (II) wherein:

$R^1$ is $C_2$-$C_6$ alkenyl, $C_2$-$C_6$ alkynyl or

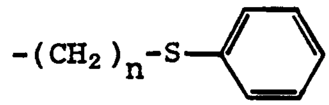

where n is an integer from 0 to 3, both inclusive;
$R^2$ is hydrogen, $C_1$-$C_6$ alkyl, $C_1$-$C_6$ alkoxy, $C_2$-$C_6$ alkenyl, $C_2$-$C_6$ alkynyl, or

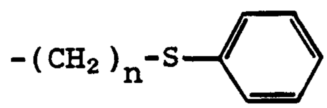

where n is an integer from 0 to 3, both inclusive;
$R^3$ is hydrogen or $C_1$-$C_6$ alkyl;
$R^4$ and $R^5$ are each hydrogen or when taken together form a bond;
$R^6$ and $R^7$ are each hydrogen or when taken together are =S, or when one of $R^6$ and $R^7$ is hydrogen the other is -OH or $-SCH_3$;
X is

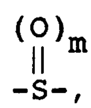

where m is 0, 1 or 2; and
Q is $-CH_2-$, -O- or $NR^8$ where $R^8$ is hydrogen, $C_1$-$C_6$ alkyl, $C_3$-$C_8$ cycloalkyl, $-SO_2CH_3$ or $-(CH_2)_n-Y$, where n is an integer from 0 to 3, both inclusive, and Y is cyano, $OR^9$,

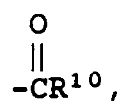

tetrazolyl, $-NR^{11}R^{12}$ or

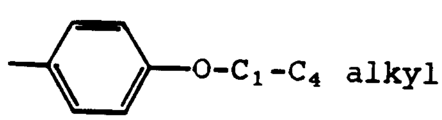

where $R^9$ is hydrogen, $C_1$-$C_4$ alkyl, or

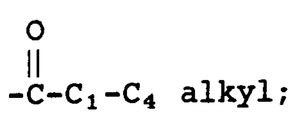

$R^{10}$ is $C_1$-$C_4$ alkyl, $C_1$-$C_4$ alkoxy or $-NH_2$; $R^{11}$ and $R^{12}$ are each independently hydrogen, $C_1$-$C_6$ alkyl, -

$(CH_2)_qOH$, $-(CH_2)_q-N(C_1-C_4\ alkyl)_2$, $-(CH_2)_q-S(C_1-C_4\ alkyl)$ or

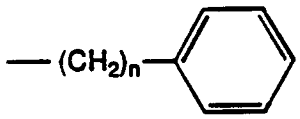

where n is as defined above and q is an integer from 1 to 6, both inclusive; or $R^{11}$ and $R^{12}$ taken together form a morpholinyl, piperidinyl, piperazinyl or N-methylpiperazinyl ring; or a pharmaceutically acceptable salt thereof.

3. A process according to Claim 2 for preparing a compound of Formula II wherein $R^1$ is $C_2$-$C_6$ alkenyl; $R^2$ is $C_2$-$C_6$ alkenyl or $C_1$-$C_6$ alkyl; $R^3$ is hydrogen; $R^4$ and $R^5$ are hydrogen or when taken together form a bond; $R^6$ and $R^7$ are each hydrogen or when taken together are =S; S is

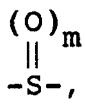

where m is O; and Q is -O-or $NR^8$, where $R^8$ is as defined in Claim 2, and pharmaceutically acceptable salts thereof.

4. A process according to Claim 3 for preparing 5-[[3,5-di-2-propenyl-4-hydroxyphenyl]methyl]-4-thiazolidinone and pharmaceutically acceptable salts thereof.

5. A process for selectively isolating, in substantially pure enantiomeric form, one of the enantiomers of a racemic mixture of a compound of the formula

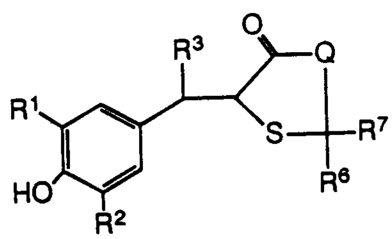

wherein:

$R^1$ and $R^2$ are each independently hydrogen, $C_1$-$C_6$ alkyl, $C_1$-$C_6$ alkoxy, $C_2$-$C_6$ alkenyl, $C_2$-$C_6$ alkynyl,

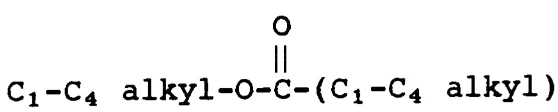

or

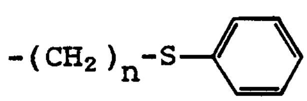

where n is an integer from 0 to 3, both inclusive;
$R^3$ is hydrogen or $C_1$-$C_6$ alkyl;
$R^8$ and $R^7$ are each hydrogen or when taken together are =S, or when one of $R^6$ and $R^7$ is hydrogen the other is -OH or $-SCH_3$;
Q is $-CH_2-$, -O- or $NR^8$ where $R^8$ is hydrogen, $C_1$-$C_6$ alkyl, $C_2$-$C_6$ alkenyl, $C_3$-$C_8$ cycloalkyl, $-SO_2CH_3$ or $-(CH_2)_n-Y$, where n is an integer from 0 to 3, both inclusive, and Y is cyano, $OR^9$,

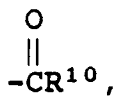

tetrazolyl, $-NR^{11}R^{12}$, -SH, $-S(C_1-C_4$ alkyl) or

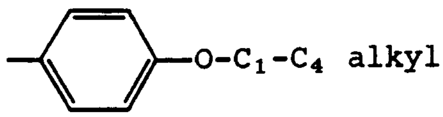

where $R^9$ is hydrogen, $C_1-C_4$ alkyl or

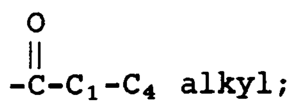

$R^{10}$ is $C_1-C_4$ alkyl, $C_1-C_4$ alkoxy or $-NH_2$; $R^{11}$ and $R^{12}$ are each independently hydrogen, $C_1-C_6$ alkyl, $C_2-C_6$ alkenyl, $C_2-C_6$ alkynyl, $-(CH_2)_qOH$, $-(CH_2)_q-N(C_1-C_4$ alkyl$)_2$, $-(CH_2)_q-S(C_1-C_4$ alkyl) or

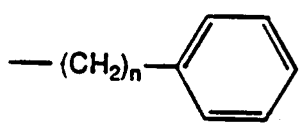

where n is as defined above and q is an integer from 1 to 6, both inclusive; or $R^{11}$ and $R^{12}$ taken together form a morpholinyl, piperidinyl, piperazinyl or N-methylpiperazinyl ring, comprising:

a) reacting the racemic sulfide compound with a reagent prepared from the combination of a tartrate ligand, a titanium alkoxide, a hydroperoxide and, optionally, water until substantially all of the undesired enantiomer of the sulfide substrate has been converted to its sulfoxide analog; and
b) separating the unreacted portion of the sulfide starting material, consisting essentially of substantially pure desired enantiomer, from the reaction mixture.

6. A process of Claim 5 which employs as starting material a racemic sulfide compound wherein $R^1$ and $R^2$ are each $C_1-C_6$ alkyl, $C_2-C_6$ alkenyl, $C_1-C_6$ alkoxy or

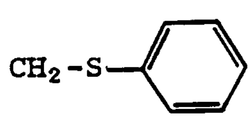 ;

$R^3$, $R^6$ and $R^7$ are hydrogen; and Q is $NR^8$ where $R^8$ is hydrogen, $C_1-C_6$ alkyl or $-(CH_2)_n-Y$ where n is 0 and Y is $NR^{11}R^{12}$ ($R^{11}$ and $R^{12}$ each being independently hydrogen or $C_1-C_6$ alkyl).

7. A process of Claim 5 or 6 which employs $Ti(O\text{-isopropyl})_4$ as the titanium oxide.

8. A process of any one of Claims 5 to 7 which employs diisopropyl tartrate as the tartrate ligand.

9. A process of any one of Claims 5 to 8 which employs t-butylhydroperoxide as the hydroperoxide.

10. A process for preparing a pharmaceutical formulation which comprises admixing a compound of the formula

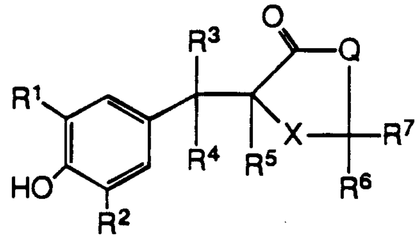

wherein:

$R^1$ is $C_2$-$C_6$ alkenyl; $C_2$-$C_6$ alkynyl or

$$-(CH_2)_n-S-$$ 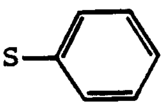

where n is an integer from 0 to 3, both inclusive;
$R^2$ is hydrogen, $C_1$-$C_6$ alkyl, $C_1$-$C_6$ alkoxy, $C_2$-$C_6$ alkenyl, $C_2$-$C_6$ alkynyl,

$$C_1-C_4\ \text{alkyl}-O-\overset{O}{\overset{||}{C}}-(C_1-C_4\ \text{alkyl})\ \text{or}\ -(CH_2)_n-S-$$ 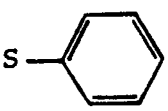

where n is an integer from 0 to 3, both inclusive;
$R^3$ is hydrogen or $C_1$-$C_6$ alkyl;
$R^4$ and $R^5$ are each hydrogen or when taken together form a bond;
$R^6$ and $R^7$ are each hydrogen or when taken together are =S, or when one of $R^6$ and $R^7$ is hydrogen the other is -OH or $-SCH_3$;
X is

$$-\overset{(O)_m}{\overset{||}{S}}-,$$

where m is 0, 1 or 2; and
Q is $-CH_2-$, -O- or $NR^8$ where $R^8$ is hydrogen, $C_1$-$C_6$ alkyl, $C_2$-$C_6$ alkenyl, $C_3$-$C_8$ cycloalkyl, $-SO_2CH_3$ or $-(CH_2)_n-Y$, where n is an integer from 0 to 3, both inclusive, and Y is cyano, $OR^9$,

$$-\overset{O}{\overset{||}{C}}R^{10},$$

tetrazolyl, $-NR^{11}R^{12}$, -SH, $-S(C_1$-$C_4$ alkyl) or

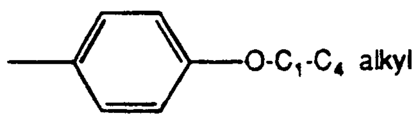

where $R^9$ is hydrogen, $C_1$-$C_4$ alkyl, or

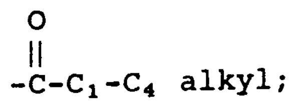

$R^{10}$ is $C_1$-$C_4$ alkyl, $C_1$-$C_4$ alkoxy or $-NH_2$; $R^{11}$ and $R^{12}$ are each independently hydrogen, $C_1$-$C_6$ alkyl, $C_2$-$C_6$ alkenyl, $C_2$-$C_6$ alkynyl, $-(CH_2)_qOH$, $-(CH_2)_q-N(C_1\text{-}C_4\ alkyl)_2$, $-(CH_2)_q-S(C_1\text{-}C_4\ alkyl)$ or

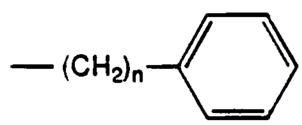

where n is as defined above and q is an integer from 1 to 6, both inclusive; or $R^{11}$ and $R^{12}$ taken together form a morpholinyl, piperidinyl, piperazinyl or N-methyl-piperazinyl ring; or a pharmaceutically acceptable salt thereof, with one or more pharmaceutically-acceptable carriers, diluents or excipients therefor.

**Patentansprüche**

**Patentansprüche für folgende Vertragsstaaten : AT, BE, CH, DK, FR, GB, IT, LI, LU, NL, SE**

**1.** Verbindung der Formel (II)

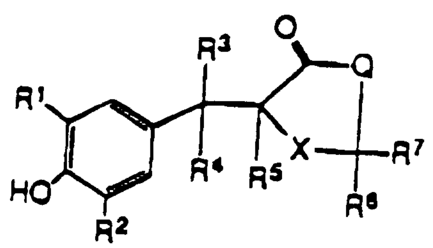

(II)

worin

$R^1$ für $C_2$-$C_6$ Alkenyl, $C_2$-$C_6$ Alkinyl oder

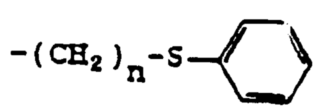

steht, worin n für eine ganze Zahl von 0 bis 3 steht, beide inbegriffen,

$R^2$ für Wasserstoff $C_1$-$C_6$ Alkyl, $C_1$-$C_6$ Alkoxy, $C_2$-$C_6$ Alkenyl, $C_2$-$C_6$ Alkinyl,

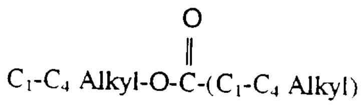 oder 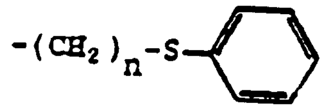

steht, worin n für eine ganze Zahl von 0 bis 3 steht, beide inbegriffen,

$R^3$ für Wasserstoff oder $C_1$-$C_6$ Alkyl steht,

$R^4$ und $R^5$ jeweils für Wasserstoff stehen oder zusammengenommen eine Bindung bilden,

$R^6$ und $R^7$ jeweils für Wasserstoff stehen oder zusammengenommen für =S stehen, oder wenn eines von $R^6$ und $R^7$ für Wasserstoff steht, das andere für -OH oder $-SCH_3$ steht, X für

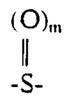

steht, worin m für 0, 1 oder 2 steht und

Q für $-CH_2$-, -O- oder $NR^8$ steht, worin $R^8$ für Wasserstoff, $C_1$-$C_6$ Alkyl, $C_2$-$C_6$ Alkenyl, $C_3$-$C_8$ Cycloalkyl, $-SO_2CH_3$ oder $-(CH_2)_n$-Y steht, worin n für eine ganze Zahl von 0 bis 3 steht, beide inbegriffen, und

Y steht für Cyano, $OR^9$,

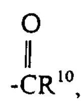

Tetrazolyl, $-NR^{11}R^{12}$, -SH, -S($C_1$-$C_4$ Alkyl) oder

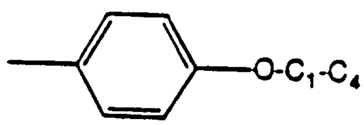

worin

$R^9$ für Wasserstoff, $C_1$-$C_4$ Alkyl oder

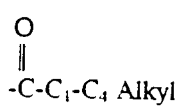

steht,

$R^{10}$ für $C_1$-$C_4$ Alkyl, $C_1$-$C_4$ Alkoxy oder $-NH_2$ steht,

$R^{11}$ und $R^{12}$ jeweils unabhängig voneinander stehen für Wasserstoff, $C_1$-$C_6$ Alkyl, $C_2$-$C_6$ Alkenyl, $C_2$-$C_6$ Alkinyl, $-(CH_2)_qOH$, $-(CH_2)_q-N(C_1-C_4\ Alkyl)_2$, $-(CH_2)_q-S(C_1-C_4\ Alkyl)$ oder

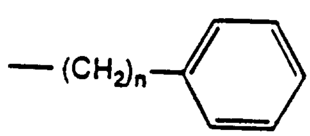

worin n wie oben definiert ist und q für eine ganze Zahl von 1 bis 6 steht, beide inbegriffen, oder

$R^{11}$ und $R^{12}$ zusammengenommen einen Morpholinyl-, Piperidinyl-, Piperazinyl- oder N-Methylpiperazinylring

bilden,
oder ein pharmazeutisch annehmbares Salz hiervon.

**2.** Verbindung nach Anspruch 1, worin

$R^1$ für $C_2$-$C_6$ Alkenyl, $C_2$-$C_6$ Alkinyl oder

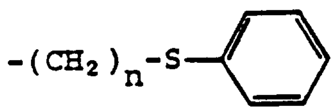

steht, worin n für eine ganze Zahl von 0 bis 3 steht, beide inbegriffen,

$R^2$ für Wasserstoff, $C_1$-$C_6$ Alkyl, $C_1$-$C_6$ Alkoxy, $C_2$-$C_6$ Alkenyl, $C_2$-$C_6$ Alkinyl, oder

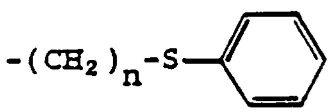

steht,
worin n für eine ganze Zahl von 0 bis 3 steht, beide inbegriffen,

$R^3$ für Wasserstoff oder $C_1$-$C_6$ Alkyl steht,

$R^4$ und $R^5$ jeweils für Wasserstoff stehen oder zusammengenommen eine Bindung bilden,

$R^6$ und $R^7$ jeweils für Wasserstoff stehen oder zusammengenommen für =S stehen, oder wenn eines von $R^6$ und $R^7$ für Wasserstoff steht, das andere für -OH oder $-SCH_3$ steht,

X für

$$\overset{(O)_m}{\overset{\|}{-S-}}$$

steht, worin m für 0, 1 oder 2 steht und

Q für $-CH_2$-, -O- oder $NR^8$ steht, worin $R^8$ für Wasserstoff, $C_1$-$C_6$ Alkyl, $C_3$-$C_8$ Cycloalkyl, $-SO_2CH_3$ oder $-(CH_2)_n$-Y steht, worin n für eine ganze Zahl von 0 bis 3 steht, beide inbegriffen, und

Y steht für Cyano,

$$OR^9, \overset{O}{\overset{\|}{-CR^{10}}},$$

Tetrazolyl, $-NR^{11}R^{12}$ oder

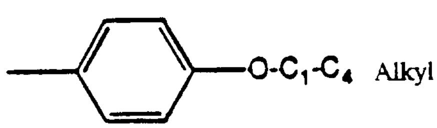

worin

$R^9$ für Wasserstoff, $C_1$-$C_4$ Alkyl oder

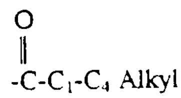

steht,

$R^{10}$ für $C_1$-$C_4$ Alkyl, $C_1$-$C_4$ Alkoxy oder $-NH_2$ steht,

$R^{11}$ und $R^{12}$ jeweils unabhängig voneinander stehen für Wasserstoff, $C_1$-$C_6$ Alkyl, $-(CH_2)_qOH$, $-(CH_2)_q-N(C_1-C_4\ Alkyl)_2$, $-(CH_2)_q-S(C_1-C_4\ Alkyl)$ oder

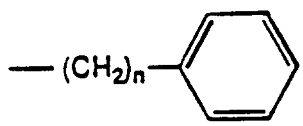

worin n wie oben definiert ist und q für eine ganze Zahl von 1 bis 6 steht, beide inbegriffen, oder

$R^{11}$ und $R^{12}$ zusammengenommen einen Morpholinyl-, Piperidinyl-, Piperazinyl- oder N-Methylpiperazinylring bilden,

oder ein pharmazeutisch annehmbares Salz hiervon.

3. Verbindung nach Anspruch 2, worin $R^1$ für $C_2$-$C_6$ Alkenyl steht, $R^2$ für $C_2$-$C_6$ Alkenyl oder $C_1$-$C_6$ Alkyl steht, $R^3$ für Wasserstoff steht, $R^4$ und $R^5$ für Wasserstoff stehen oder zusammengenommen eine Bindung bilden, $R^6$ und $R^7$ jeweils für Wasserstoff stehen oder zusammengenommen für =S stehen,

X für

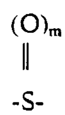

steht, worin m für 0 steht und Q für -O- oder $NR^8$ steht, worin $R^8$ wie in Anspruch 2 definiert ist und pharmazeutisch annehmbare Salze hiervon.

4. Pharmazeutische Zusammensetzung, die als Wirkstoff eine Verbindung nach einem der Ansprüche 1 bis 3 oder ein pharmazeutisch annehmbares Salz hiervon zusammen mit einem oder mehreren pharmazeutisch annehmbaren Verdünnungsmitteln, Hilfsstoffen oder Trägern hierfür enthält.

5. Verbindung nach einem der Ansprüche 1 bis 3 zur Verwendung bei der Behandlung der entzündlichen Darmerkrankung.

6. Verfahren zur Herstellung einer Verbindung der Formel II nach einem der Ansprüche 1 bis 3, gekennzeichnet durch

(A) Umsetzung einer Verbindung der Formel

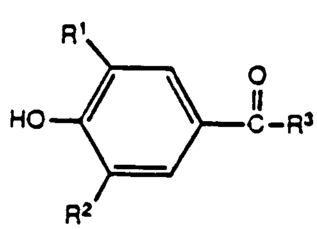

mit einer Verbindung der Formel

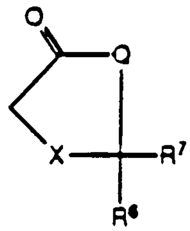

worin $R^1$, $R^2$ $R^3$ und X wie in einem der Ansprüche 1 bis 3 definiert sind, Q für $-CH_2-$ oder $NR^8$ steht (worin $R^8$ wie in einem der Ansprüche 1 bis 3 definiert ist) und $R^6$ und $R^7$ zusammengenommen für =S stehen, unter Bildung einer Verbindung der Formel

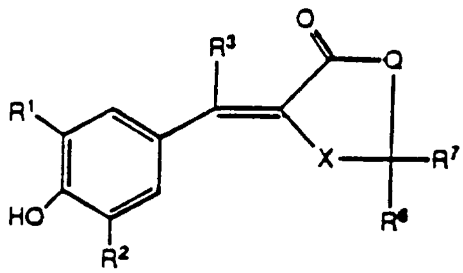

worin $R^1$, $R^2$, $R^3$, $R^6$, $R^7$, X und Q wie oben definiert sind,

(B) Reduktion einer Verbindung der Formel II, worin $R^6$ und $R^7$ zusammengenommen für =S stehen unter Bildung einer Verbindung der Formel II, worin $R^6$ und $R^7$ für Wasserstoff stehen,

(C) Reduktion einer Verbindung der Formel II, worin $R^4$ und $R^5$ zusammengenommen unter Bildung einer Verbindung der Formel II eine Bindung bilden, worin $R^4$ und $R^5$ für Wasserstoff stehen,

(D) Reduktion einer Verbindung der Formel II, worin $R^4$ und $R^5$ zusammengenommen eine Bindung bilden und $R^6$ und $R^7$ zusammengenommen für =S stehen, unter Bildung einer Verbindung der Formel II, worin $R^4$, $R^5$, $R^6$ und $R^7$ alle für Wasserstoff stehen,

(E) Alkylierung einer Verbindung der Formel II, worin $R^8$ für Wasserstoff steht unter Bildung einer Verbindung der Formel II, worin $R^8$ für $C_1$-$C_6$ Alkyl, $C_2$-$C_6$ Alkenyl, $C_3$-$C_8$ Cycloalkyl oder $-(CH_2)_n-Y$ steht (worin n für eine ganze Zahl von 0 bis 3 steht, beide inbegriffen, und Y für Cyano, $OR^9$, -SH, $-S(C_1$-$C_4$ Alkyl), $-NR^{11}R^{12}$ oder

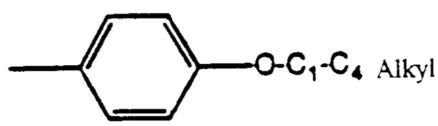

steht, worin $R^9$, $R^{11}$ und $R^{12}$ wie in einem der Ansprüche 1 bis 3 definiert sind)

(F) Acylierung einer Verbindung der Formel II, worin $R^8$ für Wasserstoff steht, unter Bildung einer Verbindung der Formel II, worin $R^8$ für $-(CH_2)_n-Y$ steht, worin n für eine ganze Zahl von 0 bis 3 steht, beide inbegriffen, und Y für

$$-\overset{O}{\overset{\|}{C}}R^{10}$$

steht, worin $R^{10}$ wie in einem der Ansprüche 1 bis 3 definiert ist,

(G) Oxidation einer Verbindung der Formel II, worin X für

$$\overset{(O)_m}{\overset{\|}{-S-}}$$

steht, worin m für 0 steht, unter Bildung einer Verbindung der Formel II, worin X für

$$\overset{(O)_m}{\overset{\|}{-S-}}$$

steht und m für 1 steht,

(H) Oxidation einer Verbindung der Formel II, worin X für

$$\overset{(O)_m}{\overset{\|}{-S-}}$$

steht, worin m für 0 steht, unter Bildung einer Verbindung der Formel II, worin X für

$$\overset{(O)_m}{\overset{\|}{-S-}}$$

steht und m für 2 steht,

(I) Oxidation einer Verbindung der Formel II, worin X für

$$\overset{(O)_m}{\overset{\|}{-S-}}$$

steht, worin m für 1 steht, unter Bildung einer Verbindung der Formel II, worin X für

$$\overset{(O)_m}{\overset{\|}{-S-}}$$

steht und m für 2 steht,

(J) Umsetzung einer Verbindung der Formel

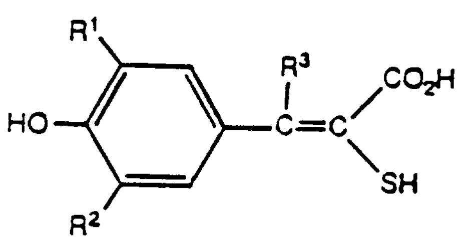

mit

i) Ameisensäure unter Bildung einer Verbindung der Formel II, worin Q für O steht, $R^4$ und $R^5$ zusammengenommen eine Bindung bilden und $R^6$ und $R^7$ für Wasserstoff stehen, oder

ii) Schwefelkohlenstoff unter Bildung einer Verbindung der Formel II, worin Q für O steht, $R^3$ und $R^4$ zusammengenommen eine Bindung bilden und $R^6$ und $R^7$ zusammengenommen für =S stehen,

(K) Umsetzung einer Verbindung der Formel

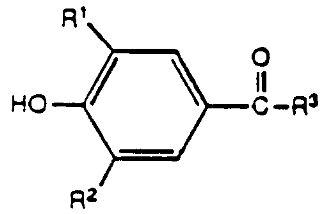

mit einer Verbindung der Formel

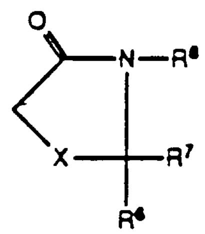

worin $R^1$, $R^2$, $R^3$ und X wie in einem der Ansprüche 1 bis 3 definiert sind,

$R^6$ und $R^7$ zusammengenommen für =S stehen und $R^8$ für $-(CH_2)_n$-Y steht (worin n für eine ganze Zahl von 0 bis 3 steht, beide inbegriffen, und Y für $OR^9$ steht, worin $R^9$ für Wasserstoff steht) unter Bildung einer Verbindung der Formel

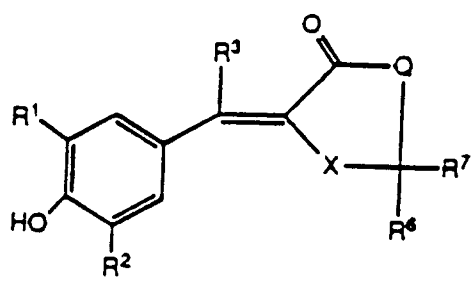

worin $R^1$, $R^2$, $R^3$, $R^6$, $R^7$ und X wie oben definiert sind und $R^8$ für $-(CH_2)_n$-Y steht (worin n für eine ganze Zahl von
0 bis 3 steht, beide inbegriffen und Y für $OR^9$ steht, worin $R^9$ für

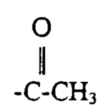

steht),

(L) Reduktion einer Verbindung der Formel II, worin $R^8$ für $-(CH_2)_n$-Y steht, worin n für 0 bis 3 steht, beide inbegriffen und Y für $OR^9$ steht, worin $R^9$ für

$$-\overset{\overset{\displaystyle O}{\|}}{C}-C_1-C_4\text{ Alkyl}$$

steht, unter Bildung einer Verbindung der Formel II, worin $R^8$ für $-(CH_2)_n$-Y steht, worin n für 0 bis 3 steht, beide inbegriffen, und Y für $OR^9$ steht, worin $R^9$ ihr Wasserstoff steht,

(M) Umsetzung einer Verbindung der Formel II, worin $R^8$ für $-(CH_2)_n$-Y steht, worin n für 0 bis 3 steht, beide inbegriffen, und Y für $-OR^9$ steht, worin $R^9$ ihr Wasserstoff steht, mit einem Tosylhalogenid unter Bildung einer Verbindung der Formel II, worin $R^8$ für $-(CH_2)_n$-Y steht, worin n für 0 bis 3 steht, beide inbegriffen, und Y für $OR^9$ steht, worin $R^9$ für Tosyl steht,

(N) Umsetzung einer Verbindung der Formel II, worin $R^8$ für $-(CH_2)_n$-Y steht, worin n für 0 bis 3 steht, beide inbegriffen, und Y für $-OR^9$ steht, worin $R^9$ für Tosyl steht, mit einem Amin der Formel $HNR^{11}R^{12}$ (worin $R^{11}$ und $R^{12}$ wie oben in einem der Ansprüche 1 bis 3 definiert sind) unter Bildung einer Verbindung der Formel II, worin $R^8$ für $-(CH_2)_n$-Y steht, worin n für 0 bis 3 steht, beide inbegriffen, und Y für $-NR^{11}R^{12}$ steht,

(O) Behandlung einer Verbindung der Formel II, worin $R^8$ für $-(CH_2)_n$-Y steht, worin n für 0 bis 3 steht, beide inbegriffen, und Y für Cyano steht mit Tri-n-butylzinnazid unter Bildung einer Verbindung der Formel II, worin $R^8$ für $-(CH_2)_n$-Y steht, worin n für 0 bis 3 steht, beide inbegriffen, und Y für Tetrazolyl steht,

(P) Umsetzung einer Verbindung der Formel

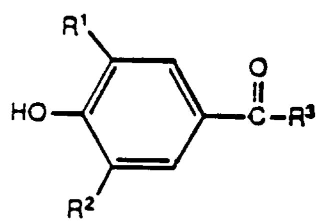

mit einer Verbindung der Formel

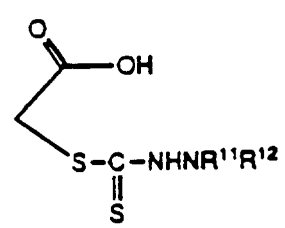

worin $R^1$, $R^2$, $R^3$, $R^{11}$ und $R^{12}$ wie in einem der Ansprüche 1 bis 3 definiert sind unter Bildung einer Verbindung der Formel

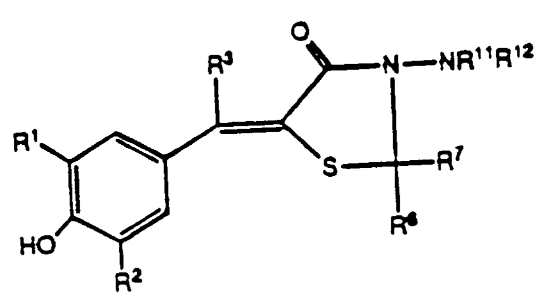

worin $R^6$ und $R^7$ zusammengenommen für =S stehen und $R^1$, $R^2$, $R^3$, $R^{11}$ und $R^{12}$ wie in einem der Ansprüche 1 bis 3 definiert sind,

(Q) Erhitzen einer Verbindung der Formel II, worin $R^8$ für $-(CH_2)_n$-Y steht und Y für $-NR^{11}R^{12}$ steht (wobei keines von $R^{11}$ oder $R^{12}$ für Wasserstoff steht) in einem Ethanol / Wasser Gemisch in Gegenwart eines Katalysators unter Bildung einer Verbindung der Formel II, worin $R^8$ für $-(CH_2)_n$-Y steht und Y für $-NR^{11}R^{12}$ steht (worin eines von $R^{11}$ oder $R^{12}$ ihr Wasserstoff und das andere nicht ihr Wasserstoff steht),

(R) Umsetzung einer Verbindung der Formel II, worin $R^6$ und $R^7$ beide für Wasserstoff stehen, mit Trifluoressigsäureanhydrid unter Bildung einer Verbindung der Formel II, worin eines von $R^6$ und $R^7$ für Wasserstoff steht und das andere für -OH steht,

(S) Salzbildung einer Verbindung der Formel II durch Umsetzung der nicht-Salzform der Verbindung mit entweder einer starken Säure oder einer starken Base.

7. Verfahren zur selektiven Isolierung einer im wesentlichen reinen Enantiomerform eines der Enantiomere eines razemischen Gemisches einer Verbindung der Formel

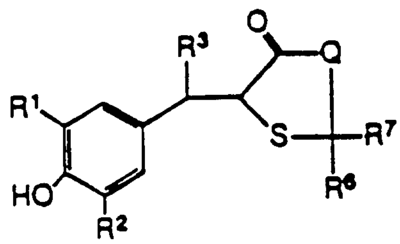

worin

$R^1$ und $R^2$ jeweils unabhängig für Wasserstoff, $C_1$-$C_6$ Alkyl, $C_1$-$C_6$ Alkoxy, $C_2$-$C_6$ Alkenyl, $C_2$-$C_6$ Alkinyl,

$C_1$-$C_4$ Alkyl-O-$\overset{O}{\overset{\|}{C}}$-($C_1$-$C_4$ Alkyl) oder 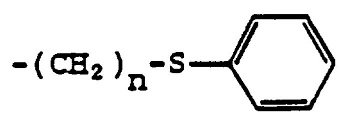

steht, worin n für eine ganze Zahl von 0 bis 3 steht, beide inbegriffen,

$R^3$ für Wasserstoff oder $C_1$-$C_6$ Alkyl steht,

$R^6$ und $R^7$ jeweils für Wasserstoff stehen oder zusammengenommen für =S stehen, oder wenn eines von $R^6$ und $R^7$ für Wasserstoff steht, das andere für -OH oder $-SCH_3$ steht,

Q für $-CH_2$-, -O- oder $NR^8$ steht, worin $R^8$ für Wasserstoff, $C_1$-$C_6$ Alkyl, $C_2$-$C_6$ Alkenyl, $C_3$-$C_8$ Cycloalkyl, $-SO_2CH_3$ oder $-(CH_2)_n$-Y steht, worin n für eine ganze Zahl von 0 bis 3 steht, beide inbegriffen, und

Y steht für Cyano, $OR^9$,

$$-\overset{O}{\overset{\|}{C}}R^{10},$$

Tetrazolyl, $-NR^{11}R^{12}$, -SH, -S($C_1$-$C_4$ Alkyl) oder

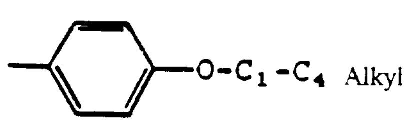

worin

$R^9$ für Wasserstoff, $C_1$-$C_4$ Alkyl oder

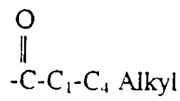

steht,

$R^{10}$ für $C_1$-$C_4$ Alkyl, $C_1$-$C_4$ Alkoxy oder $-NH_2$ steht,

$R^{11}$ und $R^{12}$ jeweils unabhängig voneinander stehen für Wasserstoff, $C_1$-$C_6$ Alkyl, $C_2$-$C_6$ Alkenyl, $C_2$-$C_6$ Alkinyl, $-(CH_2)_qOH$, $-(CH_2)_q-N(C_1$-$C_4$ Alkyl$)_2$, $-(CH_2)_q-S(C_1$-$C_4$ Alkyl) oder

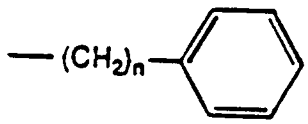

worin n wie oben definiert ist und q für eine ganze Zahl von 1 bis 6 steht, beide inbegriffen, oder

$R^{11}$ und $R^{12}$ zusammengenommen einen Morpholinyl-, Piperidinyl-, Piperazinyl- oder N-Methylpiperazinylring bilden,

gekennzeichnet durch

a) Umsetzung der razemischen Sulfidverbindung mit einem Reagenz, das durch die Kombination eines Tartratliganden, eines Titanalkoxids, eines Hydroperoxids und wahlweise Wasser hergestellt wurde, bis im wesentlichen das gesamte unerwünschte Enantiomer des Sulfidsubstrats in dessen Sulfoxidanalogon umgewandelt wurde, und

b) Abtrennung des nicht abreagierten Teils des Sulfidausgangsmaterials, das im wesentlichen aus reinem gewünschtem Enantiomer besteht, aus dem Reaktionsgemisch.

**8.** Verwendung einer Verbindung der Formel

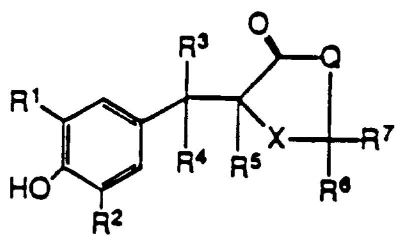

worin

$R^1$ und $R^2$ jeweils unabhängig stehen für Wasserstoff, $C_1$-$C_6$ Alkyl, $C_1$-$C_6$ Alkoxy, $C_2$-$C_6$ Alkenyl, $C_2$-$C_6$ Alkinyl,

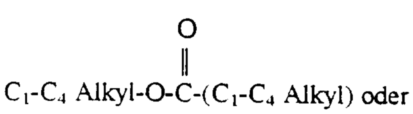 oder 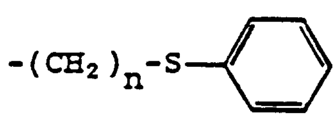 ,

worin n für eine ganze Zahl von 0 bis 3 steht, beide inbegriffen,

$R^3$ für Wasserstoff oder $C_1$-$C_6$ Alkyl steht,

$R^4$ und $R^5$ jeweils für Wasserstoff stehen oder zusammengenommen eine Bindung bilden,

$R^6$ und $R^7$ jeweils für Wasserstoff stehen oder zusammengenommen für =S stehen, oder wenn eines von $R^6$ und $R^7$ für Wasserstoff steht, das andere für -OH oder $-SCH_3$ steht,

X für

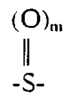

steht, worin in für 0, 1 oder 2 steht und

Q für $-CH_2-$, -O- oder $NR^8$ steht, worin $R^8$ für Wasserstoff, $C_1$-$C_6$ Alkyl , $C_2$-$C_6$ Alkenyl, $C_3$-$C_8$ Cycloalkyl, $-SO_2CH_3$ oder $-(CH_2)_n$-Y steht, worin n für eine ganze Zahl von 0 bis 3 steht, beide inbegriffen, und

Y steht für Cyano, $OR^9$,

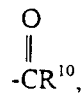

Tetrazolyl, $-NR^{11}R^{12}$, -SH, -S($C_1$-$C_4$ Alkyl) oder

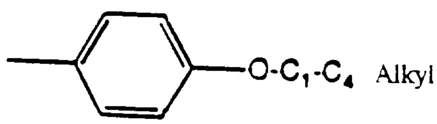

worin

$R^9$ für Wasserstoff, $C_1$-$C_4$ Alkyl oder

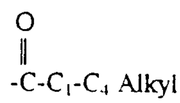

steht,

$R^{10}$ für $C_1$-$C_4$ Alkyl, $C_1$-$C_4$ Alkoxy oder $-NH_2$ steht,

$R^{11}$ und $R^{12}$ jeweils unabhängig voneinander stehen für Wasserstoff, $C_1$-$C_6$ Alkyl, $C_2$-$C_6$ Alkenyl, $C_2$-$C_6$ Alkinyl, $-(CH_2)_qOH$, $-(CH_2)_q-N(C_1-C_4\ Alkyl)_2$, $-(CH_2)_q-S(C_1-C_4\ Alkyl)$ oder

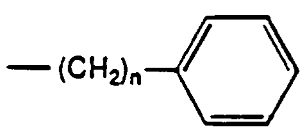

worin n wie oben definiert ist und q für eine ganze Zahl von 1 bis 6 steht, beide inbegriffen, oder

$R^{11}$ und $R^{12}$ zusammengenommen einen Morpholinyl-, Piperidinyl-, Piperazinyl- oder N-Methylpiperazinylring bilden,

oder eines pharmazeutisch annehmbaren Salzes hiervon zur Herstellung eines Arzneimittels gegen entzündliche Darmerkrankung.

9. Verwendung einer Verbindung nach Anspruch 8, worin

$R^1$ und $R^2$ jeweils unabhängig stehen für Wasserstoff, $C_1$-$C_6$ Alkyl, $C_1$-$C_6$ Alkoxy, $C_2$-$C_6$ Alkenyl, $C_2$-$C_6$ Alkinyl oder

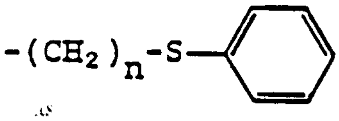 ,

worin n für eine ganze Zahl von 0 bis 3 steht, beide inbegriffen,

$R^3$ für Wasserstoff oder $C_1$-$C_6$ Alkyl steht,

$R^4$ und $R^5$ jeweils für Wasserstoff stehen oder zusammengenommen eine Bindung bilden,

$R^6$ und $R^7$ jeweils für Wasserstoff stehen oder zusammengenommen für =S stehen, oder wenn eines von $R^6$ und $R^7$ für Wasserstoff steht, das andere für -OH oder -$SCH_3$ steht,

X für

(O)$_m$
||
-S-

steht, worin m für 0, 1 oder 2 steht und

Q für -$CH_2$-, -O- oder $NR^8$ steht, worin $R^8$ für Wasserstoff, $C_1$-$C_6$ Alkyl, $C_3$-$C_8$ Cycloalkyl, -$SO_2CH_3$ oder -$(CH_2)_n$-Y steht, worin n für eine ganze Zahl von 0 bis 3 steht, beide inbegriffen, und

Y steht für Cyano, $OR^9$,

O
||
-$CR^{10}$,

Tetrazolyl, -$NR^{11}R^{12}$, oder

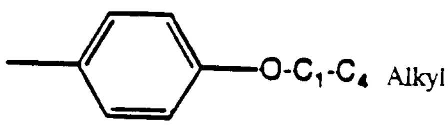

worin

$R^9$ für Wasserstoff, $C_1$-$C_4$ Alkyl oder

O
||
-C-$C_1$-$C_4$ Alkyl

steht,

$R^{10}$ für $C_1$-$C_4$ Alkyl, $C_1$-$C_4$ Alkoxy oder -$NH_2$ steht,

$R^{11}$ und $R^{12}$ jeweils unabhängig voneinander stehen für Wasserstoff, $C_1$-$C_6$ Alkyl, -$(CH_2)_q$OH, -$(CH_2)_q$-N($C_1$-$C_4$ Alkyl)$_2$, -$(CH_2)_q$-S($C_1$-$C_4$ Alkyl) oder

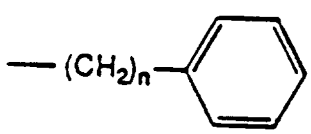

worin n wie oben definiert ist und q für eine ganze Zahl von 1 bis 6 steht, beide inbegriffen, oder

$R^{11}$ und $R^{12}$ zusammengenommen einen Morpholinyl-, Piperidinyl-, Piperazinyl- oder N-Methylpiperazinylring

bilden,
oder eines pharmazeutisch annehmbaren Salzes hiervon zur Herstellung eines Arzneimittels gegen entzündliche Darmerkrankung.

10. Verwendung von 5-[[3,5-Bis(1,1-dimethylethyl)-4-hydroxyphenyl]methyl]-4-thiazolidinon zur Herstellung eines Arzneimittels gegen entzündliche Darmerkrankung.

**Patentansprüche für folgende Vertragsstaaten : ES, GR**

1. Verfahren zur Herstellung einer Verbindung der Formel (II)

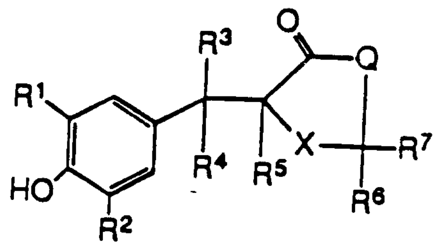

(II)

worin

R¹ für $C_2$-$C_6$ Alkenyl, $C_2$-$C_6$ Alkinyl oder

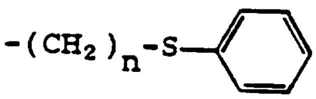

steht, worin n für eine ganze Zahl von 0 bis 3 steht, beide inbegriffen,

$R^2$ für Wasserstoff, $C_1$-$C_6$ Alkyl, $C_1$-$C_6$ Alkoxy, $C_2$-$C_6$ Alkenyl, $C_2$-$C_6$ Alkinyl,

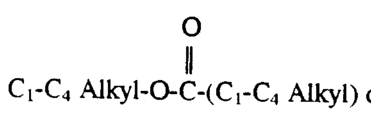

oder

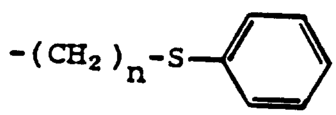

steht, worin n für eine ganze Zahl von 0 bis 3 steht, beide inbegriffen,

$R^3$ für Wasserstoff oder $C_1$-$C_6$ Alkyl steht,

$R^4$ und $R^5$ jeweils für Wasserstoff stehen oder zusammengenommen eine Bindung bilden,

$R^6$ und $R^7$ jeweils für Wasserstoff stehen oder zusammengenommen für =S stehen, oder wenn eines von $R^6$ und $R^7$ für Wasserstoff steht, das andere für -OH oder -$SCH_3$ steht,

X für

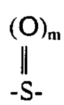

steht, worin m für 0, 1 oder 2 steht und

Q für -$CH_2$-, -O- oder $NR^8$ steht, worin $R^8$ für Wasserstoff, $C_1$-$C_6$ Alkyl, $C_2$-$C_6$ Alkenyl, $C_3$-$C_8$ Cycloalkyl, -$SO_2CH_3$ oder -$(CH_2)_n$-Y steht, worin n für eine ganze Zahl von 0 bis 3 steht, beide inbegriffen, und

Y steht für Cyano, $OR^9$

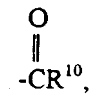

Tetrazolyl, $-NR^{11}R^{12}$, -SH, $-S(C_1-C_4$ Alkyl) oder

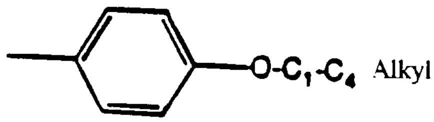

worin

$R^9$ für Wasserstoff, $C_1-C_4$ Alkyl oder

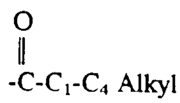

steht,

$R^{10}$ für $C_1-C_4$ Alkyl, $C_1-C_4$ Alkoxy oder $-NH_2$, steht,

$R^{11}$ und $R^{12}$ jeweils unabhängig voneinander stehen für Wasserstoff, $C_1-C_6$ Alkyl, $C_2-C_6$ Alkenyl, $C_2-C_6$ Alkinyl, $-(CH_2)_qOH$, $-(CH_2)_q-N(C_1-C_4$ $Alkyl)_2$, $-(CH_2)_q-S(C_1-C_4$ Alkyl) oder

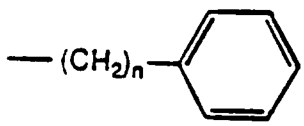

worin n wie oben definiert ist und q für eine ganze Zähl von 1 bis 6 steht, beide inbegriffen, oder

$R^{11}$ und $R^{12}$ zusammengenommen einen Morpholinyl-, Piperidinyl-, Piperazinyl- oder N-Methylpiperazinylring bilden,

oder eines pharmazeutisch annehmbaren Salzes hiervon, gekennzeichnet durch

(A) Umsetzung einer Verbindung der Formel

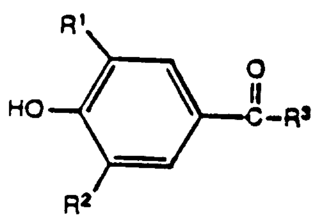

mit einer Verbindung der Formel

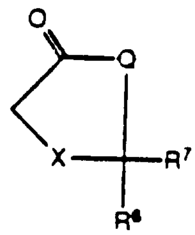

worin $R^1$, $R^2$, $R^3$ und X wie oben definiert sind, Q für $-CH_2-$ oder $NR^8$ steht (worin $R^8$ wie oben definiert ist) und $R^6$ und $R^7$ zusammengenommen für =S stehen, unter Bildung einer Verbindung der Formel

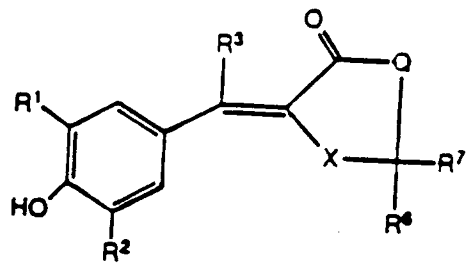

worin $R^1$, $R^2$, $R^3$, $R^6$, $R^7$, X und Q wie oben definiert sind,

(B) Reduktion einer Verbindung der Formel II, worin $R^6$ und $R^7$ zusammengenommen für =S stehen unter Bildung einer Verbindung der Formel II, worin $R^6$ und $R^7$ für Wasserstoff stehen,

(C) Reduktion einer Verbindung der Formel II, worin $R^4$ und $R^5$ zusammengenommen unter Bildung einer Verbindung der Formel II eine Bindung bilden, worin $R^4$ und $R^5$ für Wasserstoff stehen,

(D) Reduktion einer Verbindung der Formel II, worin $R^4$ und $R^5$ zusammengenommen eine Bindung bilden und $R^6$ und $R^7$ zusammengenommen für =S stehen, unter Bildung einer Verbindung der Formel II, worin $R^4$, $R^5$, $R^6$ und $R^7$ alle für Wasserstoff stehen,

(E) Alkylierung einer Verbindung der Formel II, worin $R^8$ für Wasserstoff steht unter Bildung einer Verbindung der Formel II, worin $R^8$ für $C_1$-$C_6$ Alkyl, $C_2$-$C_6$ Alkenyl, $C_3$-$C_8$ Cycloalkyl oder $-(CH_2)_n-Y$ steht (worin n für eine ganze Zahl von 0 bis 3 steht, beide inbegriffen, und Y für Cyano, $OR^9$, -SH, -S($C_1$-$C_4$ Alkyl), $-NR^{11}R^{12}$ oder

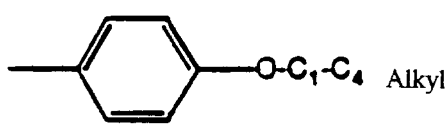

steht, worin $R^9$, $R^{11}$ und $R^{12}$ wie oben definiert sind)

(F) Acylierung einer Verbindung der Formel II, worin $R^8$ für Wasserstoff steht, unter Bildung einer Verbindung der Formel II, worin $R^8$ für $-(CH_2)_n-Y$ steht, worin n für eine ganze Zahl von 0 bis 3 steht, beide inbegriffen, und Y für

$$\overset{O}{\overset{\|}{-CR^{10}}}$$

steht, worin $R^{10}$ wie oben definiert ist,

(G) Oxidation einer Verbindung der Formel II, worin X für

$$\overset{(O)_m}{\overset{\|}{-S-}}$$

steht, worin m für 0 steht, unter Bildung einer Verbindung der Formel II, worin X für

$$\overset{(O)_m}{\overset{\|}{-S-}}$$

steht und m für 1 steht,

(H) Oxidation einer Verbindung der Formel II, worin X für

$$\overset{(O)_m}{\overset{\|}{-S-}}$$

steht, worin m für 0 steht, unter Bildung einer Verbindung der Formel II, worin X für

$$\overset{(O)_m}{\overset{\|}{-S-}}$$

steht und m für 2 steht,

(I) Oxidation einer Verbindung der Formel II, worin X für

$$\overset{(O)_m}{\overset{\|}{-S-}}$$

steht, worin m für 1 steht, unter Bildung einer Verbindung der Formel II, worin X für

$$\overset{(O)_m}{\overset{\|}{-S-}}$$

steht und m für 2 steht,

(J) Umsetzung einer Verbindung der Formel

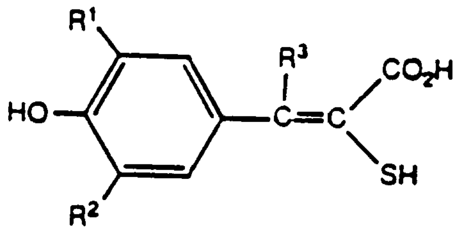

mit

i) Ameisensäure unter Bildung einer Verbindung der Formel II, worin Q für O steht, $R^4$ und $R^5$ zusammengenommen eine Bindung bilden und $R^6$ und $R^7$ für Wasserstoff stehen, oder
ii) Schwefelkohlenstoff unter Bildung einer Verbindung der Formel II, worin Q für O steht, $R^3$ und $R^4$ zusammengenommen eine Bindung bilden und $R^6$ und $R^7$ zusammengenommen für =S stehen,

(K) Umsetzung einer Verbindung der Formel

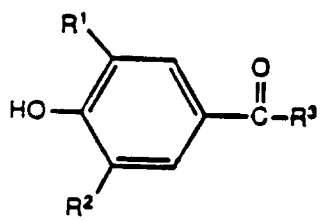

mit einer Verbindung der Formel

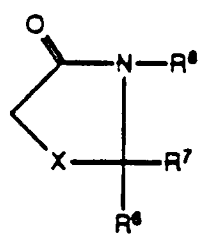

worin $R^1$, $R^2$, $R^3$ und X wie in oben definiert sind,

$R^6$ und $R^7$ zusammengenommen für =S stehen und $R^8$ für $-(CH_2)_n-Y$ steht (worin n für eine ganze Zahl von 0 bis 3 steht, beide inbegriffen, und Y für $OR^9$ steht, worin $R^9$ für Wasserstoff steht) unter Bildung einer Verbindung der Formel

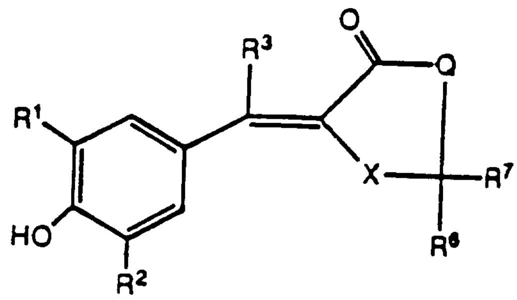

worin $R^1$, $R^2$, $R^3$, $R^6$, $R^7$ und X wie oben definiert sind und $R^8$ für $-(CH_2)_n$-Y steht (worin n für eine ganze Zahl von 0 bis 3 steht, beide inbegriffen und Y für $OR^9$ steht, worin $R^9$ für

O
‖
$-C-CH_3$

steht),

(L) Reduktion einer Verbindung der Formel II, worin $R^8$ für $-(CH_2)_n$-Y steht, worin n für 0 bis 3 steht, beide inbegriffen und Y für $OR^9$ steht, worin $R^9$ für

O
‖
$-C-C_1-C_4$ Alkyl

steht, unter Bildung einer Verbindung der Formel II, worin $R^8$ für $-(CH_2)_n$-Y steht, worin n für 0 bis 3 steht, beide inbegriffen, und Y für $OR^9$ steht, worin $R^9$ für Wasserstoff steht,

(M) Umsetzung einer Verbindung der Formel II, worin $R^8$ für $-(CH_2)_n$-Y steht, worin n für 0 bis 3 steht, beide inbegriffen, und Y für $-OR^9$ steht, worin $R^9$ für Wasserstoff steht, mit einem Tosylhalogenid unter Bildung einer Verbindung der Formel II, worin $R^8$ ihr $-(CH_2)_n$-Y steht, worin n für 0 bis 3 steht, beide inbegriffen, und Y für $OR^9$ steht, worin $R^9$ für Tosyl steht,

(N) Umsetzung einer Verbindung der Formel II, worin $R^8$ für $-(CH_2)_n$-Y steht, worin n für 0 bis 3 steht, beide inbegriffen, und Y für $-OR^9$ steht, worin $R^9$ für Tosyl steht, mit einem Amin der Formel $HNR^{11}R^{12}$ (worin $R^{11}$ und $R^{12}$ wie oben definiert sind) unter Bildung einer Verbindung der Formel II, worin $R^8$ für $-(CH_2)_n$-Y steht, worin n für 0 bis 3 steht, beide inbegriffen, und Y für $-NR^{11}R^{12}$ steht,

(O) Behandlung einer Verbindung der Formel II, worin $R^8$ für $-(CH_2)_n$-Y steht, worin n für 0 bis 3 steht, beide inbegriffen, und Y für Cyano steht mit Tri-n-butylzinnazid unter Bildung einer Verbindung der Formel II, worin $R^8$ für $-(CH_2)_n$-Y steht, worin n für 0 bis 3 steht, beide inbegriffen, und Y für Tetrazolyl steht,

(P) Umsetzung einer Verbindung der Formel

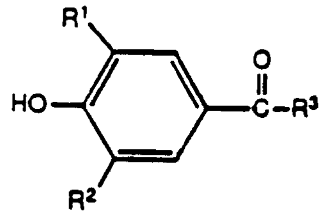

mit einer Verbindung der Formel

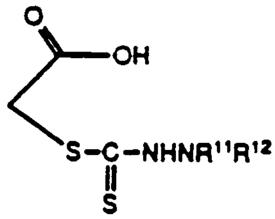

worin $R^1$, $R^2$, $R^3$, $R^{11}$ und $R^{12}$ wie oben definiert sind unter Bildung einer Verbindung der Formel

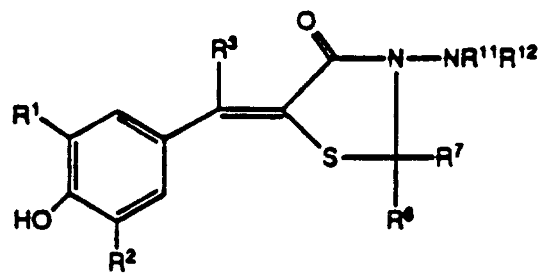

worin $R^6$ und $R^7$ zusammengenommen für =S stehen und $R^1$, $R^2$, $R^3$, $R^{11}$ und $R^{12}$ wie oben definiert sind,

(Q) Erhitzen einer Verbindung der Formel II, worin $R^8$ für $-(CH_2)_n$-Y steht und Y für $-NR^{11}R^{12}$ steht (wobei keines von $R^{11}$ oder $R^{12}$ ihr Wasserstoff steht) in einem Ethanol / Wasser Gemisch in Gegenwart eines Katalysators unter Bildung einer Verbindung der Formel II, worin $R^8$ für $-(CH_2)_n$-Y steht und Y für $-NR^{11}R^{12}$ steht (worin eines von $R^{11}$ oder $R^{12}$ für Wasserstoff und das andere nicht für Wasserstoff steht),

(R) Umsetzung einer Verbindung der Formel II, worin $R^6$ und $R^7$ beide für Wasserstoff stehen, mit Trifluoressigsäureanhydrid unter Bildung einer Verbindung der Formel II, worin eines von $R^6$ und $R^7$ ihr Wasserstoff steht und das andere für -OH steht,

(S) Salzbildung einer Verbindung der Formel II durch Umsetzung der nicht-Salzform der Verbindung mit entweder einer starken Säure oder einer starken Base.

**2.** Verfahren nach Anspruch 1 zur Herstellung einer Verbindung der Formel (II), worin

$R^1$ für $C_2$-$C_6$ Alkenyl, $C_2$-$C_6$ Alkinyl oder

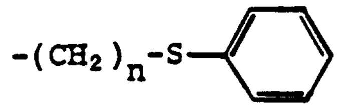

steht, worin n für eine ganze Zahl von 0 bis 3 steht, beide inbegriffen,

$R^2$ für Wasserstoff, $C_1$-$C_6$ Alkyl, $C_1$-$C_6$ Alkoxy, $C_2$-$C_6$ Alkenyl, $C_2$-$C_6$ Alkinyl, oder

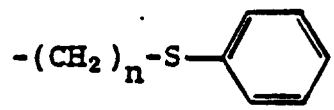

steht, worin n für eine ganze Zahl von 0 bis 3 steht, beide inbegriffen,

$R^3$ für Wasserstoff oder $C_1$-$C_6$ Alkyl steht,

$R^4$ und $R^5$ jeweils für Wasserstoff stehen oder zusammengenommen eine Bindung bilden.

$R^6$ und $R^7$ jeweils für Wasserstoff stehen oder zusammengenommen für =S stehen, oder wenn eines von $R^6$ und $R^7$ für Wasserstoff steht, das andere für -OH oder -$SCH_3$ steht,

X für

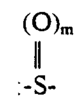

steht, worin m für 0, 1 oder 2 steht und

Q für -$CH_2$-, -O- oder $NR^8$ steht, worin $R^8$ für Wasserstoff, $C_1$-$C_6$ Alkyl, $C_3$-$C_8$ Cycloalkyl, -$SO_2CH_3$ oder -$(CH_2)_n$-Y steht, worin n für eine ganze Zahl von 0 bis 3 steht, beide inbegriffen, und

Y steht für Cyano, $OR^9$,

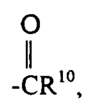

Tetrazolyl, -$NR^{11}R^{12}$ oder

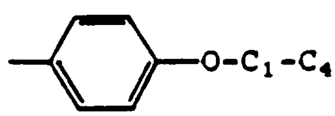 Alkyl

worin

$R^9$ für Wasserstoff, $C_1$-$C_4$ Alkyl oder

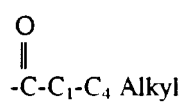

steht,

$R^{10}$ für $C_1$-$C_4$ Alkyl, $C_1$-$C_4$ Alkoxy oder -$NH_2$, steht,

$R^{11}$ und $R^{12}$ jeweils unabhängig voneinander stehen für Wasserstoff, $C_1$-$C_6$ Alkyl, -$(CH_2)_q$OH, -$(CH_2)_q$-N($C_1$-$C_4$ Alkyl)$_2$, -$(CH_2)_q$-S($C_1$-$C_4$ Alkyl) oder

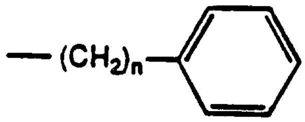

worin n wie oben definiert ist und q für eine ganze Zahl von 1 bis 6 steht, beide inbegriffen, oder

$R^{11}$ und $R^{12}$ zusammengenommen einen Morpholinyl-, Piperidinyl-, Piperazinyl- oder N-Methylpiperazinylring bilden,

oder eines pharmazeutisch annehmbaren Salzes hiervon.

3. Verfahren nach Anspruch 2 zur Herstellung einer Verbindung der Formel II, worin $R^1$ für $C_2$-$C_6$ Alkenyl steht, $R^2$ für $C_2$-$C_6$ Alkenyl oder $C_1$-$C_6$ Alkyl steht, $R^3$ für Wasserstoff steht, $R^4$ und $R^5$ für Wasserstoff stehen oder zusammengenommen eine Bindung bilden, $R^6$ und $R^7$ jeweils für Wasserstoff stehen oder zusammengenommen für =S stehen,

X für

$(O)_m$
||
-S-

steht, worin m für 0 steht und Q für -O- oder $NR^8$ steht, worin $R^8$ wie in Anspruch 2 definiert ist und pharmazeutisch annehmbarer Salze hiervon.

4. Verfahren nach Anspruch 3 zur Herstellung von 5-[[3,5-Di-2-propenyl-4-hydroxyphenyl]methyl]-4-thiazolidinon und pharmazeutisch annehmbarer Salze hiervon.

5. Verfahren zur selektiven Isolierung einer im wesentlichen reinen Enantiomerform eines der Enantiomere eines razemischen Gemisches einer Verbindung der Formel

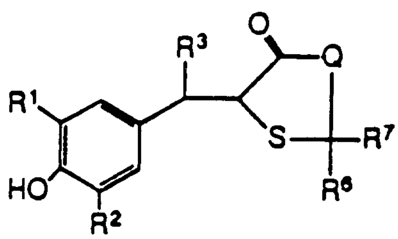

worin

$R^1$ und $R^2$ jeweils unabhängig für Wasserstoff $C_1$-$C_6$ Alkyl, $C_1$-$C_6$ Alkoxy, $C_2$-$C_6$ Alkenyl, $C_2$-$C_6$ Alkinyl,

O
||
$C_1$-$C_4$ Alkyl-O-C-($C_1$-$C_4$ Alkyl) oder 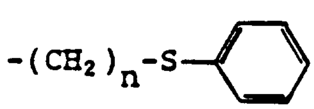 stehen,

worin n für eine ganze Zahl von 0 bis 3 steht, beide inbegriffen,

$R^3$ für Wasserstoff oder $C_1$-$C_6$ Alkyl steht,

$R^6$ und $R^7$ jeweils für Wasserstoff stehen oder zusammengenommen für =S stehen, oder wenn eines von $R^6$ und $R^7$ für Wasserstoff steht, das andere für -OH oder $-SCH_3$ steht,

Q für $-CH_2-$, -O- oder $NR^8$ steht, worin $R^8$ für Wasserstoff, $C_1$-$C_6$ Alkyl, $C_2$-$C_6$ Alkenyl, $C_3$-$C_8$ Cycloalkyl, $-SO_2CH_3$ oder $-(CH_2)_n$-Y steht, worin n für eine ganze Zahl von 0 bis 3 steht, beide inbegriffen, und

Y steht für Cyano, $OR^9$,

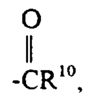

Tetrazolyl, $-NR^{11}R^{12}$, -SH, -S($C_1$-$C_4$ Alkyl) oder

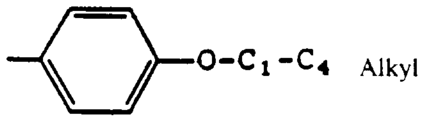

worin

$R^9$ für Wasserstoff, $C_1$-$C_4$ Alkyl oder

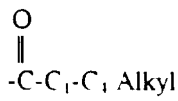

steht,

$R^{10}$ für $C_1$-$C_4$ Alkyl, $C_1$-$C_4$ Alkoxy oder $-NH_2$ steht,

$R^{11}$ und $R^{12}$ jeweils unabhängig voneinander stehen für Wasserstoff, $C_1$-$C_6$ Alkyl, $C_2$-$C_6$ Alkenyl, $C_2$-$C_6$ Alkinyl, $-(CH_2)_qOH$, $-(CH_2)_q$-N($C_1$-$C_4$ Alkyl)$_2$, $-(CH_2)_q$-S($C_1$-$C_4$ Alkyl) oder

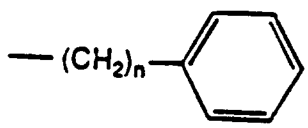

worin n wie oben definiert ist und q für eine ganze Zahl von 1 bis 6 steht, beide inbegriffen, oder

$R^{11}$ und $R^{12}$ zusammengenommen einen Morpholinyl-, Piperidinyl-, Piperazinyl- oder N-Methylpiperazinylring bilden,

gekennzeichnet durch

a) Umsetzung der razemischen Sulfidverbindung mit einem Reagenz, das durch die Kombination eines Tartratliganden, eines Titanalkoxids, eines Hydroperoxids und wahlweise Wasser hergestellt wurde, bis im wesentlichen das gesamte unerwünschte Enantiomer des Sulfidsubstrats in dessen Sulfoxidanalogon umgewandelt wurde, und

b) Abtrennung des nicht abreagierten Teils des Sulfidausgangsmaterials, das im wesentlichen aus reinem gewünschtem Enantiomer besteht, aus dem Reaktionsgemisch.

**6.** Verfahren nach Anspruch 5, das als Ausgangsmaterial eine razemische Sulfidverbindung verwendet, worin $R^1$ und $R^2$ jeweils für $C_1$-$C_6$ Alkyl, $C_2$-$C_6$ Alkenyl, $C_1$-$C_6$ Alkoxy oder

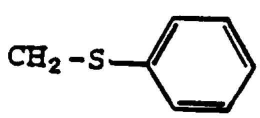

stehen,

$R^3$, $R^6$ und $R^7$ für Wasserstoff stehen und Q für $NR^8$ steht, worin $R^8$ für Wasserstoff, $C_1$-$C_6$ Alkyl oder $-(CH_2)_n$-Y steht, worin n für 0 steht und Y für $NR^{11}R^{12}$ steht (wobei $R^{11}$ und $R^{12}$ jeweils unabhängig für Wasserstoff oder $C_1$-$C_6$ Alkyl stehen).

**7.** Verfahren nach Anspruch 5 oder 6, das Ti(O-isopropyl)$_4$ als Titanoxid verwendet.

**8.** Verfahren nach einem der Ansprüche 5 bis 7, das Diisopropyltartrat als Tartratligand verwendet.

**9.** Verfahren nach einem der Ansprüche 5 bis 8, das t-Butylhydroperoxid als Hydroperoxid verwendet.

**10.** Verfahren zur Herstellung einer pharmazeutischen Formulierung, gekennzeichnet durch Mischen einer Verbindung der Formel

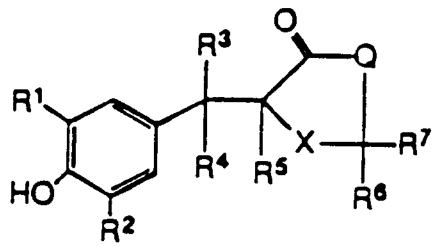

worin

$R^1$ für $C_2$-$C_6$ Alkenyl, $C_2$-$C_6$ Alkinyl oder

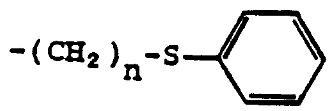

steht, worin n für eine ganze Zahl von 0 bis 3 steht, beide inbegriffen,

$R^2$ für Wasserstoff, $C_1$-$C_6$ Alkyl, $C_1$-$C_6$ Alkoxy, $C_2$-$C_6$ Alkenyl, $C_2$-$C_6$ Alkinyl,

$$C_1\text{-}C_4\ \text{Alkyl-O-}\overset{\overset{\displaystyle O}{\|}}{C}\text{-}(C_1\text{-}C_4\ \text{Alkyl})$$ oder 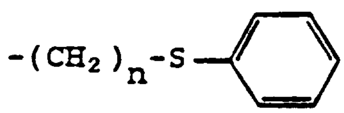

steht, worin n für eine ganze Zahl von 0 bis 3 steht, beide inbegriffen,

$R^3$ für Wasserstoff oder $C_1$-$C_6$ Alkyl steht,

$R^4$ und $R^5$ jeweils für Wasserstoff stehen oder zusammengenommen eine Bindung bilden,

$R^6$ und $R^7$ jeweils für Wasserstoff stehen oder zusammengenommen für =S stehen, oder wenn eines von $R^6$ und $R^7$ für Wasserstoff steht, das andere für -OH oder $-SCH_3$ steht,

X für

$$\overset{\overset{\displaystyle (O)_m}{\|}}{\text{-S-}}$$

steht, worin m für 0, 1 oder 2 steht und

Q für $-CH_2$-, -O- oder $NR^8$ stellt, worin $R^8$ für Wasserstoff, $C_1$-$C_6$ Alkyl, $C_2$-$C_6$ Alkenyl, $C_3$-$C_8$ Cycloalkyl, $-SO_2CH_3$ oder $-(CH_2)_n$-Y steht, worin n für eine ganze Zahl von 0 bis 3 steht, beide inbegriffen, und

Y steht für Cyano, $OR^9$,

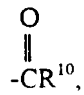

Tetrazolyl, $-NR^{11}R^{12}$, -SH, $-S(C_1-C_4$ Alkyl) oder

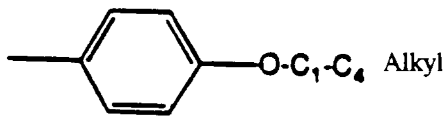

worin

$R^9$ für Wasserstoff, $C_1-C_4$ Alkyl oder

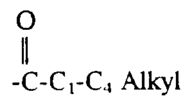

steht,

$R^{10}$ für $C_1-C_4$ Alkyl, $C_1-C_4$ Alkoxy oder $-NH_2$ steht,

$R^{11}$ und $R^{12}$ jeweils unabhängig voneinander stehen für Wasserstoff, $C_1-C_6$ Alkyl, $C_2-C_6$ Alkenyl, $C_2-C_6$ Alkinyl, $-(CH_2)_qOH$, $-(CH_2)_q-N(C_1-C_4\ Alkyl)_2$, $-(CH_2)_q-S(C_1-C_4$ Alkyl) oder

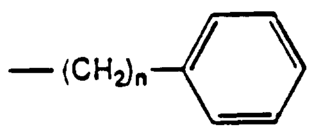

worin n wie oben definiert ist und q für eine ganze Zahl von 1 bis 6 steht, beide inbegriffen, oder

$R^{11}$ und $R^{12}$ zusammengenommen einen Morpholinyl-, Piperidinyl-, Piperazinyl- oder N-Methylpiperazinylring bilden,

oder eines pharmazeutisch annehmbaren Salzes hiervon, mit einem oder mehreren pharmazeutisch annehmbaren Trägern, Verdünnungsmitteln oder Hilfsstoffen hierfür.

## Revendications

**Revendications pour les Etats contractants suivants : AT, BE, CH, DK, FR, GB, IT, LI, LU, NL, SE**

**1.** Composé de formule (II)

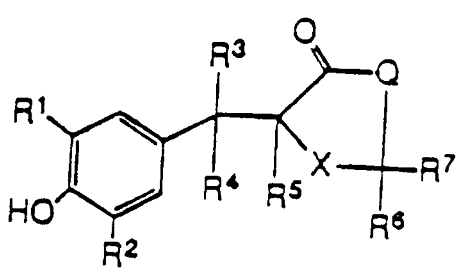

(II)

dans laquelle:

$R^1$ représente un groupe alcényle en $C_2$-$C_6$, un groupe alcynyle en $C_2$-$C_6$ ou un groupe

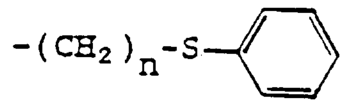

où n représente un entier de 0 à 3, ces deux entiers étant inclus;

$R^2$ représente un atome d'hydrogène, un groupe alkyle en $C_1$-$C_6$, un groupe alcoxy en $C_1$-$C_6$, un groupe alcényle en $C_2$-$C_6$, un groupe alcynyle en $C_2$-$C_6$, un groupe

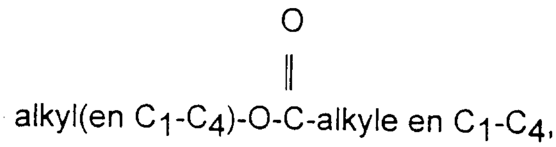

ou encore un groupe

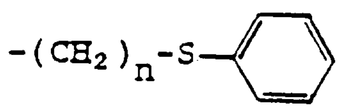

où

n représente un entier de 0 à 3, ces deux entiers étant inclus;

$R^3$ représente un atome d'hydrogène ou un groupe alkyle en $C_1$-$C_6$;

$R^4$ et $R^5$ représentent chacun un atome d'hydrogène ou, lorsqu'ils sont pris ensemble, forment une liaison;

$R^6$ et $R^7$ représentent chacun un atome d'hydrogène ou, lorsqu'ils sont pris ensemble, représentent un groupe =S, ou encore lorsqu'un des radicaux $R^6$ et $R^7$ représente un atome d'hydrogène, l'autre représente un groupe -OH ou un groupe $-SCH_3$;

X représente un groupe

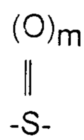

où m représente 0, 1 ou 2; et

Q représente un groupe $-CH_2-$, un groupe -O- ou un groupe $NR^8$ où $R^8$ représente un atome d'hydrogène, un groupe alkyle en $C_1$-$C_6$, un groupe alcényle en $C_2$-$C_6$, un groupe cycloalkyle en $C_3$-$C_8$, un groupe $-SO_2CH_3$ ou encore un groupe $-(CH_2)_n$-Y où n représente un entier de 0 à 3, ces deux entiers étant inclus, et Y représente un groupe cyano, un groupe $OR^9$, un groupe

O
‖
$-CR^{10}$,

un groupe tétrazolyle, un groupe $-NR^{11}R^{12}$, un groupe -SH, un groupe -S(alkyle en $C_1$-$C_4$) ou un groupe

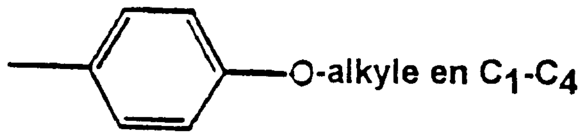

où $R^9$ représente un atome d'hydrogène, un groupe alkyle en $C_1$-$C_4$ ou un groupe

O
‖
-C-alkyle en $C_1$-$C_4$;

$R^{10}$ représente un groupe alkyle en $C_1$-$C_4$, un groupe alcoxy en $C_1$-$C_4$ ou un groupe $-NH_2$; $R^{11}$ et $R^{12}$ représentent chacun, indépendamment l'un de l'autre, un atome d'hydrogène, un groupe alkyle en $C_1$-$C_6$, un groupe alcényle en $C_2$-$C_6$, un groupe alcynyle en $C_2$-$C_6$ un groupe $-(CH_2)_qOH$, un groupe $-(CH_2)_q$-N(alkyle en $C_1$-$C_4$)$_2$, un groupe $-(CH_2)_q$-S-(alkyle en $C_1$-$C_4$) ou encore un groupe

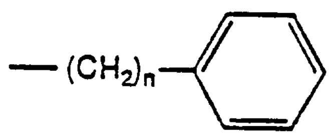

où n est tel que défini ci-dessus et q représente un entier de 1 à 6, ces deux entiers étant inclus; ou bien $R^{11}$ et $R^{12}$ pris ensemble forment un noyau morpholinyle, un noyau pipéridinyle, un noyau pipérazinyle ou un noyau N-méthylpipérazinyle; ou un de ses sels pharmaceutiquement acceptables.

**2.** Composé selon la revendication 1 dans lequel

$R^1$ représente un groupe alcényle en $C_2$-$C_6$, un groupe alcynyle en $C_2$-$C_6$ ou un groupe

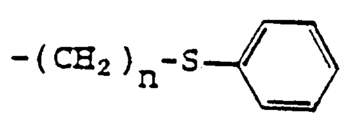

où n représente un entier de 0 à 3, ces deux entiers étant inclus;

$R^2$ représente un atome d'hydrogène, un groupe alkyle en $C_1$-$C_6$, un groupe alcoxy en $C_1$-$C_6$, un groupe alcényle en $C_2$-$C_6$, un groupe alcynyle en $C_2$-$C_6$ ou un groupe

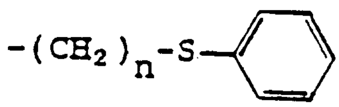

où

n représente un entier de 0 à 3, ces deux entiers étant inclus,

$R^3$ représente un atome d'hydrogène ou un groupe alkyle en $C_1$-$C_6$;

$R^4$ et $R^5$ représentent chacun un atome d'hydrogène ou, lorsqu'ils sont pris ensemble, forment une liaison;

$R^6$ et $R^7$ représentent chacun un atome d'hydrogène ou, lorsqu'ils sont pris ensemble, représentent un groupe =S, ou encore lorsqu'un des radicaux $R^6$ et $R^7$ représente un atome d'hydrogène, l'autre représente un groupe -OH ou un groupe $-SCH_3$;

X représente un groupe

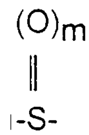

où m représente 0, 1 ou 2; et

Q représente un groupe $-CH_2-$, un groupe -O- ou un groupe $NR^8$ où $R^8$ représente un atome d'hydrogène, un groupe alkyle en $C_1$-$C_6$, un groupe cycloalkyle en $C_3$-$C_8$, un groupe $-SO_2CH_3$ ou encore un groupe $-(CH_2)_n$-Y où n représente un entier de 0 à 3, ces deux entiers étant inclus, et Y représente un groupe cyano, un groupe $OR^9$, un groupe

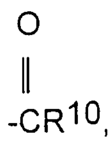

un groupe tétrazolyle, un groupe $-NR^{11}R^{12}$ ou un groupe

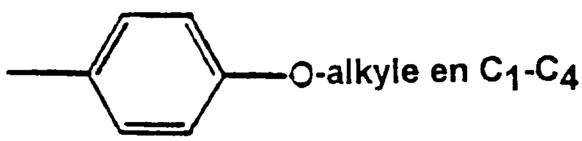

où $R^9$ représente un atome d'hydrogène, un groupe alkyle en $C_1$-$C_4$ ou un groupe

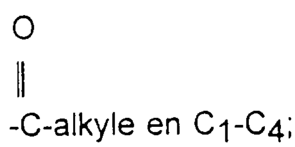

$R^{10}$ représente un groupe alkyle en $C_1$-$C_4$, un groupe alcoxy en $C_1$-$C_4$ ou un groupe $-NH_2$; $R^{11}$ et $R^{12}$ représentent chacun, indépendamment l'un de l'autre, un atome d'hydrogène, un groupe alkyle en $C_1$-$C_6$, un groupe $-(CH_2)_qOH$ un groupe $-(CH_2)_q$-N(alkyle en $C_1$-$C_4$)$_2$, un groupe $-(CH_2)_q$-S-(alkyle en $C_1$-$C_4$) ou encore un groupe

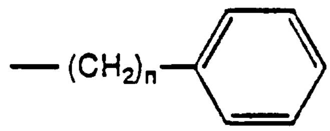

où n est tel que défini ci-dessus et q représente un entier de 1 à 6, ces deux entiers étant inclus; ou bien $R^{11}$ et $R^{12}$ pris ensemble forment un noyau morpholinyle, un noyau pipéridinyle, un noyau pipérazinyle ou un noyau N-méthylpipérazinyle; ou un de ses sels pharmaceutiquement acceptables.

3. Composé selon la revendication 2, dans lequel $R^1$ représente un groupe alcényle en $C_2$-$C_6$, $R^2$ représente un groupe alcényle en $C_2$-$C_6$ ou un groupe alkyle en $C_1$-$C_6$; $R^3$ représente un atome d'hydrogène; $R^4$ et $R^5$ représentent un atome d'hydrogène ou bien, lorsqu'ils sont pris ensemble, forment une liaison; $R^6$ et $R^7$ représentent chacun un atome d'hydrogène ou, lorsqu'ils sont pris ensemble, représentent un groupe =S; X représente un groupe

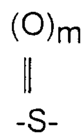

où m représente 0; et Q représente un groupe -O-ou un groupe $NR^8$ où $R^8$ est tel que défini à la revendication 2, ainsi que ses sels pharmaceutiquement acceptables.

4. Composition pharmaceutique comprenant, comme ingrédient actif, un composé selon l'une quelconque des revendications 1 à 3 ou encore un de ses sels pharmaceutiquement acceptables, en association avec un ou plusieurs diluants, excipients ou supports pharmaceutiquement acceptables pour l'ingrédient actif.

5. Composé selon l'une quelconque des revendications 1 à 3 à utiliser dans le traitement d'infections inflammatoires touchant le tube digestif.

6. Procédé pour préparer un composé de formule II selon l'une quelconque des revendications 1 à 3, qui comprend le fait de:

(A) faire réagir un composé de formule

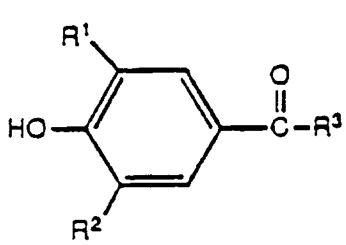

avec un composé de formule

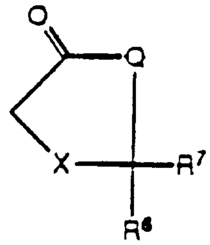

dans lesquelles $R^1$, $R^2$, $R^3$ et X sont tels que définis dans l'une quelconque des revendications 1 à 3, Q représente un groupe $-CH_2-$ ou un groupe $NR^8$ (où $R^8$ est tel que défini dans l'une quelconque des revendications 1 à 3), et $R^6$ et $R^7$ pris ensemble représentent un groupe =S, pour obtenir un composé de formule

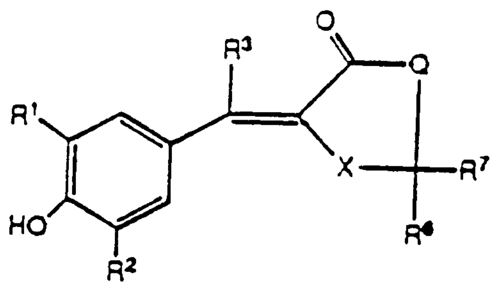

dans laquelle $R^1$, $R^2$, $R^3$, $R^6$, $R^7$, X et Q sont tels qu'indiqués ci-dessus;

(B) réduire un composé de formule II dans laquelle $R^6$ et $R^7$ pris ensemble représentent un groupe =S pour préparer un composé de formule II dans laquelle $R^6$ et $R^7$ représentent un atome d'hydrogène;

(C) réduire un composé de formule II dans laquelle $R^4$ et $R^5$ pris ensemble forment une liaison pour préparer un composé de formule II dans laquelle $R^4$ et $R^5$ représentent un atome d'hydrogène;

(D) réduire un composé de formule II dans laquelle $R^4$ et $R^5$ pris ensemble forment une liaison, et $R^6$ et $R^7$ pris ensemble représentent un groupe =S pour préparer un composé de formule II dans laquelle $R^4$, $R^5$, $R^6$ et $R^7$ représentent tous un atome d'hydrogène;

(E) alkyler un composé de formule II dans laquelle $R^8$ représente un atome d'hydrogène pour préparer un composé de formule II dans laquelle $R^8$ représente un groupe alkyle en $C_1$-$C_6$, un groupe alcényle en $C_2$-$C_6$, un groupe cycloalkyle en $C_3$-$C_8$ ou un groupe $-(CH_2)_n-Y$ (où n représente un entier de 0 à 3, ces deux entiers étant nous, et Y représente un groupe cyano, un groupe $OR^9$, un groupe -SH, un groupe -S(alkyle en $C_1$-$C_4$), un groupe $-NR^{11}R^{12}$ ou encore un groupe

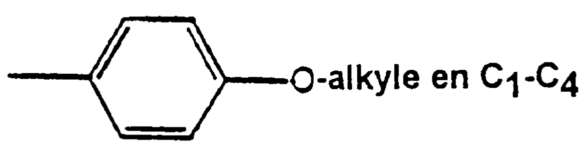

(où $R^9$, $R^{11}$ et $R^{12}$ sont tels que définis dans l'une quelconque des revendications 1 à 3);

(F) acyler un composé de formule II dans laquelle $R^8$ représente un atome d'hydrogène pour préparer un composé de formule II dans laquelle $R^8$ représente un groupe $-(CH_2)_n-Y$ où n représente un entier de 0 à 3, ces deux entiers étant nous, et Y représente un groupe

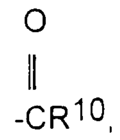

où $R^{10}$ est tel que défini dans l'une quelconque des revendications 1 à 3;

(G) oxyder un composé de formule II dans laquelle X représente un groupe

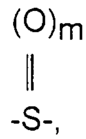

où m représente 0, pour préparer un composé de formule II dans laquelle X représente un groupe

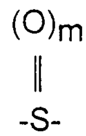

et m représente 1;

(H) oxyder un composé de formule II dans laquelle X représente un groupe

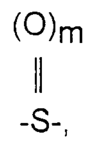

où m représente 0, pour préparer un composé de formule II dans laquelle X représente un groupe

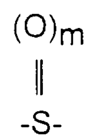

et m représente 2;

(I) oxyder un composé de formule II dans laquelle X représente un groupe

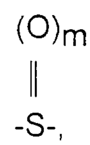

où m représente 1, pour préparer un composé de formule II dans laquelle X représente un groupe

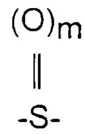

et m représente 2;

(J) faire réagir un composé de formule

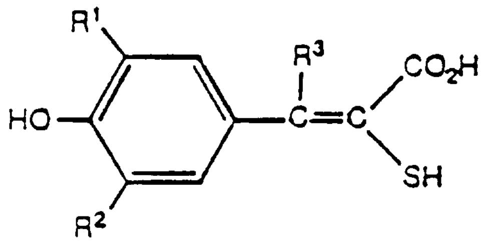

avec

i) de l'acide formique pour obtenir un composé de formule II dans laquelle Q représente un groupe O, $R^4$ et $R^5$ pris ensemble forment une liaison et $R^6$ et $R^7$ représentent un atome d'hydrogène; ou

ii) du disulfure de carbone pour obtenir un composé de formule II dans laquelle Q représente un groupe O, $R^3$ et $R^4$ pris ensemble forment une liaison et $R^6$ et $R^7$ pris ensemble représentent un groupe =S;

(K) faire réagir un composé de formule

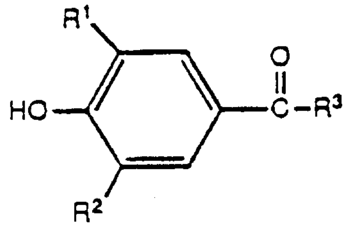

avec un composé de formule

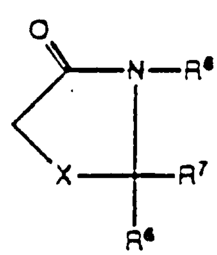

dans lesquelles $R^1$, $R^2$, $R^3$ et X sont tels que définis dans l'une quelconque des revendications 1 à 3, $R^6$ et $R^7$ pris ensemble représentent un groupe =S et $R^8$ représente un groupe $-(CH_2)_n-Y$ (où n représente un entier de 0 à 3, ces deux entiers étant inclus, et Y représente un groupe $OR^9$ où $R^9$ représente un atome d'hydrogène) pour obtenir un composé de formule

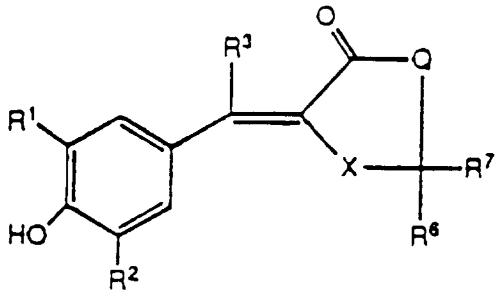

dans laquelle $R^1$, $R^2$, $R^3$, $R^6$, $R^7$ et X sont tels qu'indiqués ci-dessus et $R^8$ représente un groupe $-(CH_2)_n$-Y (où n représente un entier de 0 à 3, ces deux entiers étant inclus, et Y représente un groupe $OR^9$ où $R^9$ représente un groupe

$$\overset{\displaystyle O}{\overset{\|}{-C}}-CH_3);$$

(L) réduire un composé de formule II dans laquelle $R^8$ représente un groupe $-(CH_2)_n$-Y où n représente un entier de 0 à 3, ces deux entiers étant inclus, et Y représente un groupe $-OR^9$ où $R^9$ représente un groupe

$$\overset{\displaystyle O}{\overset{\|}{-C}}\text{-alkyle en } C_1\text{-}C_4,$$

pour préparer un composé de formule II dans laquelle $R^8$ représente un groupe $-(CH_2)_n$-Y où n représente un entier de 0 à 3, ces deux entiers étant inclus, et Y représente un groupe $OR^9$ où $R^9$ représente un atome d'hydrogène;

(M) faire réagir un composé de formule II dans laquelle $R^8$ représente un groupe $(CH_2)_n$-Y où n représente un entier de 0 à 3, ces deux entiers étant inclus, et Y représente un groupe $-OR^9$ où $R^9$ représente un atome d'hydrogène, avec un halogénure de tosyle pour préparer un composé de formule II dans laquelle $R^8$ représente un groupe $-(CH_2)_n$-Y où n représente un entier de 0 à 3, ces deux entiers étant inclus, et Y représente un groupe $OR^9$ où $R^9$ représente un groupe tosyle;

(N) faire réagir un composé de formule II dans laquelle $R^8$ représente un groupe $-(CH_2)_n$-Y où n représente un entier de 0 à 3, ces deux entiers étant inclus, et Y représente un groupe $-OR^9$ où $R^9$ représente un groupe tosyle, avec une amine de formule $HNR^{11}R^{12}$ (où $R^{11}$ et $R^{12}$ sont tels que définis dans l'une quelconque des revendications 1 à 3), pour préparer un composé de formule II dans laquelle $R^8$ représente un groupe $-(CH_2)_n$-Y où n représente un entier de 0 à 3, ces deux entiers étant nous, et Y représente un groupe $-NR^{11}R^{12}$;

(O) traiter un composé de formule II dans laquelle $R^8$ représente un groupe $-(CH_2)_n$-Y où n représente un entier de 0 à 3, ces deux entiers étant inclus, et Y représente un groupe cyano, avec de l'azide de tri-n-butylétain, pour préparer un composé de formule II dans laquelle $R^8$ représente un groupe $-(CH_2)_n$-Y où n représente un entier de 0 à 3, ces deux entiers étant inclus, et Y représente un groupe tétrazolyle;

(P) faire réagir un composé de formule

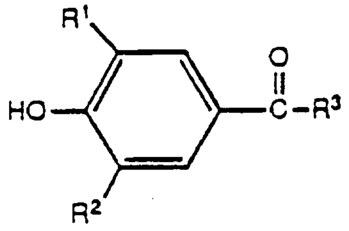

avec un composé de formule

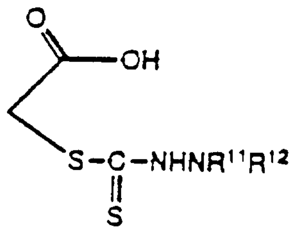

dans lesquelles $R^1$, $R^2$, $R^3$, $R^{11}$ et $R^{12}$ sont tels que définis dans l'une quelconque des revendications 1 à 3, pour obtenir un composé de formule

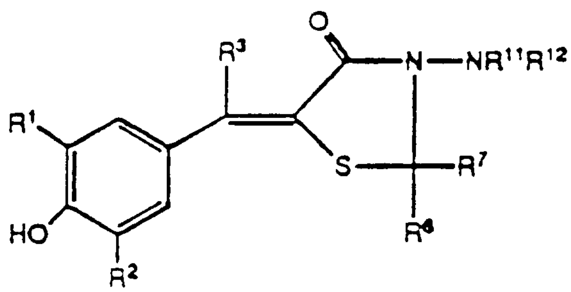

dans laquelle $R^6$ et $R^7$ pris ensemble représentent un groupe =S et $R^1$, $R^2$, $R^3$, $R^{11}$ et $R^{12}$ sont tels que définis dans l'une quelconque des revendications 1 à 3;

(Q) chauffer un composé de formule II dans laquelle $R^8$ représente un groupe $-(CH_2)_n-Y$ et Y représente un groupe $-NR^{11}R^{12}$ (aucun des radicaux $R^{11}$ ou $R^{12}$ ne représentant un atome d'hydrogène) dans un mélange éthanol/eau en présence d'un catalyseur pour préparer un composé de formule II dans laquelle $R^8$ représente un groupe $-(CH_2)_n-Y$ et Y représente un groupe $-NR^{11}R^{12}$ (où un des radicaux $R^{11}$ ou $R^{12}$ représente un atome d'hydrogène et l'autre ne représente pas un atome d'hydrogène);

(R) faire réagir un composé de formule II dans laquelle $R^6$ et $R^7$ représentent tous deux un atome d'hydrogène, avec de l'anhydride trifluoracétique, pour préparer un composé de formule II dans laquelle un des radicaux $R^6$ et $R^7$ représente un atome d'hydrogène et l'autre représente un groupe -OH;

(S) salifier un composé de formule II en faisant réagir la forme non saline du composé, soit avec un acide fort, soit avec une base forte.

7. Procédé pour isoler de manière sélective, sous une forme énantiomère essentiellement pure, un des énantiomères d'un mélange racémique d'un composé de formule

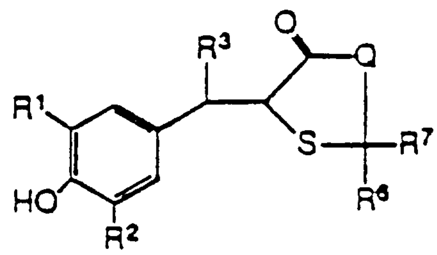

dans laquelle

$R^1$ et $R^2$ représentent chacun, indépendamment l'un de l'autre, un atome d'hydrogène, un groupe alkyle en $C_1$-$C_6$, un groupe alcoxy en $C_1$-$C_6$, un groupe alcényle en $C_2$-$C_6$, un groupe alcynyle en $C_2$-$C_6$, un groupe

$$\text{alkyl(en } C_1\text{-}C_4\text{)-O-}\overset{\overset{\displaystyle O}{\|}}{C}\text{-alkyle en } C_1\text{-}C_4$$

ou encore un groupe

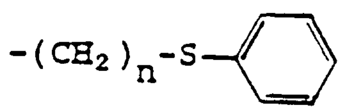

où n représente un entier de 0 à 3, ces deux entiers étant inclus;

$R^3$ représente un atome d'hydrogène ou un groupe alkyle en $C_1$-$C_6$;

$R^6$ et $R^7$ représentent chacun un atome d'hydrogène ou, lorsqu'ils sont pris ensemble, représentent un groupe =S, ou encore lorsqu'un des radicaux $R^6$ et $R^7$ représente un atome d'hydrogène, l'autre représente un groupe -OH ou un groupe $-SCH_3$;

Q représente un groupe $-CH_2-$, un groupe -O- ou un groupe $NR^8$ où $R^8$ représente un atome d'hydrogène, un groupe alkyle en $C_1$-$C_6$, un groupe alcényle en $C_2$-$C_6$, un groupe cycloalkyle en $C_3$-$C_8$, un groupe $-SO_2CH_3$ ou un groupe $-(CH_2)_n-Y$ où n représente un entier de 0 à 3, ces deux entiers étant inclus, et Y représente un groupe cyano, un groupe $OR^9$, un groupe

$$-\overset{\overset{\displaystyle O}{\|}}{C}R^{10},$$

un groupe tétrazolyle, un groupe $-NR^{11}R^{12}$, un groupe -SH, un groupe -S(alkyle en $C_1$-$C_4$) ou encore un groupe

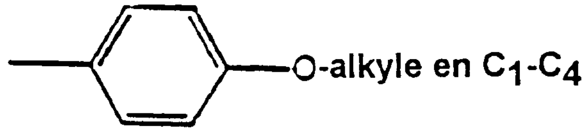

où $R^9$ représente un atome d'hydrogène, un groupe alkyle en $C_1$-$C_4$ ou un groupe

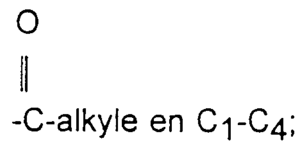

$R^{10}$ représente un groupe alkyle en $C_1$-$C_4$, un groupe alcoxy en $C_1$-$C_4$ ou un groupe $-NH_2$; $R^{11}$ et $R^{12}$ représentent chacun, indépendamment l'un de l'autre, un atome d'hydrogène, un groupe alkyle en $C_1$-$C_6$, un groupe alcényle en $C_2$-$C_6$, un groupe alcynyle en $C_2$-$C_6$, un groupe $-(CH_2)_qOH$, un groupe $-(CH_2)_q$-N(alkyle en $C_1$-$C_4$)$_2$, un groupe $-(CH_2)_q$-S-(alkyle en $C_1$-$C_4$) ou encore un groupe

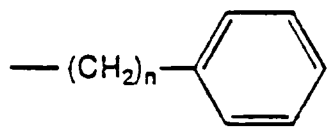

où n est tel que défini ci-dessus et q représente un entier de 1 à 6, ces deux entiers étant inclus; ou bien $R^{11}$ et $R^{12}$ pris ensemble forment un noyau morpholinyle, un noyau pipéridinyle, un noyau pipérazinyle ou un noyau N-méthylpipérazinyle, comprenant le fait de:

a) faire réagir le composé de sulfure racémique avec un réactif préparé à partir de la combinaison d'un ligand de tartrate, d'un alcoxyde de titane, d'un hydroperoxyde et, le cas échéant, d'eau jusqu'à ce qu'essentiellement la totalité de l'énantiomère non désiré du substrat de sulfure ait été transformée en son analogue sulfoxyde; et
b) séparer du mélange réactionnel la portion n'ayant pas réagi de la matière de départ de sulfure constituée essentiellement de l'énantiomère désiré essentiellement pur.

**8.** Utilisation d'un composé de formule

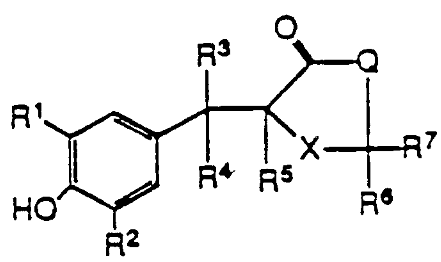

dans laquelle

$R^1$ et $R^2$ représentent chacun, indépendamment l'un de l'autre, un atome d'hydrogène, un groupe alkyle en $C_1$-$C_6$, un groupe alcoxy en $C_1$-$C_6$, un groupe alcényle en $C_2$-$C_6$, un groupe alcynyle en $C_2$-$C_6$, un groupe

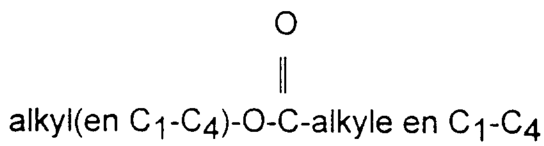

ou encore un groupe

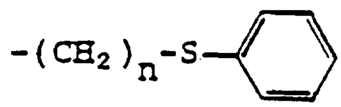

où n représente un entier de 0 à 3, ces deux entiers étant inclus;

$R^3$ représente un atome d'hydrogène ou un groupe alkyle en $C_1$-$C_6$;

$R^4$ et $R^5$ représentent chacun un atome d'hydrogène ou, lorsqu'ils sont pris ensemble, forment une liaison;

$R^6$ et $R^7$ représentent chacun un atome d'hydrogène ou, lorsqu'ils sont pris ensemble, représentent un groupe =S, ou encore lorsqu'un des radicaux $R^6$ et $R^7$ représente un atome d'hydrogène, l'autre représente' un groupe -OH ou un groupe $-SCH_3$;

X représente un groupe

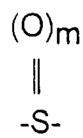

où m représente 0, 1 ou 2; et

Q représente un groupe $-CH_2$-, un groupe -O- ou $NR^8$ où $R^8$ représente un atome d'hydrogène, un groupe alkyle en $C_1$-$C_6$, un groupe alcényle en $C_2$-$C_6$, un groupe cycloalkyle en $C_3$-$C_8$, un groupe -$SO_2CH_3$ ou un groupe $-(CH_2)_n$-Y où n représente un entier de 0 à 3, ces deux entiers étant inclus, et Y représente un groupe cyano, un groupe $OR^9$, un groupe

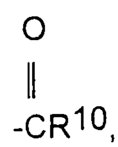

un groupe tétrazolyle, un groupe $-NR^{11}R^{12}$, un groupe -SH, un groupe -S(alkyle en $C_1$-$C_4$) ou encore un groupe

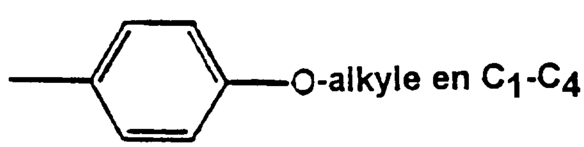

où $R^9$ représente un atome d'hydrogène, un groupe alkyle en $C_1$-$C_4$ ou un groupe

O
||
-C-alkyle en $C_1$-$C_4$;

$R^{10}$ représente un groupe alkyle en $C_1$-$C_4$, un groupe alcoxy en $C_1$-$C_4$ ou un groupe $-NH_2$; $R^{11}$ et $R^{12}$ représentent chacun, indépendamment l'un de l'autre, un atome d'hydrogène, un groupe alkyle en $C_1$-$C_6$, un groupe alcényle en $C_2$-$C_6$, un groupe alcynyle en $C_2$-$C_6$, un groupe $-(CH_2)_qOH$, un groupe $-(CH_2)_q$-N(alkyle en $C_1$-$C_4$)$_2$, un groupe $-(CH_2)_q$-S-(alkyle en $C_1$-$C_4$) ou encore un groupe

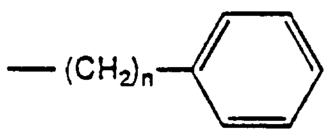

où n est tel que défini ci-dessus et q représente un entier de 1 à 6, ces deux entiers étant nous; ou bien $R^{11}$ et $R^{12}$ pris ensemble forment un noyau morpholinyle, un noyau pipéridinyle, un noyau pipérazinyle ou un noyau N-méthylpipérazinyle; ou encore un de ses sels pharmaceutiquement acceptables, pour préparer un médicament actif contre des infections inflammatoires touchant le tube digestif.

**9.** Utilisation d'un composé selon la revendication 8, dans lequel

$R^1$ et $R^2$ représentent chacun, indépendamment l'un de l'autre, un atome d'hydrogène, un groupe alkyle en $C_1$-$C_6$, un groupe alcoxy en $C_1$-$C_6$, un groupe alcényle en $C_2$-$C_6$, un groupe alcynyle en $C_2$-$C_6$ ou un groupe

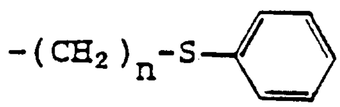

où n représente un entier de 0 à 3, ces deux entiers étant inclus;

$R^3$ représente un atome d'hydrogène ou un groupe alkyle en $C_1$-$C_6$;

$R^4$ et $R^5$ représentent chacun un atome d'hydrogène ou, lorsqu'ils sont pris ensemble, forment une liaison;

$R^6$ et $R^7$ représentent chacun un atome d'hydrogène ou, lorsqu'ils sont pris ensemble, représentent un groupe =S, ou encore lorsqu'un des radicaux $R^6$ et $R^7$ représente un atome d'hydrogène, l'autre représente un groupe -OH ou un groupe $-SCH_3$;

X représente un groupe

$(O)_m$
||
-S-

où m représente 0, 1 ou 2; et

Q représente un groupe $-CH_2-$, un groupe -O- ou un groupe $NR^8$ où $R^8$ représente un atome d'hydrogène, un groupe alkyle en $C_1$-$C_6$, un groupe cycloalkyle en $C_3$-$C_8$, un groupe $-SO_2CH_3$ ou un groupe $-(CH_2)_n$-Y où n représente un entier de 0 à 3, ces deux entiers étant inclus, et Y représente un groupe cyano, un groupe $OR^9$, un groupe

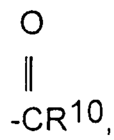

un groupe tétrazolyle, un groupe $-NR^{11}R^{12}$, ou encore un groupe

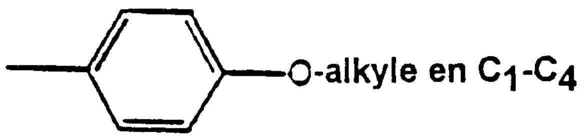

où $R^9$ représente un atome d'hydrogène, un groupe alkyle en $C_1$-$C_4$ ou un groupe

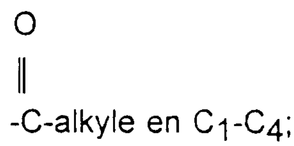

$R^{10}$ représente un groupe alkyle en $C_1$-$C_4$, un groupe alcoxy en $C_1$-$C_4$ ou un groupe $-NH_2$; $R^{11}$ et $R^{12}$ représentent chacun, indépendamment l'un de l'autre, un atome d'hydrogène, un groupe alkyle en $C_1$-$C_6$, un groupe $-(CH_2)_qOH$ un groupe $-(CH_2)_q$-N(alkyle en $C_1$-$C_4$)$_2$, un groupe $-(CH_2)_q$-S-(alkyle en $C_1$-$C_4$) ou encore un groupe

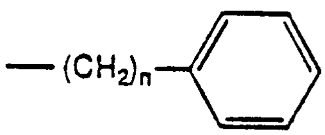

où n est tel que défini ci-dessus et q représente un entier de 1 à 6, ces deux entiers étant inclus; ou bien $R^{11}$ et $R^{12}$ pris ensemble forment un noyau morpholinyle, un noyau pipéridinyle, un noyau pipérazinyle ou un noyau N-méthylpipérazinyle; ou encore d'un de ses sels pharmaceutiquement acceptables, pour préparer un médicament actif contre des infections inflammatoires touchant le tube digestif.

**10.** Utilisation de la 5-[[3,5-bis(1,1,-diméthyléthyl)-4-hydroxyphényl]méthyl]-4-thiazolidinone pour préparer un médicament actif contre des infections inflammatoires touchant le tube digestif.

**Revendications pour les Etats contractants suivants : ES, GR**

**1.** Procédé pour préparer un composé de formule (II)

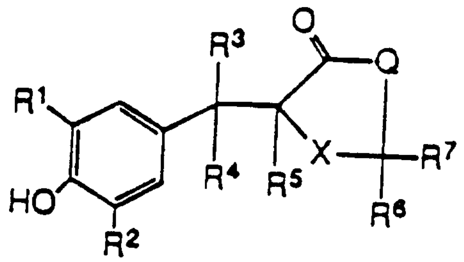 (II)

dans laquelle:

$R^1$ représente un groupe alcényle en $C_2$-$C_6$, un groupe alcynyle en $C_2$-$C_6$ ou un groupe

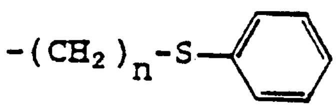

où n représente un entier de 0 à 3, ces deux entiers étant inclus;

$R^2$ représente un atome d'hydrogène, un groupe alkyle en $C_1$-$C_6$, un groupe alcoxy en $C_1$-$C_6$, un groupe alcényle en $C_2$-$C_6$, un groupe alcynyle en $C_2$-$C_6$, un groupe

$$\text{alkyl(en } C_1\text{-}C_4\text{)-O-}\overset{\overset{O}{\|}}{C}\text{-alkyle en } C_1\text{-}C_4,$$

ou encore un groupe

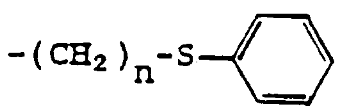

où

n représente un entier de 0 à 3, ces deux entiers étant inclus

$R^3$ représente un atome d'hydrogène ou un groupe alkyle en $C_1$-$C_6$;

$R^4$ et $R^5$ représentent chacun un atome d'hydrogène ou, lorsqu'ils sont pris ensemble, forment une liaison;

$R^6$ et $R^7$ représentent chacun un atome d'hydrogène ou, lorsqu'ils sont pris ensemble, représentent un groupe =S, ou encore lorsqu'un des radicaux $R^6$ et $R^7$ représente un atome d'hydrogène, l'autre représente un groupe -OH ou un groupe -$SCH_3$;

X représente un groupe

$(O)_m$
‖
-S-

où m représente 0, 1 ou 2; et

Q représente un groupe $-CH_2-$, un groupe -O- ou un groupe $NR^8$ où $R^8$ représente un atome d'hydrogène, un groupe alkyle en $C_1$-$C_6$, un groupe alcényle en $C_2$-$C_6$, un groupe cycloalkyle en $C_3$-$C_8$, un groupe $-SO_2CH_3$ ou encore un groupe $-(CH_2)_n$-Y où n représente un entier de 0 à 3, ces deux entiers étant inclus, et Y représente un groupe cyano, un groupe $OR^9$, un groupe

O
‖
$-CR^{10}$,

un groupe tétrazolyle, un groupe $-NR^{11}R^{12}$, un groupe -SH, un groupe -S(alkyle en $C_1$-$C_4$) ou un groupe

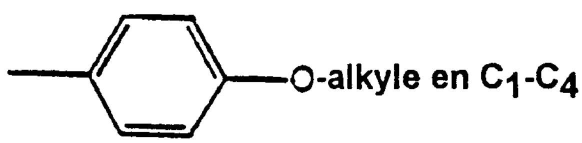

où $R^9$ représente un atome d'hydrogène, un groupe alkyle en $C_1$-$C_4$ ou un groupe

O
‖
-C-alkyle en $C_1$-$C_4$;

$R^{10}$ représente un groupe alkyle en $C_1$-$C_4$, un groupe alcoxy en $C_1$-$C_4$ ou un groupe $-NH_2$; $R^{11}$ et $R^{12}$ représentent chacun, indépendamment l'un de l'autre, un atome d'hydrogène, un groupe alkyle en $C_1$-$C_6$, un groupe alcényle en $C_2$-$C_6$, un groupe alcynyle en $C_2$-$C_6$, un groupe $-(CH_2)_qOH$, un groupe $-(CH_2)_q$-N(alkyle en $C_1$-$C_4$)$_2$, un groupe $-(CH_2)_q$-S-(alkyle en $C_1$-$C_4$) ou encore un groupe

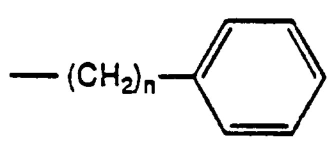

où n est tel que défini ci-dessus et q représente un entier de 1 à 6, ces deux entiers étant inclus; ou bien $R^{11}$ et $R^{12}$ pris ensemble forment un noyau morpholinyle, un noyau pipéridinyle, un noyau pipérazinyle ou un noyau N-méthylpipérazinyle; ou un de ses sels pharmaceutiquement acceptables, qui comprend le fait de:

(A) faire réagir un composé de formule

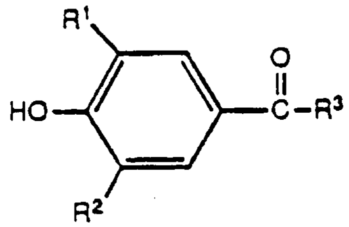

avec un composé de formule

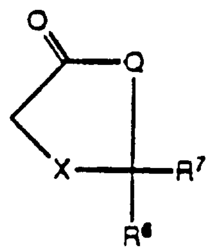

dans lesquelles $R^1$, $R^2$, $R^3$ et X sont tels que définis ci-dessus, Q représente un groupe $-CH_2-$ ou un groupe $NR^8$ (où $R^8$ est tel que défini ci-dessus), et $R^6$ et $R^7$ pris ensemble représentent un groupe =S, pour obtenir un composé de formule

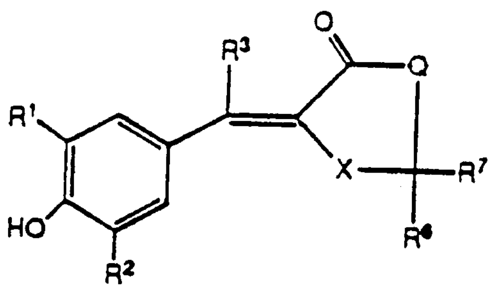

dans laquelle $R^1$, $R^2$, $R^3$, $R^6$, $R^7$, X et Q sont tels qu'indiqués ci-dessus;

(B) réduire un composé de formule II dans laquelle $R^6$ et $R^7$ pris ensemble représentent un groupe =S pour préparer un composé de formule II dans laquelle $R^6$ et $R^7$ représentent un atome d'hydrogène;

(C) réduire un composé de formule II dans laquelle $R^4$ et $R^5$ pris ensemble forment une liaison pour préparer un composé de formule II dans laquelle $R^4$ et $R^5$ représentent un atome d'hydrogène;

(D) réduire un composé de formule II dans laquelle $R^4$ et $R^5$ pris ensemble forment une liaison, et $R^6$ et $R^7$ pris ensemble représentent un groupe =S pour préparer un composé de formule II dans laquelle $R^4$, $R^5$, $R^6$ et $R^7$ représentent tous un atome d'hydrogène;

(E) alkyler un composé de formule II dans laquelle $R^8$ représente un atome d'hydrogène pour préparer un composé de formule II dans laquelle $R^8$ représente un groupe alkyle en $C_1$-$C_6$, un groupe alcényle en $C_2$-$C_6$, un groupe cycloalkyle en $C_3$-$C_8$ ou un groupe $-(CH_2)_n-Y$ (où n représente un entier de 0 à 3, ces deux entiers étant inclus, et Y représente un groupe cyano, un groupe $OR^9$, un groupe -SH, un groupe -S(alkyle en $C_1$-$C_4$), un groupe $-NR^{11}R^{12}$ ou encore un groupe

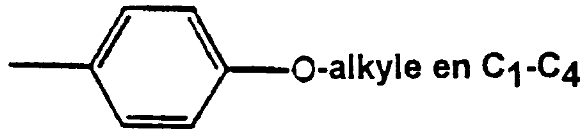

(où $R^9$, $R^{11}$ et $R^{12}$ sont tels que définis ci-dessus);

(F) acyler un composé de formule II dans laquelle $R^8$ représente un atome d'hydrogène pour préparer un composé de formule II dans laquelle $R^8$ représente un groupe $-(CH_2)_n-Y$ où n représente un entier de 0 à 3, ces deux entiers étant inclus, et Y représente un groupe

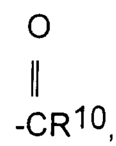

où $R^{10}$ est tel que défini ci-dessus;

(G) oxyder un composé de formule II dans laquelle X représente un groupe

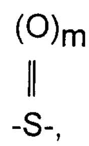

où m représente 0, pour préparer un composé de formule II dans laquelle X représente un groupe

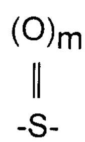

et m représente 1;

(H) oxyder un composé de formule II dans laquelle X représente un groupe

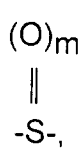

où m représente 0, pour préparer un composé de formule II dans laquelle X représente un groupe

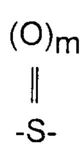

et m représente 2;

(I) oxyder un composé de formule II dans laquelle X représente un groupe

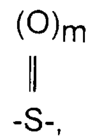

où m représente 1, pour préparer un composé de formule II dans laquelle X représente un groupe

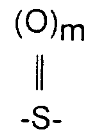

et m représente 2;

(J) faire réagir un composé de formule

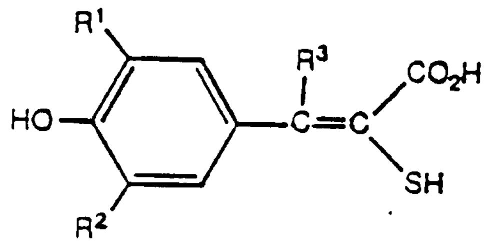

avec

i) de l'acide formique pour obtenir un composé de formule II dans laquelle Q représente un groupe O, $R^4$ et $R^5$ pris ensemble forment une liaison et $R^6$ et $R^7$ représentent un atome d'hydrogène; ou

ii) du disulfure de carbone pour obtenir un composé de formule II dans laquelle Q représente un groupe O, $R^3$ et $R^4$ pris ensemble forment une liaison et $R^6$ et $R^7$ pris ensemble représentent un groupe =S;

(K) faire réagir un composé de formule

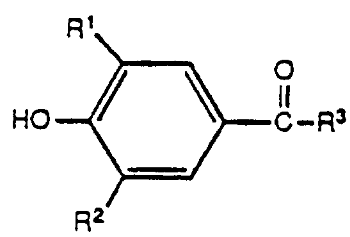

avec un composé de formule

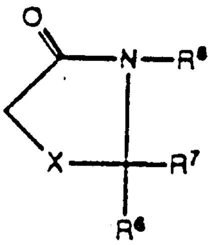

dans lesquelles $R^1$, $R^2$, $R^3$ et X sont tels que définis ci-dessus, $R^6$ et $R^7$ pris ensemble représentent un groupe =S et $R^8$ représente un groupe $-(CH_2)_n-Y$ (où n représente un entier de 0 à 3, ces deux entiers étant inclus, et Y représente un groupe $OR^9$ où $R^9$ représente un atome d'hydrogène) pour obtenir un composé de formule

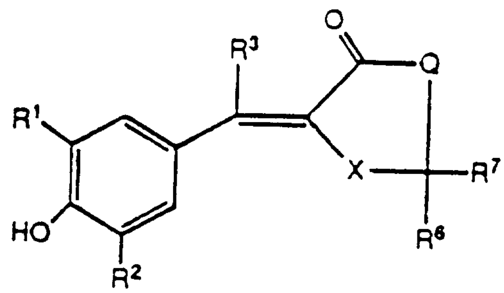

dans laquelle $R^1$, $R^2$, $R^3$, $R^6$, $R^7$ et X sont tels qu'indiqués ci-dessus et $R^8$ représente un groupe $-(CH_2)_n-Y$ (où n représente un entier de 0 à 3, ces deux entiers étant inclus, et Y représente un groupe $OR^9$ où $R^9$ représente un groupe

O
||
$-C-CH_3$);

(L) réduire un composé de formule II dans laquelle $R^8$ représente un groupe $-(CH_2)_n-Y$ où n représente un entier de 0 à 3, ces deux entiers étant inclus, et Y représente un groupe $-OR^9$ où $R^9$ représente un groupe

O
||
-C-alkyle en $C_1$-$C_4$,

pour préparer un composé de formule II dans laquelle $R^8$ représente un groupe $-(CH_2)_n-Y$ où n représente un entier de 0 à 3, ces deux entiers étant nous, et Y représente un groupe $OR^9$ où $R^9$ représente un atome d'hydrogène;

(M) faire réagir un composé de formule II dans laquelle $R^8$ représente un groupe $-(CH_2)_n-Y$ où n représente un entier de 0 à 3, ces deux entiers étant inclus, et Y représente un groupe $-OR^9$ où $R^9$ représente un atome d'hydrogène, avec un halogénure de tosyle pour préparer un composé de formule II dans laquelle $R^8$ représente un groupe $-(CH_2)_n-Y$ où n représente un entier de 0 à

3, ces deux entiers étant inclus, et Y représente un groupe $OR^9$ où $R^9$ représente un groupe tosyle;

(N) faire réagir un composé de formule II dans laquelle $R^8$ représente un groupe $-(CH_2)_n-Y$ où n représente un entier de 0 à 3, ces deux entiers étant inclus, et Y représente un groupe $-OR^9$ où $R^9$ représente un groupe tosyle, avec une amine de formule $HNR^{11}R^{12}$ (où $R^{11}$ et $R^{12}$ sont tels que définis ci-dessus), pour préparer un composé de formule II dans laquelle $R^8$ représente un groupe $-(CH_2)_n-Y$ où n représente un entier de 0 à 3, ces deux entiers étant inclus, et Y représente un groupe $-NR^{11}R^{12}$;

(O) traiter un composé de formule II dans laquelle $R^8$ représente un groupe $-(CH_2)_n-Y$ où n représente un entier de 0 à 3, ces deux entiers étant inclus, et Y représente un groupe cyano, avec de l'azide de tri-n-butylétain, pour préparer un composé de formule II dans laquelle $R^8$ représente un groupe $-(CH_2)_n-Y$ où n représente un entier de 0 à 3, ces deux entiers étant inclus, et Y représente un groupe tétrazolyle;

(P) faire réagir un composé de formule

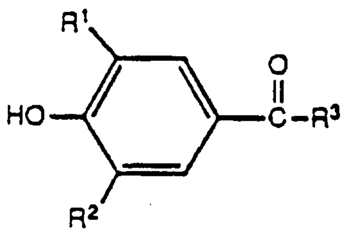

avec un composé de formule

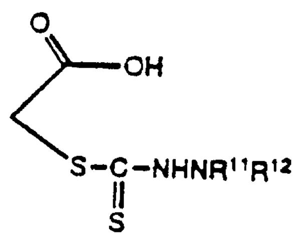

dans lesquelles $R^1$, $R^2$, $R^3$, $R^{11}$ et $R^{12}$ sont tels que définis ci-dessus, pour obtenir un composé de formule

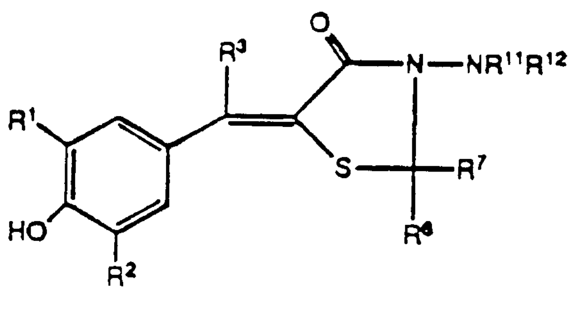

dans laquelle $R^6$ et $R^7$ pris ensemble représentent un groupe =S et $R^1$, $R^2$, $R^3$, $R^{11}$, et $R^{12}$ sont tels que définis ci-dessus;

(Q) chauffer un composé de formule II dans laquelle $R^8$ représente un groupe $-(CH_2)_n-Y$ et Y représente un groupe $-NR^{11}R^{12}$ (aucun des radicaux $R^{11}$ ou $R^{12}$ ne représentant un atome d'hydrogène) dans un mélange éthanol/eau en présence d'un catalyseur pour préparer un composé de formule II dans laquelle $R^8$ représente un groupe $-(CH_2)_n-Y$ et Y représente un groupe -

$NR^{11}R^{12}$ (où un des radicaux $R^{11}$ ou $R^{12}$ représente un atome d'hydrogène et l'autre ne représente pas un atome d'hydrogène);

(R) faire réagir un composé de formule II dans laquelle $R^6$ et $R^7$ représentent tous deux un atome d'hydrogène, avec de l'anhydride trifluoracétique, pour préparer un composé de formule II dans laquelle un des radicaux $R^6$ et $R^7$ représente un atome d'hydrogène et l'autre représente un groupe -OH;

(S) salifier un composé de formule II en faisant réagir la forme non saline du composé, soit avec un acide fort, soit avec une base forte.

**2.** Procédé selon la' revendication 1 pour préparer un composé de formule (II) dans laquelle

$R^1$ représente un groupe alcényle en $C_2$-$C_6$, un groupe alcynyle en $C_2$-$C_6$ ou un groupe

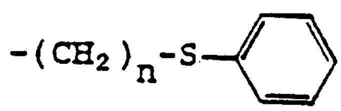

où n représente un entier de 0 à 3, ces deux entiers étant inclus;

$R^2$ représente un atome d'hydrogène, un groupe alkyle en $C_1$-$C_6$, un groupe alcoxy en $C_1$-$C_6$, un groupe alcényle en $C_2$-$C_6$, un groupe alcynyle en $C_2$-$C_6$ ou un groupe

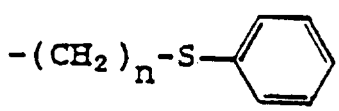

où

n représente un entier de 0 à 3, ces deux entiers étant inclus;

$R^3$ représente un atome d'hydrogène ou un groupe alkyle en $C_1$-$C_6$;

$R^4$ et $R^5$ représentent chacun un atome d'hydrogène ou, lorsqu'ils sont pris ensemble, forment une liaison;

$R^6$ et $R^7$ représentent chacun un atome d'hydrogène ou, lorsqu'ils sont pris ensemble, représentent un groupe =S, ou encore lorsqu'un des radicaux $R^6$ et $R^7$ représente un atome d'hydrogène, l'autre représente un groupe -OH ou un groupe $-SCH_3$;

X représente un groupe

(O)m
‖
-S-

où m représente 0, 1 ou 2; et

Q représente un groupe $-CH_2-$, un groupe -O- ou un groupe $NR^8$ où $R^8$ représente un atome d'hydrogène, un groupe alkyle en $C_1$-$C_6$, un groupe cycloalkyle en $C_3$-$C_8$, un groupe $-SO_2CH_3$ ou encore un groupe $-(CH_2)_n$-Y où n représente un entier de 0 à 3, ces deux entiers étant inclus, et Y représente un groupe cyano, un groupe $OR^9$, un groupe

$$-\overset{O}{\overset{\|}{C}}R^{10},$$

un groupe tétrazolyle, un groupe $-NR^{11}R^{12}$ ou un groupe

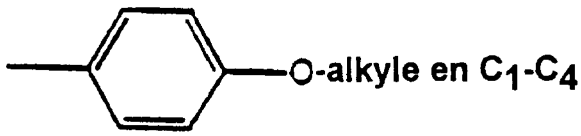

où $R^9$ représente un atome d'hydrogène, un groupe alkyle en $C_1$-$C_4$ ou un groupe

$$-\overset{O}{\overset{\|}{C}}\text{-alkyle en } C_1\text{-}C_4;$$

$R^{10}$ représente un groupe alkyle en $C_1$-$C_4$, un groupe alcoxy en $C_1$-$C_4$ ou un groupe $-NH_2$; $R^{11}$ et $R^{12}$ représentent chacun, indépendamment l'un de l'autre, un atome d'hydrogène, un groupe alkyle en $C_1$-$C_6$, un groupe $-(CH_2)_qOH$, un groupe $-(CH_2)_q$-N(alkyle en $C_1$-$C_4$)$_2$, un groupe $-(CH_2)_q$-S-(alkyle en $C_1$-$C_4$) ou encore un groupe

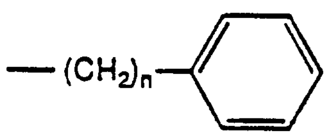

où n est tel que défini ci-dessus et q représente un entier de 1 à 6, ces deux entiers étant inclus; ou bien $R^{11}$ et $R^{12}$ pris ensemble forment un noyau morpholinyle, un noyau pipéridinyle, un noyau pipérazinyle ou un noyau N-méthylpipérazinyle; ou un de ses sels pharmaceutiquement acceptables.

**3.** Procédé selon la revendication 2 pour préparer un composé de formule (II), dans lequel $R^1$ représente un groupe alcényle en $C_2$-$C_6$, $R^2$ représente un groupe alcényle en $C_2$-$C_6$ ou un groupe alkyle en $C_1$-$C_6$; $R^3$ représente un atome d'hydrogène; $R^4$ et $R^5$ représentent un atome d'hydrogène ou bien, lorsqu'ils sont pris ensemble, forment une liaison; $R^6$ et $R^7$ représentent chacun un atome d'hydrogène ou, lorsqu'ils sont pris ensemble, représentent un groupe =S;
X représente un groupe

$$\overset{(O)_m}{\overset{\|}{-S-}}$$

où m représente 0; et Q représente un groupe -O-ou un groupe $NR^8$ où $R^8$ est tel que défini à la revendication 2, ainsi que ses sels pharmaceutiquement acceptables.

**4.** Procédé selon la revendication 3 pour préparer la 5-[[3,5-di-2-propényl-4-hydroxyphényl]méthyl]-4-thiazolidinone et ses sels pharmaceutiquement acceptables.

**5.** Procédé pour isoler de manière sélective, sous une forme énantiomère essentiellement pure, un des énantiomères d'un mélange racémique d'un composé de formule

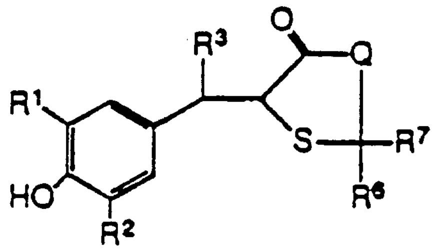

dans laquelle

$R^1$ et $R^2$ représentent chacun, indépendamment l'un de l'autre, un atome d'hydrogène, un groupe alkyle en $C_1$-$C_6$, un groupe alcoxy en $C_1$-$C_6$, un groupe alcényle en $C_2$-$C_6$, un groupe alcynyle en $C_2$-$C_6$, un groupe

$$\text{alkyl(en } C_1\text{-}C_4\text{)-O-}\overset{\overset{\displaystyle O}{\|}}{C}\text{-alkyle en } C_1\text{-}C_4$$

ou encore un groupe

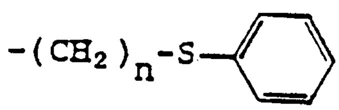

où n représente un entier de 0 à 3, ces deux entiers étant inclus;

$R^3$ représente un atome d'hydrogène ou un groupe alkyle en $C_1$-$C_6$;

$R^6$ et $R^7$ représentent chacun un atome d'hydrogène ou, lorsqu'ils sont pris ensemble, représentent un groupe =S, ou encore lorsqu'un des radicaux $R^6$ et $R^7$ représente un atome d'hydrogène, l'autre représente un groupe -OH ou un groupe $-SCH_3$;

Q représente un groupe $-CH_2-$, un groupe -O- ou un groupe $NR^8$ où $R^8$ représente un atome d'hydrogène, un groupe alkyle en $C_1$-$C_6$, un groupe alcényle en $C_2$-$C_6$, un groupe cycloalkyle en $C_3$-$C_8$, un groupe $-SO_2CH_3$ ou un groupe $-(CH_2)_n-Y$ où n représente un entier de 0 à 3, ces deux entiers étant inclus, et Y représente un groupe cyano, un groupe $OR^9$, un groupe

$$-\overset{\overset{\displaystyle O}{\|}}{C}R^{10},$$

un groupe tétrazolyle, un groupe $-NR^{11}R^{12}$, un groupe -SH, un groupe -S(alkyle en $C_1$-$C_4$) ou encore un groupe

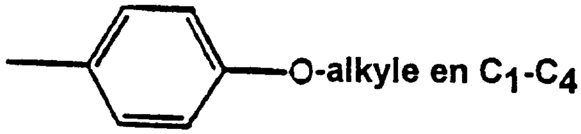

où $R^9$ représente un atome d'hydrogène, un groupe alkyle en $C_1$-$C_4$ ou un groupe

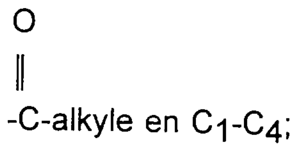

$R^{10}$ représente un groupe alkyle en $C_1$-$C_4$, un groupe alcoxy en $C_1$-$C_4$ ou un groupe $-NH_2$; $R^{11}$ et $R^{12}$ représentent chacun, indépendamment l'un de l'autre, un atome d'hydrogène, un groupe alkyle en $C_1$-$C_6$, un groupe alcényle en $C_2$-$C_6$, un groupe alcynyle en $C_2$-$C_6$, un groupe $-(CH_2)_qOH$, un groupe $-(CH_2)_q$-N(alkyle en $C_1$-$C_4$)$_2$, n groupe $-(CH_2)_q$-S-(alkyle en $C_1$-$C_4$) ou encore un groupe

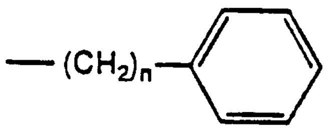

où n est tel que défini ci-dessus et q représente un entier de 1 à 6, ces deux entiers étant inclus; ou bien $R^{11}$ et $R^{12}$ pris ensemble forment un noyau morpholinyle, un noyau pipéridinyle, un noyau pipérazinyle ou un noyau N-méthylpipérazinyle, comprenant le fait de:

a) faire réagir le composé de sulfure racémique avec un réactif préparé à partir de la combinaison d'un ligand de tartrate, d'un alcoxyde de titane, d'un hydroperoxyde et, le cas échéant, d'eau jusqu'à ce qu'essentiellement la totalité de l'énantiomère non désiré du substrat de sulfure ait été transformée en son analogue sulfoxyde; et
b) séparer du mélange réactionnel la portion n'ayant pas réagi de la matière de départ de sulfure constituée essentiellement de l'énantiomère désiré essentiellement pur.

6. Procédé selon la revendication 5, dans lequel on utilise, comme matière de départ, un composé de sulfure racémique dans lequel

$R^1$ et $R^2$ représentent chacun un groupe alkyle en $C_1$-$C_6$, un groupe alcényle en $C_2$-$C_6$, un groupe alcoxy en $C_1$-$C_6$ ou un groupe

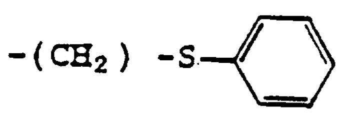

$R^3$, $R^6$ et $R^7$ représentent un atome d'hydrogène; et Q représente un groupe $NR^8$ où $R^8$ représente un atome d'hydrogène, un groupe alkyle en $C_1$-$C_6$ ou un groupe $-(CH_2)_n$-Y où n représente 0 et Y représente un groupe $NR^{11}R^{12}$ ($R^{11}$ et $R^{12}$ représentant chacun, indépendamment l'un de l'autre, un atome d'hydrogène ou un groupe alkyle en $C_1$-$C_6$).

7. Procédé selon la revendication 5 ou 6, dans lequel on utilise le Ti(O-isopropyl)$_4$ à titre d'oxyde de titane.

8. Procédé selon l'une quelconque des revendications 5 à 7, dans lequel on utilise le tartrate de diisopropyle comme ligand de tartrate.

9. Procédé selon l'une quelconque des revendications 5 à 8, dans lequel on utilise l'hydroperoxyde de t-butyle à titre d'hydroperoxyde.

10. Procédé pour préparer une formulation pharmaceutique, qui comprend le fait de mélanger un composé de formule

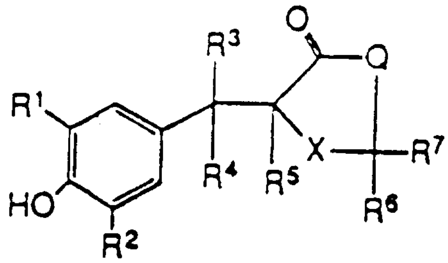

dans laquelle:

$R^1$ représente un groupe alcényle en $C_2$-$C_6$, un groupe alcynyle en $C_2$-$C_6$ ou un groupe

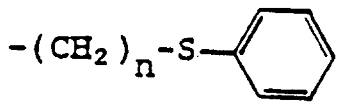

où n représente un entier de 0 à 3, ces deux entiers étant inclus;

$R^2$ représente un atome d'hydrogène, un groupe alkyle en $C_1$-$C_6$, un groupe alcoxy en $C_1$-$C_6$, un groupe alcényle en $C_2$-$C_6$, un groupe alcynyle en $C_2$-$C_6$, un groupe

O
||
alkyl(en $C_1$-$C_4$)-O-C-alkyle en $C_1$-$C_4$,

ou encore un groupe

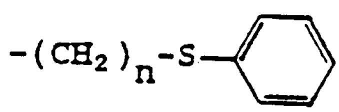

où

n représente un entier de 0 à 3, ces deux entiers étant inclus;

$R^3$ représente un atome d'hydrogène ou un groupe alkyle en $C_1$-$C_6$;